# EUROPEAN PATENT SPECIFICATION

(11) **EP 3 968 771 B1**
(45) Date of publication and mention of the grant of the patent: **31.12.2025**
(21) Application number: 20805144.1
(22) Date of filing: 14.05.2020
(51) Int. Cl.: A01N 37/42, A01N 37/44, C07C 229/08, A01P 15/00, A61K 31/19, A61K 31/198

(54) **METHODS FOR INCREASING THE POLLINATION PERFORMANCE OF BEES**
VERFAHREN ZUR ERHÖHUNG DER BESTÄUBUNGSLEISTUNG VON BIENEN
PROCÉDÉS POUR AUGMENTER LES PERFORMANCES DE POLLINISATION DES ABEILLES

(30) Priority: 15.05.2019 US 201962848392 P
(43) Date of publication of application: 23.03.2022
(73) Proprietor: Beeflow Corporation, Somis CA 93066 (US); Consejo Nacional de Investigaciones Científicas y Técnicas (CONICET), Buenos Aires C1425FQB (AR)
(72) Inventor: VIEL, Matias, Somis, California 93066 (US); NEGRI, Pedro, C1425FQB Buenos Aires (AR); SAEZ, Agustin, C1425FQB Buenos Aires (AR)
(74) Representative: Plasseraud IP
(86) International application number: PCT/US2020/032987
(87) International publication number: WO 2020/232304

(56) References cited:
- US-A1- 2013 303 373
- US-A1- 2014 271 535
- US-A1- 2018 235 191
- US-A1- 2019 090 507
- US-B1- 9 801 358
- PEDRO NEGRI ET AL: "Dietary Supplementation of Honey Bee Larvae with Arginine and Abscisic Acid Enhances Nitric Oxide and Granulocyte Immune Responses after Trauma", INSECTS, vol. 8, no. 3, 15 August 2017 (2017-08-15), pages 85, XP055761677, DOI: 10.3390/insects8030085
- NEGRI PEDRO ET AL: "Abscisic acid enhances the immune response inApis melliferaand contributes to the colony fitness", APIDOLOGIE, ARBEITSGEMEINSCHAFT DER INSTITUTE FUER BIENENFORSCHUNG, CELLE, DE, vol. 46, no. 4, 14 January 2015 (2015-01-14), pages 542 - 557, XP035737686, ISSN: 0044-8435, [retrieved on 20150114], DOI: 10.1007/S13592-014-0345-7
- RAMIREZ LEONOR ET AL: "Abscisic acid enhances cold tolerance in honeybee larvae", vol. 284, no. 1852, 5 April 2017 (2017-04-05), pages 20162140, XP093045152, ISSN: 0962-8452, Retrieved from the Internet <URL:https://royalsocietypublishing.org/doi/full-xml/10.1098/rspb.2016.2140> DOI: 10.1098/rspb.2016.2140
- RIVERO ET AL: "Nitric oxide: an antiparasitic molecule of invertebrates", TRENDS IN PARASITOLOGY, ELSEVIER CURRENT TRENDS, GB, vol. 22, no. 5, 1 May 2006 (2006-05-01), pages 219 - 225, XP028058796, ISSN: 1471-4922, [retrieved on 20060501], DOI: 10.1016/J.PT.2006.02.014
- PEDRO NEGRI, RAMIREZ LEONOR, QUINTANA SILVINA, SZAWARSKI NICOLÁS, MAGGI MATÍAS, LE CONTE YVES, LAMATTINA LORENZO, EGUARAS MARTIN: "Dietary Supplementation of Honey Bee Larvae with Arginine and Abscisic Acid Enhances Nitric Oxide and Granulocyte Immune Responses after Trauma", INSECTS, vol. 8, no. 3, 15 August 2017 (2017-08-15) - September 2017 (2017-09-01), pages 85, XP055761677, DOI: 10.3390/insects8030085

## Description

### CROSS REFERENCE

This application claims the benefit of U.S. Provisional Patent Application No. 62/848, 392 filed May 15, 2019.

### BACKGROUND

Pollination is vital to maintaining both the overall ecosystem of the Earth and civilization's agricultural productivity. Insects, and particularly the honey bee (*Apis mellifera*), are the major pollinators of our planet. Pollination by honey bees contributes significantly to global food production. European honey bees are an established component of the United States' agricultural system. Bees pollinate more that 30% of the food consumed in the world, and in the United States, it is estimated that bees pollinate up to $15 billion worth of crops each year. However, in recent years, honey bee populations have experienced a significant decline, threatening food security.

The largest managed pollination operations in the world are related to Californian almond orchards, where most (almost two million beehives) of the US honey bee colonies are trucked to the almond orchards each spring. New York's apple crop requires about 30,000 hives; Maine's blueberry crop uses about 50,000 hives each year. The US solution to the pollinator shortage, so far, has been for commercial beekeepers to become pollination contractors and to migrate. This is widely impractical due to costs and inherent inefficiency.

In the past five years, there has been a decline in winter managed beehives, which has reached an unprecedented rate of colony losses at near 30% per the US Department of Agriculture. There is a clear need across the agricultural industry for a management tool to equalize and overcome this loss in pollination. Supplementing bees to improve the bees' survival and pollination performance-under optimal and sub-optimal conditions-can increase grower returns and optimize yield from fruit plantings and address the pollinator shortages experienced today.

### SUMMARY

Provided herein are compositions and methods for improving the colony development and foraging activity as a measure of pollination performance of bees. These improvements in colony population and bees' pollination performance will help to solve many of the problems that face the beekeeping and agriculture industries today. The compositions and methods described herein improve the colony development and foraging activity of bees at low temperatures (below 12-15°C). Application of the compositions and methods described herein will greatly improve beekeeping operations related with colony livelihood, honey production and pollination services. Pollination mediated by bees and yields of many pollinator dependent crops will be enhanced too.

One aspect of the present disclosure comprises a method of enhancing fruit-yield of a flowering plant, the method comprising exposing said flowering plant to a plurality of bees, wherein said plurality of bees comprise one or more bees supplemented with a solution comprising about 1 µM to 1500 µM abscisic acid (ABA) and about 0.1 mM to 10 mM L-arginine. In some embodiments, the method further comprises exposing said flowering plant to said plurality of bees, wherein said plurality of bees comprise one or more bees supplemented with a formulation that increases said one or more bees' attractiveness to said flowering plant. In some embodiments, the method further comprises distributing one or more beehives containing said plurality of bees adjacent to a plurality of flowering plants at a rate of at most 10 beehives/hectare of land comprising said plurality of flowering plants. The one or more beehives are homogeneously distributed. In some embodiments, said solution further comprises a sugar to water ratio, wherein said sugar to water ratio is in the range of about 1:1 sugar:water and about 2:1 sugar:water. In some embodiments, said sugar comprises sucrose, fructose, glucose, or a combination thereof. In some embodiments, said sugar comprises sucrose. In some embodiments, said solution comprises about 50 µM ABA. In some embodiments, said solution comprises about 5 mM of L-arginine. The said one or more bees supplemented with said solution comprising about 1 µM to 1500 µM abscisic acid (ABA) and about 0.1 mM to 10 mM L-arginine are fed with said solution. In some embodiments, said flowering plant comprises alfalfa, blueberry, cranberry, scarlet runner bean, sunflower, carrot, guar bean, goa bean, karite, quince, lemon, lime, okra, tamarind, loquat, orange, grapefruit, tangelo, strawberry tree, tangerine, rose hips, dogroses, alsike clover, arrowleaf clover, black currant, red currant, boysenberry, broad bean, clover, cotton, cowpea, black-eyed pea, blackeye bean, crimson clover, crownvetch, flax, kiwifruit, lupine, persimmon, red clover, vetch, watermelon, white clover, almond, apricot, peach, nectarine, plum, greengage, mirabelle, sloe, sour cherry, sweet cherry, blackberry, pear, raspberry, lychee, apple, acerola, azarole, broccoli, brussels sprouts, buckwheat, cabbage, cardamom, cauliflower, chestnut, chinese cabbage, coriander, feijoa, hyacinth bean, lima bean, kidney bean, haricot bean, adzuki bean, mungo bean, string bean, green bean, rapeseed, safflower, sainfoin, turnip, canola, allspice, pigeon pea, cajan pea, congo bean, mustard, pomegranate, onion, rowanberry, caraway, celery, fennel, grape, jujube, elderberry, sesame, cucumber, squash, pumpkin, gourd, marrow, zucchini, cantaloupe, melon, carambola, starfruit, coconut, longan, hog plum, chilli pepper, red pepper, bell pepper, green pepper, macadamia, cashew, strawberry, guava, rambutan, mango, Coffea spp. *Coffea Arabica, CoffeaCanephora,* papaya, beet, or avocado. In some embodiments, said flowering plant comprises blueberry, almond, apple, kiwifruit, or raspberry. In some embodiments, said plurality of bees comprises one or more bees of the Melittidae, Apidae, Megachilidae, Andrenidae, Halictidae, Colletidae, or Stenotritidae families. In some embodiments, said plurality of bees comprises one or more bees of the Apinae, Nomadinae, or Xylocopinae sub-families. In some embodiments, said plurality of bees comprises one or more bees of the species *Apis mellifera, Apis andreniformis, Apis florea, Apis dorsata, Apis cerana, Apis koschevnikovi, Apis nigrocincta, Apis dorsata,* or *Bombus angustus, Bombus terrestris, Bombus atratus, Bombus pauloensis, Bombus impatients.* In some embodiments, said plurality of bees comprises one or more bees of the species *Apis mellifera* or *Apis cerana.* In some embodiments, said plurality of bees comprises one or more bees of the species Apis mellifera. In some embodiments, fruit-yield comprises a flower fruit-set, a fruit weight, a reduction in cull rate, or a combination thereof. In some embodiments, said enhanced fruit-yield is relative to a fruit field of a flowering plant contacted with one or more reference bees not supplemented with the solution comprising about 1 µM to 1500 µM abscisic acid (ABA) and about 0.1 mM to 10 mM L-arginine. In some embodiments, said method comprises enhancing a fruit-set of one or more blueberry flowering plants by about 1% to about 40%. In some embodiments, said method comprises enhancing a fruit-set of one or more almond plants by about 1% to about 40%. In some embodiments, said method comprises enhancing a fruit-set of one or more apple plants by about 1% to about 40%. In some embodiments, said method comprises enhancing a fruit weight of one or more blueberry plants by about 1% to about 40%. In some embodiments, said method comprises reducing a cull rate of one or more raspberry plants by 60%. In some embodiments, said enhanced fruit-yield is relative to a fruit yield of a flowering plant pollinated artificially. In some embodiments, said method comprises enhancing a fruit-set of one or more kiwifruit plants by about 1% to about 100%. In some embodiments, said method comprises enhancing a fruit weight of one or more kiwifruit by about 1% to about 40%. In some embodiments, said method is performed at most about 38 °C. In some embodiments, said method is performed at about 12 °C to about 20 °C.

Another aspect of the present disclosure comprises a method of enhancing a pollination performance of one or more bees, the method comprising contacting said one or more bees with a supplement, wherein said supplement comprises about 1 µM to 1500 µM abscisic acid (ABA) and about 0.1 mM to 10 mM L-arginine. In some embodiments, the method further comprises contacting one or more bees with a formulation that increases said one or more bees' attractiveness to a flowering plant. In some embodiments, the method further comprises distributing one or more beehives containing said one or more bees adjacent to a plurality of flowering plants at a rate of at most 10 beehives/hectare of land comprising said plurality of flowering plants. In some embodiments, said one or more beehives are homogeneously distributed. In some embodiments, said pollination performance comprises forager bee activity, visitation frequency to a flowering plant, or a combination thereof. The method of any one of embodiments 30 to 34, wherein said supplement further comprises a sugar to water ratio, wherein said sugar to water ratio is in the range of about 1:1 sugar:water and about 2:1 sugar:water. The method of any embodiment 35, wherein said sugar comprises sucrose, fructose, glucose, or a combination thereof. In some embodiments, said sugar comprises sucrose. In some embodiments, said supplement is water soluble. In some embodiments, said supplement comprises about 50 µM ABA. In some embodiments, said supplement comprises about 5 mM of L-arginine. In some embodiments, said supplement further comprises an acceptable diluent, excipient, vehicle, carrier, or preservative. In some embodiments, said contacting said one or more bees with a supplement comprises feeding said supplement to said one or more bees. In some embodiments, said flowering plant comprises alfalfa, blueberry, cranberry, scarlet runner bean, sunflower, carrot, guar bean, goa bean, karite, quince, lemon, lime, okra, tamarind, loquat, orange, grapefruit, tangelo, strawberry tree, tangerine, rose hips, dogroses, alsike clover, arrowleaf clover, black currant, red currant, boysenberry, broad bean, clover, cotton, cowpea, black-eyed pea, blackeye bean, crimson clover, crownvetch, flax, kiwifruit, lupine, persimmon, red clover, vetch, watermelon, white clover, almond, apricot, peach, nectarine, plum, greengage, mirabelle, sloe, sour cherry, sweet cherry, blackberry, pear, raspberry, lychee, apple, acerola, azarole, broccoli, brussels sprouts, buckwheat, cabbage, cardamom, cauliflower, chestnut, chinese cabbage, coriander, feijoa, hyacinth bean, lima bean, kidney bean, haricot bean, adzuki bean, mungo bean, string bean, green bean, rapeseed, safflower, sainfoin, turnip, canola, allspice, pigeon pea, cajan pea, congo bean, mustard, pomegranate, onion, rowanberry, caraway, celery, fennel, grape, jujube, elderberry, sesame, cucumber, squash, pumpkin, gourd, marrow, zucchini, cantaloupe, melon, carambola, starfruit, coconut, longan, hog plum, chilli pepper, red pepper, bell pepper, green pepper, macadamia, cashew, strawberry, guava, rambutan, mango, Coffea spp. *Coffea Arabica, CoffeaCanephora,* papaya, beet, or avocado. In some embodiments, said flowering plant comprises blueberry, almond, apple, or kiwifruit. In some embodiments, said one or more bees comprises one or more bees of the Melittidae, Apidae, Megachilidae, Andrenidae, Halictidae, Colletidae, or Stenotritidae families. In some embodiments, said one or more bees comprises one or more bees of the Apinae, Nomadinae, or Xylocopinae sub-families. In some embodiments, said one or more bees comprises one or more bees of the species *Apis mellifera, Apis andreniformis, Apis florea, Apis dorsata, Apis cerana, Apis koschevnikovi, Apis nigrocincta, Apis dorsata,* or *Bombus angustus, Bombus terrestris, Bombus atratus, Bombus pauloensis, Bombus impatients.* In some embodiments, said one or more bees comprises one or more bees of the species *Apis mellifera* or *Apis cerana.* In some embodiments, said one or more bees comprises one or more bees of the species *Apis mellifera.* In some embodiments, said enhanced pollination performance of said one or more bees is relative to one or more reference bees not supplemented with the solution comprising about 1 µM to 1500 µM abscisic acid (ABA) and about 0.1 mM to 10 mM L-arginine. In some embodiments, the method comprises an increase in forager bee activity by up to about 300%. In some embodiments, the method comprises an increase in visitation frequency to said flowering plant by about 100%. In some embodiments, the method comprises an increase in visitation frequency to said flowering plant by about 80%. In some embodiments, said method comprises improvement of flower visitation frequency of said one or more bees by about 60% to about 80%. In some embodiments, said flowering plant comprises an apple flower and the method comprises improvement of flower visitation frequency of the bees by about 73% to about 79%. In some embodiments, said flowering plant comprises an almond flower and said method comprises improvement of flower visitation frequency of the bees by about 66%. In some embodiments, said flowering plant comprises a blueberry flower and said method comprises improvement of flower visitation frequency of the bees by up to about 300%. In some embodiments, said flowering plant comprises a raspberry flower and the method comprises improvement of flower visitation frequency of the bees by about 200%. In some embodiments, said method is performed at most about 38 °C. In some embodiments, said method is performed at about 12 °C to about 20 °C.

Another aspect of the disclosure described herein comprises a method of enhancing fruit-yield of a flowering plant, the method comprising exposing said flowering plant to a plurality of bees, wherein said plurality of bees comprise one or more bees fed a solution comprising about 1 µM to 1500 µM abscisic acid (ABA) and about 0.1 mM to 10 mM L-arginine. In some embodiments, the method further comprises exposing said flowering plant to said plurality of bees, wherein said plurality of bees comprise one or more bees supplemented with a formulation that increases said one or more bees' attractiveness to said flowering plant. In some embodiments, the method further comprises distributing one or more beehives containing said plurality of bees adjacent to a plurality of flowering plants at a rate of at most 10 beehives/hectare of land comprising said plurality of flowering plants. In some embodiments, said one or more beehives are homogeneously distributed. In some embodiments, said solution further comprises a sugar to water ratio, wherein said sugar to water ratio is in the range of about 1:1 sugar:water and about 2:1 sugar:water. In some embodiments, said sugar comprises sucrose, fructose, glucose, or a combination thereof. In some embodiments, said sugar comprises sucrose. In some embodiments, said solution comprises about 50 µM ABA. In some embodiments, said solution comprises about 5 mM of L-arginine. In some embodiments, said flowering plant comprises alfalfa, blueberry, cranberry, scarlet runner bean, sunflower, carrot, guar bean, goa bean, karite, quince, lemon, lime, okra, tamarind, loquat, orange, grapefruit, tangelo, strawberry tree, tangerine, rose hips, dogroses, alsike clover, arrowleaf clover, black currant, red currant, boysenberry, broad bean, clover, cotton, cowpea, black-eyed pea, blackeye bean, crimson clover, crownvetch, flax, kiwifruit, lupine, persimmon, red clover, vetch, watermelon, white clover, almond, apricot, peach, nectarine, plum, greengage, mirabelle, sloe, sour cherry, sweet cherry, blackberry, pear, raspberry, lychee, apple, acerola, azarole, broccoli, brussels sprouts, buckwheat, cabbage, cardamom, cauliflower, chestnut, chinese cabbage, coriander, feijoa, hyacinth bean, lima bean, kidney bean, haricot bean, adzuki bean, mungo bean, string bean, green bean, rapeseed, safflower, sainfoin, turnip, canola, allspice, pigeon pea, cajan pea, congo bean, mustard, pomegranate, onion, rowanberry, caraway, celery, fennel, grape, jujube, elderberry, sesame, cucumber, squash, pumpkin, gourd, marrow, zucchini, cantaloupe, melon, carambola, starfruit, coconut, longan, hog plum, chilli pepper, red pepper, bell pepper, green pepper, macadamia, cashew, strawberry, guava, rambutan, mango, Coffea spp. *Coffea Arabica, CoffeaCanephora,* papaya, beet, or avocado. In some embodiments, said flowering plant comprises blueberry, almond, apple, kiwifruit, or raspberry. In some embodiments, said plurality of bees comprises one or more bees of the Melittidae, Apidae, Megachilidae, Andrenidae, Halictidae, Colletidae, or Stenotritidae families. In some embodiments, said plurality of bees comprises one or more bees of the Apinae, Nomadinae, or Xylocopinae sub-families. In some embodiments, said plurality of bees comprises one or more bees of the species *Apis mellifera, Apis andreniformis, Apis florea, Apis dorsata, Apis cerana, Apis koschevnikovi, Apis nigrocincta, Apis dorsata,* or *Bombus angustus, Bombus terrestris, Bombus atratus, Bombus pauloensis, Bombus impatients.* In some embodiments, said plurality of bees comprises one or more bees of the species *Apis mellifera or Apis cerana.* In some embodiments, said plurality of bees comprises one or more bees of the species *Apis mellifera.* In some embodiments, fruit-yield comprises a flower fruit-set, a fruit weight, a reduction in cull rate, or a combination thereof. In some embodiments, said enhanced fruit-yield is relative to a fruit field of a flowering plant contacted with one or more reference bees not supplemented with the solution comprising about 1 µM to 1500 µM abscisic acid (ABA) and about 0.1 mM to 10 mM L-arginine. In some embodiments, said method comprises enhancing a fruit-set of one or more blueberry flowering plants by about 1% to about 40%. The method of any one of embodiments 61 to 78 wherein said method comprises enhancing a fruit-set of one or more almond plants by about 1% to about 40%. In some embodiments, said method comprises enhancing a fruit-set of one or more apple plants by about 1% to about 40%. In some embodiments, said method comprises enhancing a fruit weight of one or more blueberry plants by about 1% to about 40%. In some embodiments, said method comprises reducing a cull rate of one or more raspberry plants by 60%. In some embodiments, said enhanced fruit-yield is relative to a fruit yield of a flowering plant pollinated artificially. In some embodiments, said method comprises enhancing a fruit-set of one or more kiwifruit plants by about 1% to about 100%. In some embodiments, said method comprises enhancing a fruit weight of one or more kiwifruit by about 1% to about 40%. In some embodiments, said method is performed at most about 38 °C. In some embodiments, said method is performed at about 12 °C to about 20 °C.

Another aspect of the disclosure described herein comprises a method of enhancing a pollination performance of one or more bees, the method comprising feeding said one or more bees a supplement, wherein said supplement comprises about 1 µM to 1500 µM abscisic acid (ABA) and about 0.1 mM to 10 mM L-arginine. In some embodiments, the method further comprises contacting one or more bees with a formulation that increases said one or more bees' attractiveness to a flowering plant. In some embodiments, the method further comprises distributing one or more beehives containing said plurality of bees adjacent to a plurality of flowering plants at a rate of at most 10 beehives/hectare of land comprising said plurality of flowering plants. In some embodiments, said one or more beehives are homogeneously distributed. In some embodiments, said pollination performance comprises forager bee activity, visitation frequency to a flowering plant, or a combination thereof. In some embodiments, said supplement further comprises a sugar to water ratio, wherein said sugar to water ratio is in the range of about 1:1 sugar:water and about 2:1 sugar:water. In some embodiments, said sugar comprises sucrose, fructose, glucose, or a combination thereof. In some embodiments, said sugar comprises sucrose. In some embodiments, said supplement comprises about 50 µM ABA. In some embodiments, said supplement comprises about 5 mM of L-arginine. In some embodiments, said supplement is formulated to be added to an existing food supply of said one or more bees. In some embodiments, said supplement further comprises an acceptable diluent, excipient, vehicle, carrier, or preservative. In some embodiments, said flowering plant comprises alfalfa, blueberry, cranberry, scarlet runner bean, sunflower, carrot, guar bean, goa bean, karite, quince, lemon, lime, okra, tamarind, loquat, orange, grapefruit, tangelo, strawberry tree, tangerine, rose hips, dogroses, alsike clover, arrowleaf clover, black currant, red currant, boysenberry, broad bean, clover, cotton, cowpea, black-eyed pea, blackeye bean, crimson clover, crownvetch, flax, kiwifruit, lupine, persimmon, red clover, vetch, watermelon, white clover, almond, apricot, peach, nectarine, plum, greengage, mirabelle, sloe, sour cherry, sweet cherry, blackberry, pear, raspberry, lychee, apple, acerola, azarole, broccoli, brussels sprouts, buckwheat, cabbage, cardamom, cauliflower, chestnut, chinese cabbage, coriander, feijoa, hyacinth bean, lima bean, kidney bean, haricot bean, adzuki bean, mungo bean, string bean, green bean, rapeseed, safflower, sainfoin, turnip, canola, allspice, pigeon pea, cajan pea, congo bean, mustard, pomegranate, onion, rowanberry, caraway, celery, fennel, grape, jujube, elderberry, sesame, cucumber, squash, pumpkin, gourd, marrow, zucchini, cantaloupe, melon, carambola, starfruit, coconut, longan, hog plum, chilli pepper, red pepper, bell pepper, green pepper, macadamia, cashew, strawberry, guava, rambutan, mango, Coffea spp. *Coffea Arabica, CoffeaCanephora,* papaya, beet, or avocado. In some embodiments, said flowering plant comprises blueberry, almond, apple, kiwifruit, or raspberry. In some embodiments, said plurality of bees comprises one or more bees of the Melittidae, Apidae, Megachilidae, Andrenidae, Halictidae, Colletidae, or Stenotritidae families. In some embodiments, said plurality of bees comprises one or more bees of the Apinae, Nomadinae, or Xylocopinae sub-families. In some embodiments, said flowering plant comprises blueberry, almond, kiwifruit, or apple. In some embodiments, said one or more bees comprises one or more bees of the Melittidae, Apidae, Megachilidae, Andrenidae, Halictidae, Colletidae, or Stenotritidae families. In some embodiments, said one or more bees comprises one or more bees of the Apinae, Nomadinae, or Xylocopinae sub-families. In some embodiments, said plurality of bees comprises one or more bees of the species *Apis mellifera, Apis andreniformis, Apis florea, Apis dorsata, Apis cerana, Apis koschevnikovi, Apis nigrocincta, Apis dorsata,* or *Bombus angustus, Bombus terrestris, Bombus atratus, Bombus pauloensis, Bombus impatients.* In some embodiments, said one or more bees comprises one or more bees of the species *Apis mellifera* or *Apis cerana.* In some embodiments, said one or more bees comprises one or more bees of the species *Apis mellifera.* In some embodiments, said enhanced pollination performance of said one or more bees is relative to one or more reference bees not supplemented with the solution comprising about 1 µM to 1500 µM abscisic acid (ABA) and about 0.1 mM to 10 mM L-arginine. In some embodiments, the method comprises an increase in forager bee activity by up to about 300%. In some embodiments, the method comprises an increase in visitation frequency to said flowering plant by about 100%. In some embodiments, the method comprises an increase in visitation frequency to said flowering plant by about 80%. In some embodiments, said method comprises improvement of flower visitation frequency of said one or more bees by about 60% to about 80%. In some embodiments, said flowering plant comprises an apple flower and the method comprises improvement of flower visitation frequency of the bees by about 73% to about 79%. In some embodiments, said flowering plant comprises an almond flower and said method comprises improvement of flower visitation frequency of the bees by about 66%. In some embodiments, said flowering plant comprises a blueberry flower and said method comprises improvement of flower visitation frequency of the bees by up to about 300%. In some embodiments, said flowering plant comprises a raspberry flower and the method comprises improvement of flower visitation frequency of the bees by about 200%. In some embodiments, said method is performed at most about 38 °C. In some embodiments, said method is performed at about 12 °C to about 20 °C.

Another aspect of the disclosure described herein comprises a method of improving colony development, and foraging activity as a measure of pollination performance of one or more bee(s) comprising feeding the bee(s) a composition comprising abscisic acid (ABA) and/or L-arginine. In some embodiments, the improved in colony development and foraging activity is relative to pollination performance of one or more reference bee(s) not fed the composition. In some embodiments, the improved colony development and foraging activity relative to pollination performance of the bee(s) occurs at temperatures of lower than about 12°C. In some embodiments, the food supply comprises sugar syrup. The method in any one of the preceding claims, wherein the sugar is selected from sucrose, fructose, and glucose. In some embodiments, the composition is water soluble. In some embodiments, the composition further comprises an acceptable diluent, excipient, vehicle, carrier, or preservative. In some embodiments, the ABA is present in the range of 1 µM to 1500 µM. In some embodiments, the L-arginine is present in the range of 0.2 mM to 5 mM. In some embodiments, the composition further comprises supplementing the bee(s) with a nutritional supplement. In some embodiments, the improved colony development and foraging activity of the bee(s) comprises an increase in one or more of: number of combs with bee brood (larvae-pupae) that are part of the population of bees, a number of bees that carry pollen in a population of bees (foragers entering to the beehive per minute), a flower visitation frequency of the bee(s), and an average distance of distribution of the bee(s) relative to a beehive. In some embodiments, a flowering plant pollinated by the bee(s) comprises an improved fruit-yield relative to a flowering plant pollinated by one or more reference bee(s) not fed the composition. In some embodiments, fruit-yield comprises flower fruit-set and/or fruit weight. In some embodiments, the improved pollination performance is of a pollination of a plant selected from blueberry, almond, kiwifruit, and apple. In some embodiments, the improved pollination performance is of a pollination of a plant selected from Alfalfa, Blueberry, Cranberry, Scarlet Runner Bean, Sunflower, Carrot, Guar Bean, Goa Bean, Karite, Quince, Lemon, Lime, Okra, Tamarind, Loquat, Orange, Grapefruit, Tangelo, Strawberry Tree, Tangerine, Rose Hips, Dogroses, Alsike Clover, Arrowleaf Clover, Black Currant, Red Currant, Boysenberry, Broad Bean, Clover, Cotton, Cowpea, Black-Eyed Pea, Blackeye Bean, Crimson Clover, Crownvetch, Flax, Kiwifruit, Lupine, Persimmon, Red Clover, Vetch, Watermelon, White Clover, Almond, Apricot, Peach, Nectarine, Plum, Greengage, Mirabelle, Sloe, Sour Cherry, Sweet Cherry, Blackberry, Pear, Raspberry, Lychee, Apple, Acerola, Azarole, Broccoli, Brussels Sprouts, Buckwheat, Cabbage, Cardamom, Cauliflower, Chestnut, Chinese Cabbage, Coriander, Feijoa, Hyacinth Bean, Lima Bean, Kidney Bean, Haricot Bean, Adzuki Bean, Mungo Bean, String Bean, Green Bean, Rapeseed, Safflower, Sainfoin, Turnip, Canola, Allspice, Pigeon Pea, Cajan Pea, Congo Bean, Mustard, Pomegranate, Onion, Rowanberry, Caraway, Celery, Fennel, Grape, Jujube, Elderberry, Sesame, Cucumber, Squash, Pumpkin, Gourd, Marrow, Zucchini, Cantaloupe, Melon, Carambola, Starfruit, Coconut, Longan, Hog Plum, Chilli Pepper, Red Pepper, Bell Pepper, Green Pepper, Macadamia, Cashew, Strawberry, Guava, Rambutan, Mango, Coffea spp. *Coffea Arabica, Coffea Canephora,* Papaya, Beet, and Avocado. In some embodiments, the method comprises improvement of fruit-set of one or more blueberry plant(s) by about 1% to about 40% in blueberry plant(s) pollinated by the bee(s) relative to one or more blueberry plant(s) pollinated by the reference bee(s). In some embodiments, the method comprises improvement of fruit-set of one or more almond plant(s) by about 1% to about 40% in almond plant(s) pollinated by the bee(s) relative to one or more almond plant(s) pollinated by the reference bee(s). In some embodiments, the method comprises improvement of fruit-set of one or more kiwifruit plant(s) by about 1% to about 80% in kiwifruit plant(s) pollinated by the bee(s) relative to one or more kiwifruit plant(s) pollinated by the reference bee(s). In some embodiments, the method comprises improvement of fruit-set of one or more apple plants about 1% to about 40% in apple plant(s) pollinated by the bee(s) relative to one or more apple plants pollinated by the reference bee(s). In some embodiments, the method comprises improvement of fruit weight of one or more blueberry plant(s) by about 1% to about 40% in blueberry plant(s) pollinated by the bee(s) relative to one or more blueberry plant(s) pollinated by the reference bee(s). In some embodiments, the method comprises improvement of fruit weight of one or more kiwifruit by about 1% to about 40% in kiwifruit plant(s) pollinated by the bee(s) relative to one or more kiwifruit plant(s) pollinated by the reference bee(s). In some embodiments, a colony of bees comprising the bee(s) comprises 100% more bees entering or exiting a beehive housing the colony relative to a population of bees comprising the reference bee(s) at about 12°C. In some embodiments, a colony of bees comprising the bee(s) comprises 66% more bees entering or exiting a beehive housing the colony relative to a population of bees comprising the reference bee(s) at about 15 °C to about 20 °C. In some embodiments, a colony of bees comprising the bee(s) comprises 300% more bees entering or exiting a beehive housing the colony relative to a population of bees comprising the reference bee(s) at about 12 °C to about 20 °C. In some embodiments, the method comprises improvement of flower visitation frequency of the bee(s) by about 60% to about 80% relative to a flower visitation frequency of the reference bee(s). In some embodiments, the flower is an almond flower and the method comprises improvement of flower visitation frequency of the bees by about 66% relative to the flower visitation frequency of the reference bee(s) to an almond flower. In some embodiments, the flower is a blueberry flower and the method comprises improvement of flower visitation frequency of the bees by about 70% relative to the flower visitation frequency of the reference bee(s) to a blueberry flower. In some embodiments, the flower is an apple flower and the method comprises improvement of flower visitation frequency of the bees by about 73% to about 79% relative to the flower visitation frequency of the reference bee(s) to an apple plant. In some embodiments, a population of bees comprising the bee(s) is distributed more consistently relative to the population's beehive relative to a population of bees comprising the reference bee(s). In some embodiments, a colony of bees comprising the bee(s) comprises 100% more bees entering a beehive housing the colony relative to a population of bees comprising the reference bee(s) at about <12°C. In some embodiments, a colony of bees comprising the bee(s) comprises 66% more bees entering a beehive housing the colony relative to a population of bees comprising the reference bee(s) at about 15 °C.

Another aspect of the disclosure described herein comprises a method of improving pollination performance of one or more bee(s) comprising supplementing a food supply of the bee(s) with a composition comprising ABA and/or L-arginine. In some embodiments, the improved pollination performance is relative to pollination performance of one or more reference bee(s) not fed the composition. In some embodiments, the improved pollination performance of the bee(s) occurs at temperatures of lower than about 20°C. In some embodiments, the food supply comprises a sugar. The method in any one of the preceding claims, wherein the sugar is selected from sucrose, fructose, and glucose. In some embodiments, the composition is water soluble. In some embodiments, the composition further comprises a sugar. In some embodiments, the sugar is selected from sucrose, fructose, and glucose. In some embodiments, the composition further comprises an acceptable diluent, excipient, vehicle, carrier, or preservative. In some embodiments, the ABA is present in the range of 1 µM to 1500 µM. In some embodiments, the L-arginine is present in the range of 0.2 mM to 5 mM. In some embodiments, the composition further comprises supplementing the bee(s) with a nutritional supplement. In some embodiments, the improved pollination performance of the bee(s) comprises an increase in one or more of: a number of bees that are foragers in a population of bees, a number of bees that carry pollen in a population of bees, a flower visitation frequency of the bee(s), a rate at which the bee(s) enter or exit a beehive, and an average distance of distribution of the bee(s) relative to a beehive. In some embodiments, a flowering plant pollinated by the bee(s) comprises an improved fruit-yield relative to a flowering plant pollinated by one or more reference bee(s) not fed the composition. In some embodiments, fruit-yield comprises flower fruit-set and/or fruit weight. In some embodiments, the improved pollination performance is of a pollination of a plant selected from blueberry, almond, kiwifruit, and apple. In some embodiments, the improved pollination performance is of a pollination of a plant selected from Alfalfa, Blueberry, Cranberry, Scarlet Runner Bean, Sunflower, Carrot, Guar Bean, Goa Bean, Karite, Quince, Lemon, Lime, Okra, Tamarind, Loquat, Orange, Grapefruit, Tangelo, Strawberry Tree, Tangerine, Rose Hips, Dogroses, Alsike Clover, Arrowleaf Clover, Black Currant, Red Currant, Boysenberry, Broad Bean, Clover, Cotton, Cowpea, Black-Eyed Pea, Blackeye Bean, Crimson Clover, Crownvetch, Flax, Kiwifruit, Lupine, Persimmon, Red Clover, Vetch, Watermelon, White Clover, Almond, Apricot, Peach, Nectarine, Plum, Greengage, Mirabelle, Sloe, Sour Cherry, Sweet Cherry, Blackberry, Pear, Raspberry, Lychee, Apple, Acerola, Azarole, Broccoli, Brussels Sprouts, Buckwheat, Cabbage, Cardamom, Cauliflower, Chestnut, Chinese Cabbage, Coriander, Feijoa, Hyacinth Bean, Lima Bean, Kidney Bean, Haricot Bean, Adzuki Bean, Mungo Bean, String Bean, Green Bean, Rapeseed, Safflower, Sainfoin, Turnip, Canola, Allspice, Pigeon Pea, Cajan Pea, Congo Bean, Mustard, Pomegranate, Onion, Rowanberry, Caraway, Celery, Fennel, Grape, Jujube, Elderberry, Sesame, Cucumber, Squash, Pumpkin, Gourd, Marrow, Zucchini, Cantaloupe, Melon, Carambola, Starfruit, Coconut, Longan, Hog Plum, Chilli Pepper, Red Pepper, Bell Pepper, Green Pepper, Macadamia, Cashew, Strawberry, Guava, Rambutan, Mango, Coffea spp. *Coffea Arabica, Coffea Canephora,* Papaya, Beet, and Avocado. In some embodiments, the method comprises improvement of fruit-set of one or more blueberry plant(s) by about 1% to about 40% in blueberry plant(s) pollinated by the bee(s) relative to one or more blueberry plant(s) pollinated by the reference bee(s). In some embodiments, the method comprises improvement of fruit-set of one or more almond plant(s) by about 1% to about 40% in almond plant(s) pollinated by the bee(s) relative to one or more almond plant(s) pollinated by the reference bee(s). In some embodiments, the method comprises improvement of fruit-set of one or more kiwifruit plant(s) by about 1% to about 80% in kiwifruit plant(s) pollinated by the bee(s) relative to one or more kiwifruit plant(s) pollinated by the reference bee(s). In some embodiments, the method comprises improvement of fruit-set of one or more apple plants about 1% to about 40% in apple plant(s) pollinated by the bee(s) relative to one or more apple plants pollinated by the reference bee(s). In some embodiments, the method comprises improvement of fruit weight of one or more blueberry plant(s) by about 1% to about 40% in blueberry plant(s) pollinated by the bee(s) relative to one or more blueberry plant(s) pollinated by the reference bee(s). In some embodiments, the method comprises improvement of fruit weight of one or more kiwifruit by about 1% to about 40% in kiwifruit plant(s) pollinated by the bee(s) relative to one or more kiwifruit plant(s) pollinated by the reference bee(s). In some embodiments, a colony of bees comprising the bee(s) comprises 100% more bees entering or exiting a beehive housing the colony relative to a population of bees comprising the reference bee(s) at about 12°C. In some embodiments, a colony of bees comprising the bee(s) comprises 66% more bees entering or exiting a beehive housing the colony relative to a population of bees comprising the reference bee(s) at about 15 °C to about 20 °C. In some embodiments, a colony of bees comprising the bee(s) comprises 300% more bees entering or exiting a beehive housing the colony relative to a population of bees comprising the reference bee(s) at about 12 °C to about 20 °C. In some embodiments, the method comprises improvement of flower visitation frequency of the bee(s) by about 60% to about 80% relative to a flower visitation frequency of the reference bee(s). In some embodiments, the flower is an almond flower and the method comprises improvement of flower visitation frequency of the bees by about 66% relative to the flower visitation frequency of the reference bee(s) to an almond flower. In some embodiments, the flower is a blueberry flower and the method comprises improvement of flower visitation frequency of the bees by about 70% relative to the flower visitation frequency of the reference bee(s) to a blueberry flower. In some embodiments, the flower is an apple flower and the method comprises improvement of flower visitation frequency of the bees by about 73% to about 79% relative to the flower visitation frequency of the reference bee(s) to an apple plant. In some embodiments, a population of bees comprising the bee(s) is distributed more consistently relative to the population's beehive relative to a population of bees comprising the reference bee(s).

Another aspect of the disclosure described herein comprises a method of increasing delivery of pollen to a flowering plant comprising supplementing one or more bee(s) with a composition comprising ABA and/or L-arginine, wherein the bee(s) pollinates the flowering plant. In some embodiments, the improved pollination performance is relative to pollination performance of one or more reference bee(s) not fed the composition. In some embodiments, the improved pollination performance of the bee(s) occurs at temperatures of lower than about 20°C. In some embodiments, the food supply comprises a sugar. The method in any one of the preceding claims, wherein the sugar is selected from sucrose, fructose, and glucose. In some embodiments, the composition is water soluble. In some embodiments, the composition further comprises a sugar. In some embodiments, the sugar is selected from sucrose, fructose, and glucose. In some embodiments, the composition further comprises an acceptable diluent, excipient, vehicle, carrier, or preservative. In some embodiments, the ABA is present in the range of 1 µM to 1500 µM. In some embodiments, the L-arginine is present in the range of 0.2 mM to 5 mM. In some embodiments, the composition further comprises supplementing the bee(s) with a nutritional supplement. In some embodiments, the improved pollination performance of the bee(s) comprises an increase in one or more of: a number of bees that are foragers in a population of bees, a number of bees that carry pollen in a population of bees, a flower visitation frequency of the bee(s), a rate at which the bee(s) enter or exit a beehive, and an average distance of distribution of the bee(s) relative to a beehive. In some embodiments, a flowering plant pollinated by the bee(s) comprises an improved fruit-yield relative to a flowering plant pollinated by one or more reference bee(s) not fed the composition. In some embodiments, fruit-yield comprises flower fruit-set and/or fruit weight. In some embodiments, the improved pollination performance is of a pollination of a plant selected from blueberry, almond, kiwifruit, and apple. In some embodiments, the improved pollination performance is of a pollination of a plant selected from Alfalfa, Blueberry, Cranberry, Scarlet Runner Bean, Sunflower, Carrot, Guar Bean, Goa Bean, Karite, Quince, Lemon, Lime, Okra, Tamarind, Loquat, Orange, Grapefruit, Tangelo, Strawberry Tree, Tangerine, Rose Hips, Dogroses, Alsike Clover, Arrowleaf Clover, Black Currant, Red Currant, Boysenberry, Broad Bean, Clover, Cotton, Cowpea, Black-Eyed Pea, Blackeye Bean, Crimson Clover, Crownvetch, Flax, Kiwifruit, Lupine, Persimmon, Red Clover, Vetch, Watermelon, White Clover, Almond, Apricot, Peach, Nectarine, Plum, Greengage, Mirabelle, Sloe, Sour Cherry, Sweet Cherry, Blackberry, Pear, Raspberry, Lychee, Apple, Acerola, Azarole, Broccoli, Brussels Sprouts, Buckwheat, Cabbage, Cardamom, Cauliflower, Chestnut, Chinese Cabbage, Coriander, Feijoa, Hyacinth Bean, Lima Bean, Kidney Bean, Haricot Bean, Adzuki Bean, Mungo Bean, String Bean, Green Bean, Rapeseed, Safflower, Sainfoin, Turnip, Canola, Allspice, Pigeon Pea, Cajan Pea, Congo Bean, Mustard, Pomegranate, Onion, Rowanberry, Caraway, Celery, Fennel, Grape, Jujube, Elderberry, Sesame, Cucumber, Squash, Pumpkin, Gourd, Marrow, Zucchini, Cantaloupe, Melon, Carambola, Starfruit, Coconut, Longan, Hog Plum, Chilli Pepper, Red Pepper, Bell Pepper, Green Pepper, Macadamia, Cashew, Strawberry, Guava, Rambutan, Mango, Coffea spp. *Coffea Arabica, Coffea Canephora,* Papaya, Beet, and Avocado. In some embodiments, the method comprises improvement of fruit-set of one or more blueberry plant(s) by about 1% to about 40% in blueberry plant(s) pollinated by the bee(s) relative to one or more blueberry plant(s) pollinated by the reference bee(s). In some embodiments, the method comprises improvement of fruit-set of one or more almond plant(s) by about 1% to about 40% in almond plant(s) pollinated by the bee(s) relative to one or more almond plant(s) pollinated by the reference bee(s). In some embodiments, the method comprises improvement of fruit-set of one or more kiwifruit plant(s) by about 1% to about 80% in kiwifruit plant(s) pollinated by the bee(s) relative to one or more kiwifruit plant(s) pollinated by the reference bee(s). In some embodiments, the method comprises improvement of fruit-set of one or more apple plants about 1% to about 40% in apple plant(s) pollinated by the bee(s) relative to one or more apple plants pollinated by the reference bee(s). In some embodiments, the method comprises improvement of fruit weight of one or more blueberry plant(s) by about 1% to about 40% in blueberry plant(s) pollinated by the bee(s) relative to one or more blueberry plant(s) pollinated by the reference bee(s). In some embodiments, the method comprises improvement of fruit weight of one or more kiwifruit by about 1% to about 40% in kiwifruit plant(s) pollinated by the bee(s) relative to one or more kiwifruit plant(s) pollinated by the reference bee(s). In some embodiments, a colony of bees comprising the bee(s) comprises 100% more bees entering or exiting a beehive housing the colony relative to a population of bees comprising the reference bee(s) at about 12°C. In some embodiments, a colony of bees comprising the bee(s) comprises 66% more bees entering or exiting a beehive housing the colony relative to a population of bees comprising the reference bee(s) at about 15 °C to about 20 °C. In some embodiments, a colony of bees comprising the bee(s) comprises 300% more bees entering or exiting a beehive housing the colony relative to a population of bees comprising the reference bee(s) at about 12 °C to about 20 °C. In some embodiments, the method comprises improvement of flower visitation frequency of the bee(s) by about 60% to about 80% relative to a flower visitation frequency of the reference bee(s). In some embodiments, the flower is an almond flower and the method comprises improvement of flower visitation frequency of the bees by about 66% relative to the flower visitation frequency of the reference bee(s) to an almond flower. In some embodiments, the flower is a blueberry flower and the method comprises improvement of flower visitation frequency of the bees by about 70% relative to the flower visitation frequency of the reference bee(s) to a blueberry flower. In some embodiments, the flower is an apple flower and the method comprises improvement of flower visitation frequency of the bees by about 73% to about 79% relative to the flower visitation frequency of the reference bee(s) to an apple plant. In some embodiments, a population of bees comprising the bee(s) is distributed more consistently relative to the population's beehive relative to a population of bees comprising the reference bee(s).

Another aspect of the disclosure described herein comprises a composition comprising ABA and L-Arg. In some embodiments, the ABA is present at a concentration of 10 µM. In some embodiments, the L-Arg is present at a concentration of 5 mM. In some embodiments, the composition further comprises a 2:1 sucrose:water syrup. In some embodiments, the ABA is present at a concentration of 50 µM. In some embodiments, the L-Arg is present at a concentration of 5 mM. In some embodiments, comprising a 1:1 sucrose:water syrup.

Another aspect described herein comprises a composition comprising ABA and L-Arg.In some embodiments, the ABA is present at a concentration of 10 µM. In some embodiments, the L-Arg is present at a concentration of 5 mM. In some embodiments, the composition further comprises a 2:1 sucrose:water syrup.

Another aspect described herein comprises a composition comprising ABA and L-Arg.In some embodiments, the ABA is present at a concentration of 50 µM. In some embodiments, the L-Arg is present at a concentration of 5 mM. In some embodiments, the composition further comprises a 1:1 sucrose:water syrup.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:
**Figs. 1A-B** show the effect of the compositions described herein on honey bee performance. **Fig. 1A** shows in the increase in brood size in a population of bees fed the compositions described herein over the period of 24 days. **Fig. 1B** shows an increase in the number of bees entering or exiting a beehive housing the bees' colony in a population of bees fed the compositions described herein over the period of 24 days.

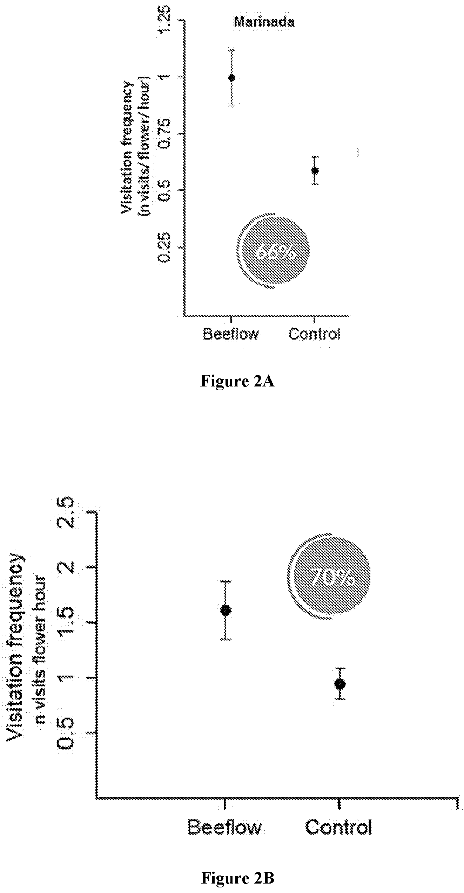

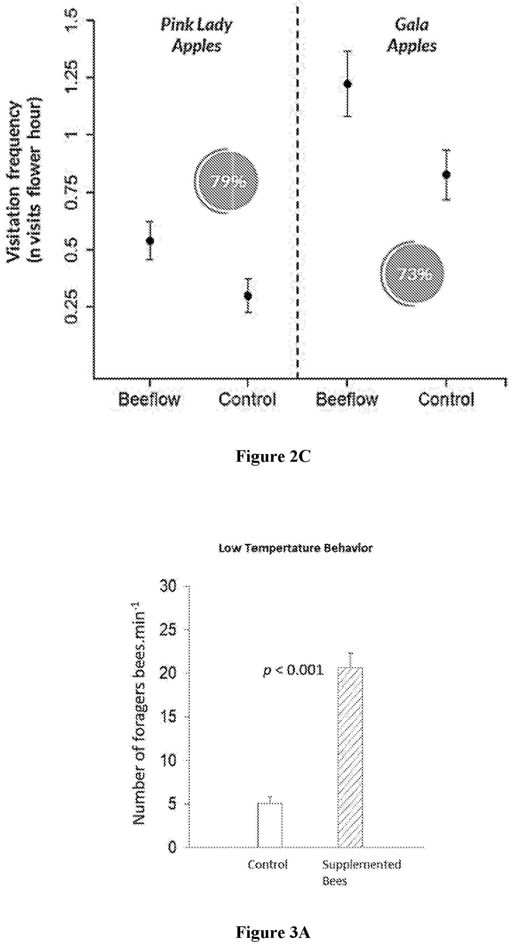

**Figs. 2A-C** show the effect of the compositions and methods described herein on honey bee pollination performance. **Fig. 2A** shows the increase in flower visitation frequency of bees fed the compositions described herein and subjected to the methods described herein. Fig. **2B** shows the increase in flower visitation frequency of bees, fed the compositions described herein and subjected to the methods described herein, for blueberry plants. **Fig. 2C** shows the increase in flower visitation frequency of bees fed the compositions described herein and subjected to the methods described herein for apple plants.
**Figs. 3A-B** show forager bee activity at low temperatures of honey bees fed the compositions described herein. **Fig. 3A** shows the number of forager bees entering or exiting a beehive per minute at temperatures between about 9-10°C. **Fig. 3B** shows forager bee activity at low temperatures of honey bees fed the compositions described herein. It shows the number of forager bees entering or exiting a beehive per minute at temperatures between about 12-15°C (Mann-Whitney test). Different letters indicate significative differences at the *p* values indicated in each graph.
**Fig. 4** shows the increase in fruit-set of almond plants pollinated by honey bees fed the compositions and methods described herein.
**Fig. 5** shows the increase in fruit-yield for blueberry plants pollinated by honey bees fed the compositions and methods described herein. **Fig. 5A** shows the fruit-set. **Fig. 5B** shows the fruit weight.
**Fig. 6** shows the homogeneity of kiwifruit weight produced from plants pollinated by honey bee populations fed the compositions and methods described.
**Fig. 7** shows the increase in fruit-set for apple plants pollinated by honey bees fed the compositions and methods described herein.
**Fig. 8** shows the increase in flower visitation frequency in plots with bees fed with the compositions described above vs plots with bees without the composition in blueberry plants. Visitation frequency (i.e. n honey bee visits · flower⁻¹ · hour⁻¹) observed in Control and Beeflow plots. White bar depicts the observed (±SE) visitation frequency in Control plots, while grey bar in Beeflow ones. Beeflow plot showed 57% more visitation frequency to flowers than Control plots (estimated difference Beeflow-Control = 0.45, *SE*=0.09, *SE*=4.6, *P*<0.001).
**Fig. 9** shows the increase in the fruit-set in plots with bees fed with the compositions described above vs plots with bees without the composition in blueberry plants. Fruit set (i.e. proportion of flowers setting a fruit) observed in Control and Beeflow plots. White bar depicts the observed (±SE) fruit set in Control plots, while grey bar in Beeflow ones. Beeflow plot showed 11% more fruit set that Control plots (estimated difference (in *logit* scale) Beeflow-Control = 0.55, *SE=0.25,* Z=2.16, *P*=0.03)*.*
**Fig. 10A** shows the reduction in raspberries developed with cladosprium.
**Fig. 10B** shows in decrease in cull rate of raspberries produced by treated bees.
**Fig. 10C** shows the increase in flowers visitation frequency of bees fed the composition described herein and subjected to the methods described herein for raspberry plants.

### DETAILED DESCRIPTION OF THE INVENTION

Bees are social insects that live in colonies. Bee colonies consist of a single queen, hundreds of male drones and 10,000 to 80,000 female worker bees. Each bee colony also consists of developing eggs, larvae and pupae (referred to as the "brood").

The number of individuals within a bee colony depends largely upon seasonal changes. A colony could reach up to 80,000 individuals during the active season, when workers forage for food, store honey for winter and build combs. However, this population will decrease dramatically during colder seasons. Due to their highly social life history, bee colonies can be considered superorganisms. This means the entire colony, rather than the bees individually, is viewed as the biological unit.

Forager bees are worker bees from the colony which are specialized in collecting nectar and pollen from flowers. These are the bees responsible for the process of pollination and their activity can be used as a measure of the pollination performance of a honeybee colony.

A "beehive" is used to describe an artificial, man-made structure to house a bee colony. For honey production the western honey bee (*Apis mellifera*) and the eastern honey bee (*Apis cerana)* are the main species kept in hives. Beehives comprise multiple "frames." A hive frame is a structural element in a beehive that holds the honeycomb or brood comb within the hive enclosure or box. As used herein, "frames" refer to Langstroth frames. Beehives typically have a single opening for entry and exit used by forager bees during their pollination-associated flights. Thus, evaluating the number of forager bees arriving through the beehive's entrance can be used as a measure related to pollination performance. Provided herein are compositions and methods for improving the colony development, foraging activity and pollination performance of bees. Provided herein are compositions and methods that relate to the use of abscisic acid and/or L-arginine to improve the colony development, foraging activity and pollination performance of bees and a composition to either feed to bees or supplement the feed of bees. In particular, the disclosure provided herein relates to the use of abscisic acid and L-arginine to improve the colony development, foraging activity and pollination performance of the bee colonies of *Apis mellifera.* Furthermore, in some embodiments, the composition comprises a formulation to supplement bee hives comprising abscisic acid, L-arginine, a sugar, and water. In some embodiments, the compositions described herein are fed to the bees by being placed in the targeted bees' beehive.

### I. COMPOSITIONS

Described herein are compositions comprising abscisic acid ("ABA"). ABA is also named (2Z,4E)-5-[(1S)-1-hydroxy-2,6,6-trimethyl-4-oxocyclohex-2-en-1-yl]-3-methylpenta-2,4-dienoic acid. The *molecular weight* of ABA is 264.32 g/mol. The chemical structure of ABA is:

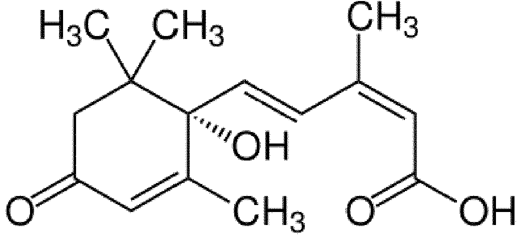

In some embodiments, the ABA is present in the form of a salt or derivative thereof. In some embodiments, the ABA is added to a hive in the form of a composition comprising water and sugar. In some embodiments, the compositions can be applied to the hive according to the traditional techniques used in beekeeping or can be administered to the hive by techniques specially developed for this purpose. In some embodiments, the compositions containing ABA are in the form of an aqueous solution comprising ABA and a sugar. In some embodiments, the compositions can be applied in the hives as, for example, solid formulations, creams, syrups and gels. In preferred embodiments, the composition is water-soluble.

In some embodiments, the ABA is present in the composition in the amount of about 1 µg/g to about 350 µg/g. In some embodiments, the ABA is present in the composition in the amount of about 1 µg/g to about 25 µg/g, about 1 µg/g to about 50 µg/g, about 1 µg/g to about 75 µg/g, about 1 µg/g to about 100 µg/g, about 1 µg/g to about 125 µg/g, about 1 µg/g to about 150 µg/g, about 1 µg/g to about 175 µg/g, about 1 µg/g to about 200 µg/g, about 1 µg/g to about 225 µg/g, about 1 µg/g to about 250 µg/g, about 1 µg/g to about 350 µg/g, about 25 µg/g to about 50 µg/g, about 25 µg/g to about 75 µg/g, about 25 µg/g to about 100 µg/g, about 25 µg/g to about 125 µg/g, about 25 µg/g to about 150 µg/g, about 25 µg/g to about 175 µg/g, about 25 µg/g to about 200 µg/g, about 25 µg/g to about 225 µg/g, about 25 µg/g to about 250 µg/g, about 25 µg/g to about 350 µg/g, about 50 µg/g to about 75 µg/g, about 50 µg/g to about 100 µg/g, about 50 µg/g to about 125 µg/g, about 50 µg/g to about 150 µg/g, about 50 µg/g to about 175 µg/g, about 50 µg/g to about 200 µg/g, about 50 µg/g to about 225 µg/g, about 50 µg/g to about 250 µg/g, about 50 µg/g to about 350 µg/g, about 75 µg/g to about 100 µg/g, about 75 µg/g to about 125 µg/g, about 75 µg/g to about 150 µg/g, about 75 µg/g to about 175 µg/g, about 75 µg/g to about 200 µg/g, about 75 µg/g to about 225 µg/g, about 75 µg/g to about 250 µg/g, about 75 µg/g to about 350 µg/g, about 100 µg/g to about 125 µg/g, about 100 µg/g to about 150 µg/g, about 100 µg/g to about 175 µg/g, about 100 µg/g to about 200 µg/g, about 100 µg/g to about 225 µg/g, about 100 µg/g to about 250 µg/g, about 100 µg/g to about 350 µg/g, about 125 µg/g to about 150 µg/g, about 125 µg/g to about 175 µg/g, about 125 µg/g to about 200 µg/g, about 125 µg/g to about 225 µg/g, about 125 µg/g to about 250 µg/g, about 125 µg/g to about 350 µg/g, about 150 µg/g to about 175 µg/g, about 150 µg/g to about 200 µg/g, about 150 µg/g to about 225 µg/g, about 150 µg/g to about 250 µg/g, about 150 µg/g to about 350 µg/g, about 175 µg/g to about 200 µg/g, about 175 µg/g to about 225 µg/g, about 175 µg/g to about 250 µg/g, about 175 µg/g to about 350 µg/g, about 200 µg/g to about 225 µg/g, about 200 µg/g to about 250 µg/g, about 200 µg/g to about 350 µg/g, about 225 µg/g to about 250 µg/g, about 225 µg/g to about 350 µg/g, or about 250 µg/g to about 350 µg/g. In some embodiments, the ABA is present in the composition in the amount of about 1 µg/g, about 25 µg/g, about 50 µg/g, about 75 µg/g, about 100 µg/g, about 125 µg/g, about 150 µg/g, about 175 µg/g, about 200 µg/g, about 225 µg/g, about 250 µg/g, or about 350 µg/g. In some embodiments, the ABA is present in the composition in the amount of at least about 1 µg/g, about 25 µg/g, about 50 µg/g, about 75 µg/g, about 100 µg/g, about 125 µg/g, about 150 µg/g, about 175 µg/g, about 200 µg/g, about 225 µg/g, or about 250 µg/g. In some embodiments, the ABA is present in the composition in the amount of at most about 25 µg/g, about 50 µg/g, about 75 µg/g, about 100 µg/g, about 125 µg/g, about 150 µg/g, about 175 µg/g, about 200 µg/g, about 225 µg/g, about 250 µg/g, or about 350 µg/g.

In some embodiments, the ABA is present in the range of 1 µM to 1,500 µM. In some embodiments, the ABA is present in the range of 1 µM to 10 µM, 1 µM to 50 µM, 1 µM to 100 µM, 1 µM to 200 µM, 1 µM to 400 µM, 1 µM to 600 µM, 1 µM to 800 µM, 1 µM to 1,000 µM, 1 µM to 1,200 µM, 1 µM to 1,400 µM, 1 µM to 1,500 µM, 10 µM to 50 µM, 10 µM to 100 µM, 10 µM to 200 µM, 10 µM to 400 µM, 10 µM to 600 µM, 10 µM to 800 µM, 10 µM to 1,000 µM, 10 µM to 1,200 µM, 10 µM to 1,400 µM, 10 µM to 1,500 µM, 50 µM to 100 µM, 50 µM to 200 µM, 50 µM to 400 µM, 50 µM to 600 µM, 50 µM to 800 µM, 50 µM to 1,000 µM, 50 µM to 1,200 µM, 50 µM to 1,400 µM, 50 µM to 1,500 µM, 100 µM to 200 µM, 100 µM to 400 µM, 100 µM to 600 µM, 100 µM to 800 µM, 100 µM to 1,000 µM, 100 µM to 1,200 µM, 100 µM to 1,400 µM, 100 µM to 1,500 µM, 200 µM to 400 µM, 200 µM to 600 µM, 200 µM to 800 µM, 200 µM to 1,000 µM, 200 µM to 1,200 µM, 200 µM to 1,400 µM, 200 µM to 1,500 µM, 400 µM to 600 µM, 400 µM to 800 µM, 400 µM to 1,000 µM, 400 µM to 1,200 µM, 400 µM to 1,400 µM, 400 µM to 1,500 µM, 600 µM to 800 µM, 600 µM to 1,000 µM, 600 µM to 1,200 µM, 600 µM to 1,400 µM, 600 µM to 1,500 µM, 800 µM to 1,000 µM, 800 µM to 1,200 µM, 800 µM to 1,400 µM, 800 µM to 1,500 µM, 1,000 µM to 1,200 µM, 1,000 µM to 1,400 µM, 1,000 µM to 1,500 µM, 1,200 µM to 1,400 µM, 1,200 µM to 1,500 µM, or 1,400 µM to 1,500 µM. In some embodiments, the ABA is present in the range of 1 µM, 10 µM, 50 µM, 100 µM, 200 µM, 400 µM, 600 µM, 800 µM, 1,000 µM, 1,200 µM, 1,400 µM, or 1,500 µM. In some embodiments, the ABA is present in the range of at least 1 µM, 10 µM, 50 µM, 100 µM, 200 µM, 400 µM, 600 µM, 800 µM, 1,000 µM, 1,200 µM, or 1,400 µM. In some embodiments, the ABA is present in the range of at most 10 µM, 50 µM, 100 µM, 200 µM, 400 µM, 600 µM, 800 µM, 1,000 µM, 1,200 µM, 1,400 µM, or 1,500 µM. In some embodiments, there is about 50 µM ABA present in the composition.

Described herein are the compositions comprising L-arginine ("L-Arg"). L-Arg is also named (S)-2-Amino-5-guanidinopentanoic acid. The molecular weight for L-arginine is 174.2 g/mol. The chemical structure of L-Arg is:

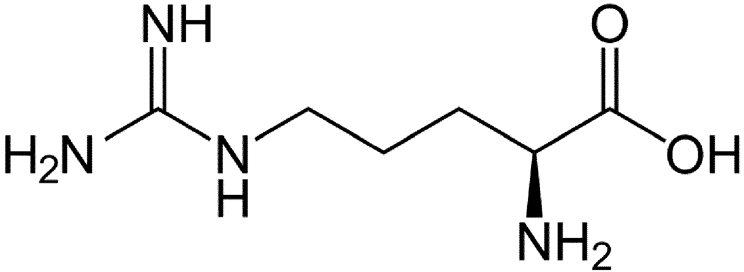

In some embodiments, the L-Arg is present in the form of a salt or derivative thereof. In some embodiments, the L-Arg is added to a hive in the form of a composition comprising water and sugar. In some embodiments, the compositions containing L-Arg are in the form of an aqueous solution comprising L-Arg and a sugar. In some embodiments, the compositions can be applied in the hives as, for example, solid formulations, creams, syrups and gels. In preferred embodiments, the composition is water-soluble.

In some embodiments, the L-Arg is present in the composition in the amount of about 0.2 mM to about 5 mM. In some embodiments, the L-Arg is present in the composition in the amount of about 0.2 mM to about 0.5 mM, about 0.2 mM to about 1 mM, about 0.2 mM to about 1.5 mM, about 0.2 mM to about 2 mM, about 0.2 mM to about 2.5 mM, about 0.2 mM to about 3 mM, about 0.2 mM to about 3.5 mM, about 0.2 mM to about 4 mM, about 0.2 mM to about 4.5 mM, about 0.2 mM to about 5 mM, about 0.5 mM to about 1 mM, about 0.5 mM to about 1.5 mM, about 0.5 mM to about 2 mM, about 0.5 mM to about 2.5 mM, about 0.5 mM to about 3 mM, about 0.5 mM to about 3.5 mM, about 0.5 mM to about 4 mM, about 0.5 mM to about 4.5 mM, about 0.5 mM to about 5 mM, about 1 mM to about 1.5 mM, about 1 mM to about 2 mM, about 1 mM to about 2.5 mM, about 1 mM to about 3 mM, about 1 mM to about 3.5 mM, about 1 mM to about 4 mM, about 1 mM to about 4.5 mM, about 1 mM to about 5 mM, about 1.5 mM to about 2 mM, about 1.5 mM to about 2.5 mM, about 1.5 mM to about 3 mM, about 1.5 mM to about 3.5 mM, about 1.5 mM to about 4 mM, about 1.5 mM to about 4.5 mM, about 1.5 mM to about 5 mM, about 2 mM to about 2.5 mM, about 2 mM to about 3 mM, about 2 mM to about 3.5 mM, about 2 mM to about 4 mM, about 2 mM to about 4.5 mM, about 2 mM to about 5 mM, about 2.5 mM to about 3 mM, about 2.5 mM to about 3.5 mM, about 2.5 mM to about 4 mM, about 2.5 mM to about 4.5 mM, about 2.5 mM to about 5 mM, about 3 mM to about 3.5 mM, about 3 mM to about 4 mM, about 3 mM to about 4.5 mM, about 3 mM to about 5 mM, about 3.5 mM to about 4 mM, about 3.5 mM to about 4.5 mM, about 3.5 mM to about 5 mM, about 4 mM to about 4.5 mM, about 4 mM to about 5 mM, or about 4.5 mM to about 5 mM. In some embodiments, the L-Arg is present in the composition in the amount of about 0.2 mM, about 0.5 mM, about 1 mM, about 1.5 mM, about 2 mM, about 2.5 mM, about 3 mM, about 3.5 mM, about 4 mM, about 4.5 mM, or about 5 mM. In some embodiments, the L-Arg is present in the composition in the amount of at least about 0.2 mM, about 0.5 mM, about 1 mM, about 1.5 mM, about 2 mM, about 2.5 mM, about 3 mM, about 3.5 mM, about 4 mM, or about 4.5 mM. In some embodiments, the L-Arg is present in the composition in the amount of at most about 0.5 mM, about 1 mM, about 1.5 mM, about 2 mM, about 2.5 mM, about 3 mM, about 3.5 mM, about 4 mM, about 4.5 mM, or about 5 mM. In some embodiments, there is about 5 mM L-Arg present in the composition.

In some embodiments, the compositions described herein comprise both ABA and L-Arg. In some embodiments, the ABA is present in the range of 1 µM to 1,500 µM. In some embodiments, the ABA is present in the range of 1 µM to 10 µM, 1 µM to 50 µM, 1 µM to 100 µM, 1 µM to 200 µM, 1 µM to 400 µM, 1 µM to 600 µM, 1 µM to 800 µM, 1 µM to 1,000 µM, 1 µM to 1,200 µM, 1 µM to 1,400 µM, 1 µM to 1,500 µM, 10 µM to 50 µM, 10 µM to 100 µM, 10 µM to 200 µM, 10 µM to 400 µM, 10 µM to 600 µM, 10 µM to 800 µM, 10 µM to 1,000 µM, 10 µM to 1,200 µM, 10 µM to 1,400 µM, 10 µM to 1,500 µM, 50 µM to 100 µM, 50 µM to 200 µM, 50 µM to 400 µM, 50 µM to 600 µM, 50 µM to 800 µM, 50 µM to 1,000 µM, 50 µM to 1,200 µM, 50 µM to 1,400 µM, 50 µM to 1,500 µM, 100 µM to 200 µM, 100 µM to 400 µM, 100 µM to 600 µM, 100 µM to 800 µM, 100 µM to 1,000 µM, 100 µM to 1,200 µM, 100 µM to 1,400 µM, 100 µM to 1,500 µM, 200 µM to 400 µM, 200 µM to 600 µM, 200 µM to 800 µM, 200 µM to 1,000 µM, 200 µM to 1,200 µM, 200 µM to 1,400 µM, 200 µM to 1,500 µM, 400 µM to 600 µM, 400 µM to 800 µM, 400 µM to 1,000 µM, 400 µM to 1,200 µM, 400 µM to 1,400 µM, 400 µM to 1,500 µM, 600 µM to 800 µM, 600 µM to 1,000 µM, 600 µM to 1,200 µM, 600 µM to 1,400 µM, 600 µM to 1,500 µM, 800 µM to 1,000 µM, 800 µM to 1,200 µM, 800 µM to 1,400 µM, 800 µM to 1,500 µM, 1,000 µM to 1,200 µM, 1,000 µM to 1,400 µM, 1,000 µM to 1,500 µM, 1,200 µM to 1,400 µM, 1,200 µM to 1,500 µM, or 1,400 µM to 1,500 µM. In some embodiments, the ABA is present in the range of 1 µM, 10 µM, 50 µM, 100 µM, 200 µM, 400 µM, 600 µM, 800 µM, 1,000 µM, 1,200 µM, 1,400 µM, or 1,500 µM. In some embodiments, the ABA is present in the range of at least 1 µM, 10 µM, 50 µM, 100 µM, 200 µM, 400 µM, 600 µM, 800 µM, 1,000 µM, 1,200 µM, or 1,400 µM. In some embodiments, the ABA is present in the range of at most 10 µM, 50 µM, 100 µM, 200 µM, 400 µM, 600 µM, 800 µM, 1,000 µM, 1,200 µM, 1,400 µM, or 1,500 µM. In some embodiments, the L-Arg is present in the composition in the amount of about 0.2 mM to about 5 mM. In some embodiments, the L-Arg is present in the composition in the amount of about 0.2 mM to about 0.5 mM, about 0.2 mM to about 1 mM, about 0.2 mM to about 1.5 mM, about 0.2 mM to about 2 mM, about 0.2 mM to about 2.5 mM, about 0.2 mM to about 3 mM, about 0.2 mM to about 3.5 mM, about 0.2 mM to about 4 mM, about 0.2 mM to about 4.5 mM, about 0.2 mM to about 5 mM, about 0.5 mM to about 1 mM, about 0.5 mM to about 1.5 mM, about 0.5 mM to about 2 mM, about 0.5 mM to about 2.5 mM, about 0.5 mM to about 3 mM, about 0.5 mM to about 3.5 mM, about 0.5 mM to about 4 mM, about 0.5 mM to about 4.5 mM, about 0.5 mM to about 5 mM, about 1 mM to about 1.5 mM, about 1 mM to about 2 mM, about 1 mM to about 2.5 mM, about 1 mM to about 3 mM, about 1 mM to about 3.5 mM, about 1 mM to about 4 mM, about 1 mM to about 4.5 mM, about 1 mM to about 5 mM, about 1.5 mM to about 2 mM, about 1.5 mM to about 2.5 mM, about 1.5 mM to about 3 mM, about 1.5 mM to about 3.5 mM, about 1.5 mM to about 4 mM, about 1.5 mM to about 4.5 mM, about 1.5 mM to about 5 mM, about 2 mM to about 2.5 mM, about 2 mM to about 3 mM, about 2 mM to about 3.5 mM, about 2 mM to about 4 mM, about 2 mM to about 4.5 mM, about 2 mM to about 5 mM, about 2.5 mM to about 3 mM, about 2.5 mM to about 3.5 mM, about 2.5 mM to about 4 mM, about 2.5 mM to about 4.5 mM, about 2.5 mM to about 5 mM, about 3 mM to about 3.5 mM, about 3 mM to about 4 mM, about 3 mM to about 4.5 mM, about 3 mM to about 5 mM, about 3.5 mM to about 4 mM, about 3.5 mM to about 4.5 mM, about 3.5 mM to about 5 mM, about 4 mM to about 4.5 mM, about 4 mM to about 5 mM, or about 4.5 mM to about 5 mM. In some embodiments, the L-Arg is present in the composition in the amount of about 0.2 mM, about 0.5 mM, about 1 mM, about 1.5 mM, about 2 mM, about 2.5 mM, about 3 mM, about 3.5 mM, about 4 mM, about 4.5 mM, or about 5 mM. In some embodiments, the L-Arg is present in the composition in the amount of at least about 0.2 mM, about 0.5 mM, about 1 mM, about 1.5 mM, about 2 mM, about 2.5 mM, about 3 mM, about 3.5 mM, about 4 mM, or about 4.5 mM. In some embodiments, the L-Arg is present in the composition in the amount of at most about 0.5 mM, about 1 mM, about 1.5 mM, about 2 mM, about 2.5 mM, about 3 mM, about 3.5 mM, about 4 mM, about 4.5 mM, or about 5 mM.

In some embodiments, the compositions further comprise a sugar. In some embodiments, the sugar is present in the composition at a ratio of 2:1, w/v. In some embodiments, the sugar is present in the composition at a ratio of 1:1, w/v. In some embodiments, the sugar is sucrose, fructose, or glucose.

In some embodiments, the compositions further comprise water.

In some embodiments, the compositions comprise 50 µM of ABA and 5mM of L-Arg in a 2:1 sucrose:water syrup. In preferred embodiments, the compositions comprise 10 µM of ABA and 5mM of L-Arg in a 2:1 sucrose:water syrup.

In some embodiments, the compositions comprise 50 µM of ABA and 5mM of L-Arg in a 1:1 sucrose:water syrup. In preferred embodiments, the compositions comprise 10 µM of ABA and 5mM of L-Arg in a 1:1 sucrose:water syrup.

In some embodiments, the compositions also comprise other ingredients that may be beneficial to bees, such as excipients, and such as preservatives, antioxidants, colorants, and other additives that may be necessary for the preservation stored commercialization of the formulation or nutritional supplements.

In some embodiments, the compositions can be applied to the beehive according to the traditional techniques used in beekeeping or can be administered to bee or the hive by techniques specially developed for this purpose.

In some embodiments, the compositions described herein are administered to a bee in form of an aerosol. In some embodiments the compositions described herein are coupled to a structure that is coupled to a beehive, including but not limited to, a net.

In some embodiments, about 1 liter (L) / week of the composition is fed to honey bees. In some embodiments, the amount fed to honey bees is about 0.2 L/week to about 2.2 L/week. In some embodiments, the amount fed to honey bees is about 0.2 L/week to about 0.4 L/week, about 0.2 L/week to about 0.6 L/week, about 0.2 L/week to about 0.8 L/week, about 0.2 L/week to about 1 L/week, about 0.2 L/week to about 1.2 L/week, about 0.2 L/week to about 1.4 L/week, about 0.2 L/week to about 1.6 L/week, about 0.2 L/week to about 1.8 L/week, about 0.2 L/week to about 2 L/week, about 0.2 L/week to about 2.2 L/week, about 0.4 L/week to about 0.6 L/week, about 0.4 L/week to about 0.8 L/week, about 0.4 L/week to about 1 L/week, about 0.4 L/week to about 1.2 L/week, about 0.4 L/week to about 1.4 L/week, about 0.4 L/week to about 1.6 L/week, about 0.4 L/week to about 1.8 L/week, about 0.4 L/week to about 2 L/week, about 0.4 L/week to about 2.2 L/week, about 0.6 L/week to about 0.8 L/week, about 0.6 L/week to about 1 L/week, about 0.6 L/week to about 1.2 L/week, about 0.6 L/week to about 1.4 L/week, about 0.6 L/week to about 1.6 L/week, about 0.6 L/week to about 1.8 L/week, about 0.6 L/week to about 2 L/week, about 0.6 L/week to about 2.2 L/week, about 0.8 L/week to about 1 L/week, about 0.8 L/week to about 1.2 L/week, about 0.8 L/week to about 1.4 L/week, about 0.8 L/week to about 1.6 L/week, about 0.8 L/week to about 1.8 L/week, about 0.8 L/week to about 2 L/week, about 0.8 L/week to about 2.2 L/week, about 1 L/week to about 1.2 L/week, about 1 L/week to about 1.4 L/week, about 1 L/week to about 1.6 L/week, about 1 L/week to about 1.8 L/week, about 1 L/week to about 2 L/week, about 1 L/week to about 2.2 L/week, about 1.2 L/week to about 1.4 L/week, about 1.2 L/week to about 1.6 L/week, about 1.2 L/week to about 1.8 L/week, about 1.2 L/week to about 2 L/week, about 1.2 L/week to about 2.2 L/week, about 1.4 L/week to about 1.6 L/week, about 1.4 L/week to about 1.8 L/week, about 1.4 L/week to about 2 L/week, about 1.4 L/week to about 2.2 L/week, about 1.6 L/week to about 1.8 L/week, about 1.6 L/week to about 2 L/week, about 1.6 L/week to about 2.2 L/week, about 1.8 L/week to about 2 L/week, about 1.8 L/week to about 2.2 L/week, or about 2 L/week to about 2.2 L/week. In some embodiments, the amount fed to honey bees is about 0.2 L/week, about 0.4 L/week, about 0.6 L/week, about 0.8 L/week, about 1 L/week, about 1.2 L/week, about 1.4 L/week, about 1.6 L/week, about 1.8 L/week, about 2 L/week, or about 2.2 L/week. In some embodiments, the amount fed to honey bees is at least about 0.2 L/week, about 0.4 L/week, about 0.6 L/week, about 0.8 L/week, about 1 L/week, about 1.2 L/week, about 1.4 L/week, about 1.6 L/week, about 1.8 L/week, or about 2 L/week. In some embodiments, the amount fed to honey bees is at most about 0.4 L/week, about 0.6 L/week, about 0.8 L/week, about 1 L/week, about 1.2 L/week, about 1.4 L/week, about 1.6 L/week, about 1.8 L/week, about 2 L/week, or about 2.2 L/week.

### Composition for Attractiveness to Specific Flowering Plant

In some embodiments, an additional distinct composition is described herein that increases bees' attractiveness to a specific flowering plant. In some embodiments, this composition that increases bees' attractiveness is formulated specific to a particular flowering plant (e.g. the flowering plants described herein). This composition for blueberries is described in WO2019021209; this composition for pears is described in WO2019021210; this composition for apples is described in WO2013005200; this composition for sunflowers is described in WO201300519.

### II. DISTRIBUTION OF BEEHIVES

In some embodiments of the methods described herein, beehives containing the bees contacted with the composition described herein are individually distributed throughout plots of the flowering plants described herein.

In some embodiments, the beehives are homogeneously spaced.

In some embodiments, the beehives are distributed at about 1 hive/hectare to 12 hives/hectare of the flowering plants described herein. In some embodiments, the beehives are distributed at about 1 hive/hectare to about 12 hives/hectare. In some embodiments, the beehives are distributed at about 1 hive/hectare to about 2 hives/hectare, about 1 hive/hectare to about 3 hives/hectare, about 1 hive/hectare to about 4 hives/hectare, about 1 hive/hectare to about 5 hives/hectare, about 1 hive/hectare to about 6 hives/hectare, about 1 hive/hectare to about 7 hives/hectare, about 1 hive/hectare to about 8 hives/hectare, about 1 hive/hectare to about 9 hives/hectare, about 1 hive/hectare to about 10 hives/hectare, about 1 hive/hectare to about 11 hives/hectare, about 1 hive/hectare to about 12 hives/hectare, about 2 hives/hectare to about 3 hives/hectare, about 2 hives/hectare to about 4 hives/hectare, about 2 hives/hectare to about 5 hives/hectare, about 2 hives/hectare to about 6 hives/hectare, about 2 hives/hectare to about 7 hives/hectare, about 2 hives/hectare to about 8 hives/hectare, about 2 hives/hectare to about 9 hives/hectare, about 2 hives/hectare to about 10 hives/hectare, about 2 hives/hectare to about 11 hives/hectare, about 2 hives/hectare to about 12 hives/hectare, about 3 hives/hectare to about 4 hives/hectare, about 3 hives/hectare to about 5 hives/hectare, about 3 hives/hectare to about 6 hives/hectare, about 3 hives/hectare to about 7 hives/hectare, about 3 hives/hectare to about 8 hives/hectare, about 3 hives/hectare to about 9 hives/hectare, about 3 hives/hectare to about 10 hives/hectare, about 3 hives/hectare to about 11 hives/hectare, about 3 hives/hectare to about 12 hives/hectare, about 4 hives/hectare to about 5 hives/hectare, about 4 hives/hectare to about 6 hives/hectare, about 4 hives/hectare to about 7 hives/hectare, about 4 hives/hectare to about 8 hives/hectare, about 4 hives/hectare to about 9 hives/hectare, about 4 hives/hectare to about 10 hives/hectare, about 4 hives/hectare to about 11 hives/hectare, about 4 hives/hectare to about 12 hives/hectare, about 5 hives/hectare to about 6 hives/hectare, about 5 hives/hectare to about 7 hives/hectare, about 5 hives/hectare to about 8 hives/hectare, about 5 hives/hectare to about 9 hives/hectare, about 5 hives/hectare to about 10 hives/hectare, about 5 hives/hectare to about 11 hives/hectare, about 5 hives/hectare to about 12 hives/hectare, about 6 hives/hectare to about 7 hives/hectare, about 6 hives/hectare to about 8 hives/hectare, about 6 hives/hectare to about 9 hives/hectare, about 6 hives/hectare to about 10 hives/hectare, about 6 hives/hectare to about 11 hives/hectare, about 6 hives/hectare to about 12 hives/hectare, about 7 hives/hectare to about 8 hives/hectare, about 7 hives/hectare to about 9 hives/hectare, about 7 hives/hectare to about 10 hives/hectare, about 7 hives/hectare to about 11 hives/hectare, about 7 hives/hectare to about 12 hives/hectare, about 8 hives/hectare to about 9 hives/hectare, about 8 hives/hectare to about 10 hives/hectare, about 8 hives/hectare to about 11 hives/hectare, about 8 hives/hectare to about 12 hives/hectare, about 9 hives/hectare to about 10 hives/hectare, about 9 hives/hectare to about 11 hives/hectare, about 9 hives/hectare to about 12 hives/hectare, about 10 hives/hectare to about 11 hives/hectare, about 10 hives/hectare to about 12 hives/hectare, or about 11 hives/hectare to about 12 hives/hectare. In some embodiments, the beehives are distributed at about 1 hive/hectare, about 2 hives/hectare, about 3 hives/hectare, about 4 hives/hectare, about 5 hives/hectare, about 6 hives/hectare, about 7 hives/hectare, about 8 hives/hectare, about 9 hives/hectare, about 10 hives/hectare, about 11 hives/hectare, or about 12 hives/hectare. In some embodiments, the beehives are distributed at least about 1 hive/hectare, about 2 hives/hectare, about 3 hives/hectare, about 4 hives/hectare, about 5 hives/hectare, about 6 hives/hectare, about 7 hives/hectare, about 8 hives/hectare, about 9 hives/hectare, about 10 hives/hectare, or about 11 hives/hectare. In some embodiments, the beehives are distributed at most about 2 hives/hectare, about 3 hives/hectare, about 4 hives/hectare, about 5 hives/hectare, about 6 hives/hectare, about 7 hives/hectare, about 8 hives/hectare, about 9 hives/hectare, about 10 hives/hectare, about 11 hives/hectare, or about 12 hives/hectare.

### III. METHODS

In some embodiments, prior to blooming season of a flowering plant, beehives are selected based on (a) number of combs covered with adult bees, (b) number of combs with brood, and (c) the presence of an active egg-laying queen. In some embodiments of the present disclosure, the compounds described herein are fed to bees from the selected beehives to increase the bees' pollination performance (e.g. foraging activity). In some embodiments, bees are supplemented with the composition comprising 50 µM abscisic acid (ABA) and about 5 mM L-arginine (L-Arg). In some embodiments, the supplement is fed to the bees prior to blooming period of the flowering plant. In some embodiments, the supplement was fed to the bees about 1 month prior to the blooming period of the flowering plant to about 6 months prior to the blooming period of the flowering plant. In some embodiments, the supplement was fed to the bees about 1 month prior to the blooming period of the flowering plant to about 2 months prior to the blooming period of the flowering plant, about 1 month prior to the blooming period of the flowering plant to about 3 months prior to the blooming period of the flowering plant, about 1 month prior to the blooming period of the flowering plant to about 4 months prior to the blooming period of the flowering plant, about 1 month prior to the blooming period of the flowering plant to about 5 months prior to the blooming period of the flowering plant, about 1 month prior to the blooming period of the flowering plant to about 6 months prior to the blooming period of the flowering plant, about 2 months prior to the blooming period of the flowering plant to about 3 months prior to the blooming period of the flowering plant, about 2 months prior to the blooming period of the flowering plant to about 4 months prior to the blooming period of the flowering plant, about 2 months prior to the blooming period of the flowering plant to about 5 months prior to the blooming period of the flowering plant, about 2 months prior to the blooming period of the flowering plant to about 6 months prior to the blooming period of the flowering plant, about 3 months prior to the blooming period of the flowering plant to about 4 months prior to the blooming period of the flowering plant, about 3 months prior to the blooming period of the flowering plant to about 5 months prior to the blooming period of the flowering plant, about 3 months prior to the blooming period of the flowering plant to about 6 months prior to the blooming period of the flowering plant, about 4 months prior to the blooming period of the flowering plant to about 5 months prior to the blooming period of the flowering plant, about 4 months prior to the blooming period of the flowering plant to about 6 months prior to the blooming period of the flowering plant, or about 5 months prior to the blooming period of the flowering plant to about 6 months prior to the blooming period of the flowering plant. In some embodiments, the supplement was fed to the bees about 1 month prior to the blooming period of the flowering plant, about 2 months prior to the blooming period of the flowering plant, about 3 months prior to the blooming period of the flowering plant, about 4 months prior to the blooming period of the flowering plant, about 5 months prior to the blooming period of the flowering plant, or about 6 months prior to the blooming period of the flowering plant. In some embodiments, the supplement was fed to the bees at least about 1 month prior to the blooming period of the flowering plant, about 2 months prior to the blooming period of the flowering plant, about 3 months prior to the blooming period of the flowering plant, about 4 months prior to the blooming period of the flowering plant, or about 5 months prior to the blooming period of the flowering plant. In some embodiments, the supplement was fed to the bees at most about 2 months prior to the blooming period of the flowering plant, about 3 months prior to the blooming period of the flowering plant, about 4 months prior to the blooming period of the flowering plant, about 5 months prior to the blooming period of the flowering plant, or about 6 months prior to the blooming period of the flowering plant. In some embodiments, the bees are supplemented with a composition described herein that increases bees' attractiveness to a specific flowering plant. In some embodiments, the bees are supplemented with the composition that increases bees' attractiveness to the specific flowering plant at the start of the blooming period for the flowering plant.

In some embodiments, the bees are fed supplement every about 1 day to about 10 days. In some embodiments, the bees are fed supplement every about 1 day to about 2 days, about 1 day to about 3 days, about 1 day to about 4 days, about 1 day to about 5 days, about 1 day to about 6 days, about 1 day to about 7 days, about 1 day to about 8 days, about 1 day to about 9 days, about 1 day to about 10 days, about 2 days to about 3 days, about 2 days to about 4 days, about 2 days to about 5 days, about 2 days to about 6 days, about 2 days to about 7 days, about 2 days to about 8 days, about 2 days to about 9 days, about 2 days to about 10 days, about 3 days to about 4 days, about 3 days to about 5 days, about 3 days to about 6 days, about 3 days to about 7 days, about 3 days to about 8 days, about 3 days to about 9 days, about 3 days to about 10 days, about 4 days to about 5 days, about 4 days to about 6 days, about 4 days to about 7 days, about 4 days to about 8 days, about 4 days to about 9 days, about 4 days to about 10 days, about 5 days to about 6 days, about 5 days to about 7 days, about 5 days to about 8 days, about 5 days to about 9 days, about 5 days to about 10 days, about 6 days to about 7 days, about 6 days to about 8 days, about 6 days to about 9 days, about 6 days to about 10 days, about 7 days to about 8 days, about 7 days to about 9 days, about 7 days to about 10 days, about 8 days to about 9 days, about 8 days to about 10 days, or about 9 days to about 10 days. In some embodiments, the bees are fed supplement every about 1 day, about 2 days, about 3 days, about 4 days, about 5 days, about 6 days, about 7 days, about 8 days, about 9 days, or about 10 days. In some embodiments, the bees are fed supplement every at least about 1 day, about 2 days, about 3 days, about 4 days, about 5 days, about 6 days, about 7 days, about 8 days, or about 9 days. In some embodiments, the bees are fed supplement every at most about 2 days, about 3 days, about 4 days, about 5 days, about 6 days, about 7 days, about 8 days, about 9 days, or about 10 days. In some embodiments, the bees are fed the supplement every 7 days,

In some embodiments, the beehives containing the bees fed the compositions described herein are individually and homogeneously distributed among the flowering plants. In some embodiments, the beehives are distributed once the flowering plants start to bloom. In some embodiments, the beehives were removed from the flowering plants once about 95% of the female flowers became senescent.

In some embodiments, the beehives are protected from pesticides typically sprayed on the flowering plants. In some embodiments, the beehives are protected by a net. In some embodiments, the net is mesh. In some embodiments, the beehives are protected by a pesticide retardant. In some embodiments, the beehives are protected by a means know in the art.

In some embodiments, the beehives are monitored and assessed during the flowering period of the flowering plants described herein. In some embodiments, the beehives are assessed for the growth of healthy bees and a queen's laying of eggs.

### IV. BEE PERFORMANCE

The compositions described herein, when fed to a bee, improve the colony development and foraging activity as a measure of pollination performance of the bee. The colony development and foraging activity can be measured in a variety of ways. In some embodiments, colony development is measured in the number of brood within a given population of bees that belong to a particular beehive. In some embodiments, foraging activity is measured in the number of forager bees (bees that pollinate flowers) within a given population of bees or a given population of bees that belong to a particular beehive. In some embodiments, foraging activity is measured by the number of bees entering a particular beehive per minute. In some embodiments, pollination performance is measured in the amount of pollen carried by forager bees. In some embodiments, pollination performance is measured flower visitation frequency. Flower visitation frequency is defined as the amount of occurrences a bee from a bee population visits a defined quantity of flowers per unit of time. The visitation rate to flowers was estimated at the same time in precision and conventional plots, at different times of the day. Pollinator censuses were performed by recording the number of flowers visited by pollinators for a period of 5 mins (e.g, number of visits . flower-1 . 5 min-1). Each census involved a different randomly selected group of flowers, within a randomized location within the plantation.

In some embodiments, the amount of forager bees entering to the beehive from a population of bees fed the compositions described herein improved relative to reference bees by about 20% to about 240%. In some embodiments, the amount of forager bees in a population of bees fed the compositions described herein improved relative to reference bees by about 20% to about 40%, about 20% to about 60%, about 20% to about 80%, about 20% to about 100%, about 20% to about 120%, about 20% to about 140%, about 20% to about 160%, about 20% to about 180%, about 20% to about 200%, about 20% to about 220%, about 20% to about 240%, about 40% to about 60%, about 40% to about 80%, about 40% to about 100%, about 40% to about 120%, about 40% to about 140%, about 40% to about 160%, about 40% to about 180%, about 40% to about 200%, about 40% to about 220%, about 40% to about 240%, about 60% to about 80%, about 60% to about 100%, about 60% to about 120%, about 60% to about 140%, about 60% to about 160%, about 60% to about 180%, about 60% to about 200%, about 60% to about 220%, about 60% to about 240%, about 80% to about 100%, about 80% to about 120%, about 80% to about 140%, about 80% to about 160%, about 80% to about 180%, about 80% to about 200%, about 80% to about 220%, about 80% to about 240%, about 100% to about 120%, about 100% to about 140%, about 100% to about 160%, about 100% to about 180%, about 100% to about 200%, about 100% to about 220%, about 100% to about 240%, about 120% to about 140%, about 120% to about 160%, about 120% to about 180%, about 120% to about 200%, about 120% to about 220%, about 120% to about 240%, about 140% to about 160%, about 140% to about 180%, about 140% to about 200%, about 140% to about 220%, about 140% to about 240%, about 160% to about 180%, about 160% to about 200%, about 160% to about 220%, about 160% to about 240%, about 180% to about 200%, about 180% to about 220%, about 180% to about 240%, about 200% to about 220%, about 200% to about 240%, or about 220% to about 240%. In some embodiments, the amount of forager bees in a population of bees fed the compositions described herein improved relative to reference bees by about 20%, about 40%, about 60%, about 80%, about 100%, about 120%, about 140%, about 160%, about 180%, about 200%, about 220%, or about 240%. In some embodiments, the amount of forager bees in a population of bees fed the compositions described herein improved relative to reference bees by at least about 20%, about 40%, about 60%, about 80%, about 100%, about 120%, about 140%, about 160%, about 180%, about 200%, or about 220%. In some embodiments, the amount of forager bees in a population of bees fed the compositions described herein improved relative to reference bees by at most about 40%, about 60%, about 80%, about 100%, about 120%, about 140%, about 160%, about 180%, about 200%, about 220%, or about 240%.

In some embodiments, the amount of forager bees entering to the beehive from a population of bees fed the compositions described herein improved relative to reference bees by about 66% to about 100%. In some embodiments, the amount of forager bees entering to the beehive from a population of bees fed the compositions described herein improved relative to reference bees by about 66 % to about 100 %. In some embodiments, the amount of forager bees entering to the beehive from a population of bees fed the compositions described herein improved relative to reference bees by about 66 % to about 70 %, about 66 % to about 75 %, about 66 % to about 80 %, about 66 % to about 85 %, about 66 % to about 90 %, about 66 % to about 95 %, about 66 % to about 100 %, about 70 % to about 75 %, about 70 % to about 80 %, about 70 % to about 85 %, about 70 % to about 90 %, about 70 % to about 95 %, about 70 % to about 100 %, about 75 % to about 80 %, about 75 % to about 85 %, about 75 % to about 90 %, about 75 % to about 95 %, about 75 % to about 100 %, about 80 % to about 85 %, about 80 % to about 90 %, about 80 % to about 95 %, about 80 % to about 100 %, about 85 % to about 90 %, about 85 % to about 95 %, about 85 % to about 100 %, about 90 % to about 95 %, about 90 % to about 100 %, or about 95 % to about 100 %. In some embodiments, the amount of forager bees entering to the beehive from a population of bees fed the compositions described herein improved relative to reference bees by about 66 %, about 70 %, about 75 %, about 80 %, about 85 %, about 90 %, about 95 %, or about 100 %. In some embodiments, the amount of forager bees entering to the beehive from a population of bees fed the compositions described herein improved relative to reference bees by at least about 66 %, about 70 %, about 75 %, about 80 %, about 85 %, about 90 %, or about 95 %. In some embodiments, the amount of forager bees entering to the beehive from a population of bees fed the compositions described herein improved relative to reference bees by at most about 70 %, about 75 %, about 80 %, about 85 %, about 90 %, about 95 %, or about 100 %. In some embodiments, the amount of forager bees entering to the beehive from a population of bees fed the compositions described herein improved relative to reference bees by about 66% at a temperature of 15°C, p = 0.026. In some embodiments, the amount of forager bees entering to the beehive from a population of bees fed the compositions described herein improved relative to reference bees by about 100% at temperature of 12°C, p = 0.009.

In some embodiments, the amount of times a bee in a population of bees fed the compositions described herein enters or exits a beehive improved relative to a reference bee by about 40% to about 500%. In some embodiments, the amount of times a bee in a population of bees fed the compositions described herein enters or exits a beehive improved relative to a reference bee by about 40% to about 80%, about 40% to about 120%, about 40% to about 160%, about 40% to about 200%, about 40% to about 240%, about 40% to about 280%, about 40% to about 320%, about 40% to about 360%, about 40% to about 400%, about 40% to about 440%, about 40% to about 500%, about 80% to about 120%, about 80% to about 160%, about 80% to about 200%, about 80% to about 240%, about 80% to about 280%, about 80% to about 320%, about 80% to about 360%, about 80% to about 400%, about 80% to about 440%, about 80% to about 500%, about 120% to about 160%, about 120% to about 200%, about 120% to about 240%, about 120% to about 280%, about 120% to about 320%, about 120% to about 360%, about 120% to about 400%, about 120% to about 440%, about 120% to about 500%, about 160% to about 200%, about 160% to about 240%, about 160% to about 280%, about 160% to about 320%, about 160% to about 360%, about 160% to about 400%, about 160% to about 440%, about 160% to about 500%, about 200% to about 240%, about 200% to about 280%, about 200% to about 320%, about 200% to about 360%, about 200% to about 400%, about 200% to about 440%, about 200% to about 500%, about 240% to about 280%, about 240% to about 320%, about 240% to about 360%, about 240% to about 400%, about 240% to about 440%, about 240% to about 500%, about 280% to about 320%, about 280% to about 360%, about 280% to about 400%, about 280% to about 440%, about 280% to about 500%, about 320% to about 360%, about 320% to about 400%, about 320% to about 440%, about 320% to about 500%, about 360% to about 400%, about 360% to about 440%, about 360% to about 500%, about 400% to about 440%, about 400% to about 500%, or about 440% to about 500%. In some embodiments, the amount of times a forager bee in a population of bees fed the compositions described herein enters or exits a beehive improved relative to a reference bee by about 40%, about 80%, about 120%, about 160%, about 200%, about 240%, about 280%, about 320%, about 360%, about 400%, about 440%, or about 500%. In some embodiments, the amount of times a bee in a population of bees fed the compositions described herein enters or exits a beehive improved relative to a reference bee by at least about 40%, about 80%, about 120%, about 160%, about 200%, about 240%, about 280%, about 320%, about 360%, about 400%, or about 440%. In some embodiments, the amount of times a bee in a population of bees fed the compositions described herein enters or exits a beehive improved relative to a reference bee by at most about 80%, about 120%, about 160%, about 200%, about 240%, about 280%, about 320%, about 360%, about 400%, about 440%, or about 500%.

In some embodiments, the flower visitation frequency of the bee(s) fed the compositions described herein improved relative to reference bees by about 60% to about 80%. In some embodiments, the improvement of flower visitation frequency of the bee(s) is about 60% to about 62%, about 60% to about 64%, about 60% to about 66%, about 60% to about 68%, about 60% to about 70%, about 60% to about 72%, about 60% to about 74%, about 60% to about 76%, about 60% to about 78%, about 60% to about 80%, about 62% to about 64%, about 62% to about 66%, about 62% to about 68%, about 62% to about 70%, about 62% to about 72%, about 62% to about 74%, about 62% to about 76%, about 62% to about 78%, about 62% to about 80%, about 64% to about 66%, about 64% to about 68%, about 64% to about 70%, about 64% to about 72%, about 64% to about 74%, about 64% to about 76%, about 64% to about 78%, about 64% to about 80%, about 66% to about 68%, about 66% to about 70%, about 66% to about 72%, about 66% to about 74%, about 66% to about 76%, about 66% to about 78%, about 66% to about 80%, about 68% to about 70%, about 68% to about 72%, about 68% to about 74%, about 68% to about 76%, about 68% to about 78%, about 68% to about 80%, about 70% to about 72%, about 70% to about 74%, about 70% to about 76%, about 70% to about 78%, about 70% to about 80%, about 72% to about 74%, about 72% to about 76%, about 72% to about 78%, about 72% to about 80%, about 74% to about 76%, about 74% to about 78%, about 74% to about 80%, about 76% to about 78%, about 76% to about 80%, or about 78% to about 80%. In some embodiments, the improvement of flower visitation frequency of the bee(s) is about 60%, about 62%, about 64%, about 66%, about 68%, about 70%, about 72%, about 74%, about 76%, about 78%, or about 80%. In some embodiments, the improvement of flower visitation frequency of the bee(s) is at least about 60%, about 62%, about 64%, about 66%, about 68%, about 70%, about 72%, about 74%, about 76%, or about 78%. In some embodiments, the improvement of flower visitation frequency of the bee(s) is at most about 62%, about 64%, about 66%, about 68%, about 70%, about 72%, about 74%, about 76%, about 78%, or about 80%.

In some embodiments, the improved foraging activity of the bee(s) occurs at temperatures of about 5°C to about 20°C. In some embodiments, the improved foraging activity of the bee(s) occurs at temperatures of about 5°C to about 7°C, about 5°C to about 9°C, about 5°C to about 10°C, about 5°C to about 12°C, about 5°C to about 14°C, about 5°C to about 16°C, about 5°C to about 18°C, about 5°C to about 20°C, about 7°C to about 9°C, about 7°C to about 10°C, about 7°C to about 12°C, about 7°C to about 14°C, about 7°C to about 16°C, about 7°C to about 18°C, about 7°C to about 20°C, about 9°C to about 10°C, about 9°C to about 12°C, about 9°C to about 14°C, about 9°C to about 16°C, about 9°C to about 18°C, about 9°C to about 20°C, about 10°C to about 12°C, about 10°C to about 14°C, about 10°C to about 16°C, about 10°C to about 18°C, about 10°C to about 20°C, about 12°C to about 14°C, about 12°C to about 16°C, about 12°C to about 18°C, about 12°C to about 20°C, about 14°C to about 16°C, about 14°C to about 18°C, about 14°C to about 20°C, about 16°C to about 18°C, about 16°C to about 20°C, or about 18°C to about 20°C. In some embodiments, the improved foraging activity of the bee(s) occurs at temperatures of about 5°C, about 7°C, about 9°C, about 10°C, about 12°C, about 14°C, about 16°C, about 18°C, or about 20°C. In some embodiments, the improved foraging activity of the bee(s) occurs at temperatures of at least about 5°C, about 7°C, about 9°C, about 10°C, about 12°C, about 14°C, about 16°C, or about 18°C. In some embodiments, the foraging activity performance of the bee(s) occurs at temperatures of at most about 7°C, about 9°C, about 10°C, about 12°C, about 14°C, about 16°C, about 18°C, or about 20°C.

In some embodiments, the improved foraging activity of the bee(s) occurs at temperatures of about 20 °C to about 38 °C. In some embodiments, the improved foraging activity of the bee(s) occurs at temperatures of about 20 °C to about 22 °C, about 20 °C to about 24 °C, about 20 °C to about 26 °C, about 20 °C to about 28 °C, about 20 °C to about 30 °C, about 20 °C to about 32 °C, about 20 °C to about 34 °C, about 20 °C to about 36 °C, about 20 °C to about 38 °C, about 22 °C to about 24 °C, about 22 °C to about 26 °C, about 22 °C to about 28 °C, about 22 °C to about 30 °C, about 22 °C to about 32 °C, about 22 °C to about 34 °C, about 22 °C to about 36 °C, about 22 °C to about 38 °C, about 24 °C to about 26 °C, about 24 °C to about 28 °C, about 24 °C to about 30 °C, about 24 °C to about 32 °C, about 24 °C to about 34 °C, about 24 °C to about 36 °C, about 24 °C to about 38 °C, about 26 °C to about 28 °C, about 26 °C to about 30 °C, about 26 °C to about 32 °C, about 26 °C to about 34 °C, about 26 °C to about 36 °C, about 26 °C to about 38 °C, about 28 °C to about 30 °C, about 28 °C to about 32 °C, about 28 °C to about 34 °C, about 28 °C to about 36 °C, about 28 °C to about 38 °C, about 30 °C to about 32 °C, about 30 °C to about 34 °C, about 30 °C to about 36 °C, about 30 °C to about 38 °C, about 32 °C to about 34 °C, about 32 °C to about 36 °C, about 32 °C to about 38 °C, about 34 °C to about 36 °C, about 34 °C to about 38 °C, or about 36 °C to about 38 °C. In some embodiments, the improved foraging activity of the bee(s) occurs at temperatures of about 20 °C, about 22 °C, about 24 °C, about 26 °C, about 28 °C, about 30 °C, about 32 °C, about 34 °C, about 36 °C, or about 38 °C. In some embodiments, the improved foraging activity of the bee(s) occurs at temperatures of at least about 20 °C, about 22 °C, about 24 °C, about 26 °C, about 28 °C, about 30 °C, about 32 °C, about 34 °C, or about 36 °C. In some embodiments, the improved foraging activity of the bee(s) occurs at temperatures of at most about 22 °C, about 24 °C, about 26 °C, about 28 °C, about 30 °C, about 32 °C, about 34 °C, about 36 °C, or about 38 °C.

In some embodiments, a population of bees comprising the bee(s) comprises 66% more bees entering a beehive housing the population relative to a population of bees comprising the reference bee(s) at about 15°C.to about 20°C. In some embodiments, a population of bees comprising the bee(s) comprises 66% more entering a beehive housing the population relative to a population of bees comprising the reference bee(s) at about 15°C to about 16°C, about 15°C to about 17°C, about 15°C to about 18°C, about 15°C to about 19°C, about 15°C to about 20°C, about 16°C to about 17°C, about 16°C to about 18°C, about 16°C to about 19°C, about 16°C to about 20°C, about 17°C to about 18°C, about 17°C to about 19°C, about 17°C to about 20°C, about 18°C to about 19°C, about 18°C to about 20°C, or about 19°C to about 20°C. In some embodiments, a population of bees comprising the bee(s) comprises 66% more bees entering or exiting a beehive housing the population relative to a population of bees comprising the reference bee(s) at about 15°C, about 16°C, about 17°C, about 18°C, about 19°C, or about 20°C. In some embodiments, a population of bees comprising the bee(s) comprises 66% more forager bees relative to a population of bees comprising the reference bee(s) at least about 15°C, about 16°C, about 17°C, about 18°C, or about 19°C. In some embodiments, a population of bees comprising the bee(s) comprises 66% more forager bees relative to a population of bees comprising the reference bee(s) at most about 16°C, about 17°C, about 18°C, about 19°C, or about 20°C.

In some embodiments, a population of bees comprising the bee(s) comprises 66% more bees entering a beehive housing the population relative to a population of bees comprising the reference bee(s) at about about 20 °C to about 38 °C. In some embodiments, a population of bees comprising the bee(s) comprises 66% more bees entering a beehive housing the population relative to a population of bees comprising the reference bee(s) at about about 20 °C to about 22 °C, about 20 °C to about 24 °C, about 20 °C to about 26 °C, about 20 °C to about 28 °C, about 20 °C to about 30 °C, about 20 °C to about 32 °C, about 20 °C to about 34 °C, about 20 °C to about 36 °C, about 20 °C to about 38 °C, about 22 °C to about 24 °C, about 22 °C to about 26 °C, about 22 °C to about 28 °C, about 22 °C to about 30 °C, about 22 °C to about 32 °C, about 22 °C to about 34 °C, about 22 °C to about 36 °C, about 22 °C to about 38 °C, about 24 °C to about 26 °C, about 24 °C to about 28 °C, about 24 °C to about 30 °C, about 24 °C to about 32 °C, about 24 °C to about 34 °C, about 24 °C to about 36 °C, about 24 °C to about 38 °C, about 26 °C to about 28 °C, about 26 °C to about 30 °C, about 26 °C to about 32 °C, about 26 °C to about 34 °C, about 26 °C to about 36 °C, about 26 °C to about 38 °C, about 28 °C to about 30 °C, about 28 °C to about 32 °C, about 28 °C to about 34 °C, about 28 °C to about 36 °C, about 28 °C to about 38 °C, about 30 °C to about 32 °C, about 30 °C to about 34 °C, about 30 °C to about 36 °C, about 30 °C to about 38 °C, about 32 °C to about 34 °C, about 32 °C to about 36 °C, about 32 °C to about 38 °C, about 34 °C to about 36 °C, about 34 °C to about 38 °C, or about 36 °C to about 38 °C. In some embodiments, a population of bees comprising the bee(s) comprises 66% more bees entering a beehive housing the population relative to a population of bees comprising the reference bee(s) at about about 20 °C, about 22 °C, about 24 °C, about 26 °C, about 28 °C, about 30 °C, about 32 °C, about 34 °C, about 36 °C, or about 38 °C. In some embodiments, a population of bees comprising the bee(s) comprises 66% more bees entering a beehive housing the population relative to a population of bees comprising the reference bee(s) at about at least about 20 °C, about 22 °C, about 24 °C, about 26 °C, about 28 °C, about 30 °C, about 32 °C, about 34 °C, or about 36 °C. In some embodiments, a population of bees comprising the bee(s) comprises 66% more bees entering a beehive housing the population relative to a population of bees comprising the reference bee(s) at about at most about 22 °C, about 24 °C, about 26 °C, about 28 °C, about 30 °C, about 32 °C, about 34 °C, about 36 °C, or about 38 °C.

In some embodiments, a population of bees comprising the bee(s) comprises 100% more bees entering or exiting a beehive housing the population relative to a population of bees comprising the reference bee(s) at about 12°C to about 20°C. In some embodiments, a population of bees comprising the bee(s) comprises 100% more bees entering or exiting a beehive housing the population relative to a population of bees comprising the reference bee(s) at about 12°C to about 13°C, about 12°C to about 14°C, about 12°C to about 15°C, about 12°C to about 16°C, about 12°C to about 17°C, about 12°C to about 18°C, about 12°C to about 19°C, about 12°C to about 20°C, about 13°C to about 14°C, about 13°C to about 15°C, about 13°C to about 16°C, about 13°C to about 17°C, about 13°C to about 18°C, about 13°C to about 19°C, about 13°C to about 20°C, about 14°C to about 15°C, about 14°C to about 16°C, about 14°C to about 17°C, about 14°C to about 18°C, about 14°C to about 19°C, about 14°C to about 20°C, about 15°C to about 16°C, about 15°C to about 17°C, about 15°C to about 18°C, about 15°C to about 19°C, about 15°C to about 20°C, about 16°C to about 17°C, about 16°C to about 18°C, about 16°C to about 19°C, about 16°C to about 20°C, about 17°C to about 18°C, about 17°C to about 19°C, about 17°C to about 20°C, about 18°C to about 19°C, about 18°C to about 20°C, or about 19°C to about 20°C. In some embodiments, a population of bees comprising the bee(s) comprises 300% more bees entering or exiting a beehive housing the population relative to a population of bees comprising the reference bee(s) at about 12°C, about 13°C, about 14°C, about 15°C, about 16°C, about 17°C, about 18°C, about 19°C, or about 20°C. In some embodiments, a population of bees comprising the bee(s) comprises 300% more bees entering or exiting a beehive housing the population relative to a population of bees comprising the reference bee(s) at least about 12°C, about 13°C, about 14°C, about 15°C, about 16°C, about 17°C, about 18°C, or about 19°C. In some embodiments, a population of bees comprising the bee(s) comprises 300% more bees entering or exiting a beehive housing the population relative to a population of bees comprising the reference bee(s) at most about 13°C, about 14°C, about 15°C, about 16°C, about 17°C, about 18°C, about 19°C, or about 20°C. In some embodiments, a population of bees comprising the bee(s) comprises about 100% more bees entering or exiting a beehive housing the population relative to a population of bees comprising the reference bee(s) at about 12°C to about 15°C to about 320% more bees entering or exiting a beehive housing the population relative to a population of bees comprising the reference bee(s) at about 12°C to about 15°C. In some embodiments, a population of bees comprising the bee(s) comprises about 100% more bees entering or exiting a beehive housing the population relative to a population of bees comprising the reference bee(s) at about 12°C to about 15°C to about 120% more bees entering or exiting a beehive housing the population relative to a population of bees comprising the reference bee(s) at about 12°C to about 15°C, about 100% more bees entering or exiting a beehive housing the population relative to a population of bees comprising the reference bee(s) at about 12°C to about 15°C to about 140% more bees entering or exiting a beehive housing the population relative to a population of bees comprising the reference bee(s) at about 12°C to about 15°C, about 100% more bees entering or exiting a beehive housing the population relative to a population of bees comprising the reference bee(s) at about 12°C to about 15°C to about 160% more bees entering or exiting a beehive housing the population relative to a population of bees comprising the reference bee(s) at about 12°C to about 15°C, about 100% more bees entering or exiting a beehive housing the population relative to a population of bees comprising the reference bee(s) at about 12°C to about 15°C to about 180% more bees entering or exiting a beehive housing the population relative to a population of bees comprising the reference bee(s) at about 12°C to about 15°C, about 100% more bees entering or exiting a beehive housing the population relative to a population of bees comprising the reference bee(s) at about 12°C to about 15°C to about 200% more bees entering or exiting a beehive housing the population relative to a population of bees comprising the reference bee(s) at about 12°C to about 15°C, about 100% more bees entering or exiting a beehive housing the population relative to a population of bees comprising the reference bee(s) at about 12°C to about 15°C to about 220% more bees entering or exiting a beehive housing the population relative to a population of bees comprising the reference bee(s) at about 12°C to about 15°C, about 100% more bees entering or exiting a beehive housing the population relative to a population of bees comprising the reference bee(s) at about 12°C to about 15°C to about 240% more bees entering or exiting a beehive housing the population relative to a population of bees comprising the reference bee(s) at about 12°C to about 15°C, about 100% more bees entering or exiting a beehive housing the population relative to a population of bees comprising the reference bee(s) at about 12°C to about 15°C to about 260% more bees entering or exiting a beehive housing the population relative to a population of bees comprising the reference bee(s) at about 12°C to about 15°C, about 100% more bees entering or exiting a beehive housing the population relative to a population of bees comprising the reference bee(s) at about 12°C to about 15°C to about 280% more bees entering or exiting a beehive housing the population relative to a population of bees comprising the reference bee(s) at about 12°C to about 15°C, about 100% more bees entering or exiting a beehive housing the population relative to a population of bees comprising the reference bee(s) at about 12°C to about 15°C to about 300% more bees entering or exiting a beehive housing the population relative to a population of bees comprising the reference bee(s) at about 12°C to about 15°C, about 100% more bees entering or exiting a beehive housing the population relative to a population of bees comprising the reference bee(s) at about 12°C to about 15°C to about 320% more bees entering or exiting a beehive housing the population relative to a population of bees comprising the reference bee(s) at about 12°C to about 15°C, about 120% more bees entering or exiting a beehive housing the population relative to a population of bees comprising the reference bee(s) at about 12°C to about 15°C to about 140% more bees entering or exiting a beehive housing the population relative to a population of bees comprising the reference bee(s) at about 12°C to about 15°C, about 120% more bees entering or exiting a beehive housing the population relative to a population of bees comprising the reference bee(s) at about 12°C to about 15°C to about 160% more bees entering or exiting a beehive housing the population relative to a population of bees comprising the reference bee(s) at about 12°C to about 15°C, about 120% more bees entering or exiting a beehive housing the population relative to a population of bees comprising the reference bee(s) at about 12°C to about 15°C to about 180% more bees entering or exiting a beehive housing the population relative to a population of bees comprising the reference bee(s) at about 12°C to about 15°C, about 120% more bees entering or exiting a beehive housing the population relative to a population of bees comprising the reference bee(s) at about 12°C to about 15°C to about 200% more bees entering or exiting a beehive housing the population relative to a population of bees comprising the reference bee(s) at about 12°C to about 15°C, about 120% more bees entering or exiting a beehive housing the population relative to a population of bees comprising the reference bee(s) at about 12°C to about 15°C to about 220% more bees entering or exiting a beehive housing the population relative to a population of bees comprising the reference bee(s) at about 12°C to about 15°C, about 120% more bees entering or exiting a beehive housing the population relative to a population of bees comprising the reference bee(s) at about 12°C to about 15°C to about 240% more bees entering or exiting a beehive housing the population relative to a population of bees comprising the reference bee(s) at about 12°C to about 15°C, about 120% more bees entering or exiting a beehive housing the population relative to a population of bees comprising the reference bee(s) at about 12°C to about 15°C to about 260% more bees entering or exiting a beehive housing the population relative to a population of bees comprising the reference bee(s) at about 12°C to about 15°C, about 120% more bees entering or exiting a beehive housing the population relative to a population of bees comprising the reference bee(s) at about 12°C to about 15°C to about 280% more bees entering or exiting a beehive housing the population relative to a population of bees comprising the reference bee(s) at about 12°C to about 15°C, about 120% more bees entering or exiting a beehive housing the population relative to a population of bees comprising the reference bee(s) at about 12°C to about 15°C to about 300% more bees entering or exiting a beehive housing the population relative to a population of bees comprising the reference bee(s) at about 12°C to about 15°C, about 120% more bees entering or exiting a beehive housing the population relative to a population of bees comprising the reference bee(s) at about 12°C to about 15°C to about 320% more bees entering or exiting a beehive housing the population relative to a population of bees comprising the reference bee(s) at about 12°C to about 15°C, about 140% more bees entering or exiting a beehive housing the population relative to a population of bees comprising the reference bee(s) at about 12°C to about 15°C to about 160% more bees entering or exiting a beehive housing the population relative to a population of bees comprising the reference bee(s) at about 12°C to about 15°C, about 140% more bees entering or exiting a beehive housing the population relative to a population of bees comprising the reference bee(s) at about 12°C to about 15°C to about 180% more bees entering or exiting a beehive housing the population relative to a population of bees comprising the reference bee(s) at about 12°C to about 15°C, about 140% more bees entering or exiting a beehive housing the population relative to a population of bees comprising the reference bee(s) at about 12°C to about 15°C to about 200% more bees entering or exiting a beehive housing the population relative to a population of bees comprising the reference bee(s) at about 12°C to about 15°C, about 140% more bees entering or exiting a beehive housing the population relative to a population of bees comprising the reference bee(s) at about 12°C to about 15°C to about 220% more bees entering or exiting a beehive housing the population relative to a population of bees comprising the reference bee(s) at about 12°C to about 15°C, about 140% more bees entering or exiting a beehive housing the population relative to a population of bees comprising the reference bee(s) at about 12°C to about 15°C to about 240% more bees entering or exiting a beehive housing the population relative to a population of bees comprising the reference bee(s) at about 12°C to about 15°C, about 140% more bees entering or exiting a beehive housing the population relative to a population of bees comprising the reference bee(s) at about 12°C to about 15°C to about 260% more bees entering or exiting a beehive housing the population relative to a population of bees comprising the reference bee(s) at about 12°C to about 15°C, about 140% more bees entering or exiting a beehive housing the population relative to a population of bees comprising the reference bee(s) at about 12°C to about 15°C to about 280% more bees entering or exiting a beehive housing the population relative to a population of bees comprising the reference bee(s) at about 12°C to about 15°C, about 140% more bees entering or exiting a beehive housing the population relative to a population of bees comprising the reference bee(s) at about 12°C to about 15°C to about 300% more bees entering or exiting a beehive housing the population relative to a population of bees comprising the reference bee(s) at about 12°C to about 15°C, about 140% more bees entering or exiting a beehive housing the population relative to a population of bees comprising the reference bee(s) at about 12°C to about 15°C to about 320% more bees entering or exiting a beehive housing the population relative to a population of bees comprising the reference bee(s) at about 12°C to about 15°C, about 160% more bees entering or exiting a beehive housing the population relative to a population of bees comprising the reference bee(s) at about 12°C to about 15°C to about 180% more bees entering or exiting a beehive housing the population relative to a population of bees comprising the reference bee(s) at about 12°C to about 15°C, about 160% more bees entering or exiting a beehive housing the population relative to a population of bees comprising the reference bee(s) at about 12°C to about 15°C to about 200% more bees entering or exiting a beehive housing the population relative to a population of bees comprising the reference bee(s) at about 12°C to about 15°C, about 160% more bees entering or exiting a beehive housing the population relative to a population of bees comprising the reference bee(s) at about 12°C to about 15°C to about 220% more bees entering or exiting a beehive housing the population relative to a population of bees comprising the reference bee(s) at about 12°C to about 15°C, about 160% more bees entering or exiting a beehive housing the population relative to a population of bees comprising the reference bee(s) at about 12°C to about 15°C to about 240% more bees entering or exiting a beehive housing the population relative to a population of bees comprising the reference bee(s) at about 12°C to about 15°C, about 160% more bees entering or exiting a beehive housing the population relative to a population of bees comprising the reference bee(s) at about 12°C to about 15°C to about 260% more bees entering or exiting a beehive housing the population relative to a population of bees comprising the reference bee(s) at about 12°C to about 15°C, about 160% more bees entering or exiting a beehive housing the population relative to a population of bees comprising the reference bee(s) at about 12°C to about 15°C to about 280% more bees entering or exiting a beehive housing the population relative to a population of bees comprising the reference bee(s) at about 12°C to about 15°C, about 160% more bees entering or exiting a beehive housing the population relative to a population of bees comprising the reference bee(s) at about 12°C to about 15°C to about 300% more bees entering or exiting a beehive housing the population relative to a population of bees comprising the reference bee(s) at about 12°C to about 15°C, about 160% more bees entering or exiting a beehive housing the population relative to a population of bees comprising the reference bee(s) at about 12°C to about 15°C to about 320% more bees entering or exiting a beehive housing the population relative to a population of bees comprising the reference bee(s) at about 12°C to about 15°C, about 180% more bees entering or exiting a beehive housing the population relative to a population of bees comprising the reference bee(s) at about 12°C to about 15°C to about 200% more bees entering or exiting a beehive housing the population relative to a population of bees comprising the reference bee(s) at about 12°C to about 15°C, about 180% more bees entering or exiting a beehive housing the population relative to a population of bees comprising the reference bee(s) at about 12°C to about 15°C to about 220% more bees entering or exiting a beehive housing the population relative to a population of bees comprising the reference bee(s) at about 12°C to about 15°C, about 180% more bees entering or exiting a beehive housing the population relative to a population of bees comprising the reference bee(s) at about 12°C to about 15°C to about 240% more bees entering or exiting a beehive housing the population relative to a population of bees comprising the reference bee(s) at about 12°C to about 15°C, about 180% more bees entering or exiting a beehive housing the population relative to a population of bees comprising the reference bee(s) at about 12°C to about 15°C to about 260% more bees entering or exiting a beehive housing the population relative to a population of bees comprising the reference bee(s) at about 12°C to about 15°C, about 180% more bees entering or exiting a beehive housing the population relative to a population of bees comprising the reference bee(s) at about 12°C to about 15°C to about 280% more bees entering or exiting a beehive housing the population relative to a population of bees comprising the reference bee(s) at about 12°C to about 15°C, about 180% more bees entering or exiting a beehive housing the population relative to a population of bees comprising the reference bee(s) at about 12°C to about 15°C to about 300% more bees entering or exiting a beehive housing the population relative to a population of bees comprising the reference bee(s) at about 12°C to about 15°C, about 180% more bees entering or exiting a beehive housing the population relative to a population of bees comprising the reference bee(s) at about 12°C to about 15°C to about 320% more bees entering or exiting a beehive housing the population relative to a population of bees comprising the reference bee(s) at about 12°C to about 15°C, about 200% more bees entering or exiting a beehive housing the population relative to a population of bees comprising the reference bee(s) at about 12°C to about 15°C to about 220% more bees entering or exiting a beehive housing the population relative to a population of bees comprising the reference bee(s) at about 12°C to about 15°C, about 200% more bees entering or exiting a beehive housing the population relative to a population of bees comprising the reference bee(s) at about 12°C to about 15°C to about 240% more bees entering or exiting a beehive housing the population relative to a population of bees comprising the reference bee(s) at about 12°C to about 15°C, about 200% more bees entering or exiting a beehive housing the population relative to a population of bees comprising the reference bee(s) at about 12°C to about 15°C to about 260% more bees entering or exiting a beehive housing the population relative to a population of bees comprising the reference bee(s) at about 12°C to about 15°C, about 200% more bees entering or exiting a beehive housing the population relative to a population of bees comprising the reference bee(s) at about 12°C to about 15°C to about 280% more bees entering or exiting a beehive housing the population relative to a population of bees comprising the reference bee(s) at about 12°C to about 15°C, about 200% more bees entering or exiting a beehive housing the population relative to a population of bees comprising the reference bee(s) at about 12°C to about 15°C to about 300% more bees entering or exiting a beehive housing the population relative to a population of bees comprising the reference bee(s) at about 12°C to about 15°C, about 200% more bees entering or exiting a beehive housing the population relative to a population of bees comprising the reference bee(s) at about 12°C to about 15°C to about 320% more bees entering or exiting a beehive housing the population relative to a population of bees comprising the reference bee(s) at about 12°C to about 15°C, about 220% more bees entering or exiting a beehive housing the population relative to a population of bees comprising the reference bee(s) at about 12°C to about 15°C to about 240% more bees entering or exiting a beehive housing the population relative to a population of bees comprising the reference bee(s) at about 12°C to about 15°C, about 220% more bees entering or exiting a beehive housing the population relative to a population of bees comprising the reference bee(s) at about 12°C to about 15°C to about 260% more bees entering or exiting a beehive housing the population relative to a population of bees comprising the reference bee(s) at about 12°C to about 15°C, about 220% more bees entering or exiting a beehive housing the population relative to a population of bees comprising the reference bee(s) at about 12°C to about 15°C to about 280% more bees entering or exiting a beehive housing the population relative to a population of bees comprising the reference bee(s) at about 12°C to about 15°C, about 220% more bees entering or exiting a beehive housing the population relative to a population of bees comprising the reference bee(s) at about 12°C to about 15°C to about 300% more bees entering or exiting a beehive housing the population relative to a population of bees comprising the reference bee(s) at about 12°C to about 20°C, about 220% more bees entering or exiting a beehive housing the population relative to a population of bees comprising the reference bee(s) at about 12°C to about 15°C to about 320% more bees entering or exiting a beehive housing the population relative to a population of bees comprising the reference bee(s) at about 12°C to about 15°C, about 240% more bees entering or exiting a beehive housing the population relative to a population of bees comprising the reference bee(s) at about 12°C to about 15°C to about 260% more bees entering or exiting a beehive housing the population relative to a population of bees comprising the reference bee(s) at about 12°C to about 15°C, about 240% more bees entering or exiting a beehive housing the population relative to a population of bees comprising the reference bee(s) at about 12°C to about 15°C to about 280% more bees entering or exiting a beehive housing the population relative to a population of bees comprising the reference bee(s) at about 12°C to about 15°C, about 240% more bees entering or exiting a beehive housing the population relative to a population of bees comprising the reference bee(s) at about 12°C to about 15°C to about 300% more bees entering or exiting a beehive housing the population relative to a population of bees comprising the reference bee(s) at about 12°C to about 15°C, about 240% more bees entering or exiting a beehive housing the population relative to a population of bees comprising the reference bee(s) at about 12°C to about 15°C, about 12°C to about 16°C, about 12°C to about 17°C, about 12°C to about 18°C, about 12°C to about 19°C, about 12°C to about 20°C, about 13°C to about 14°C, about 13°C to about 15°C, about 13°C to about 16°C, about 13°C to about 17°C, about 13°C to about 18°C, about 13°C to about 19°C, about 13°C to about 20°C, about 14°C to about 15°C, about 14°C to about 16°C, about 14°C to about 17°C, about 14°C to about 18°C, about 14°C to about 19°C, about 14°C to about 20°C, about 15°C to about 16°C, about 15°C to about 17°C, about 15°C to about 18°C, about 15°C to about 19°C, about 15°C to about 20°C, about 16°C to about 17°C, about 16°C to about 18°C, about 16°C to about 19°C, about 16°C to about 20°C, about 17°C to about 18°C, about 17°C to about 19°C, about 17°C to about 20°C, about 18°C to about 19°C, about 18°C to about 20°C, or about 19°C to about 20°C. In some embodiments, to about 320% more bees entering or exiting a beehive housing the population relative to a population of bees comprising the reference bee(s) at about 12°C to about 15°C, about 260% more bees entering or exiting a beehive housing the population relative to a population of bees comprising the reference bee(s) at about 12°C to about 15°C to about 280% more bees entering or exiting a beehive housing the population relative to a population of bees comprising the reference bee(s) at about 12°C to about 15°C, about 260% more bees entering or exiting a beehive housing the population relative to a population of bees comprising the reference bee(s) at about 12°C to about 15°C to about 300% more bees entering or exiting a beehive housing the population relative to a population of bees comprising the reference bee(s) at about 12°C to about 13°C, about 14°C, about 15°C, about 16°C, about 17°C, about 18°C, about 19°C, or about 20°C. In some embodiments,260% more bees entering or exiting a beehive housing the population relative to a population of bees comprising the reference bee(s) at about 12°C to about 15°C to about 320% more bees entering or exiting a beehive housing the population relative to a population of bees comprising the reference bee(s) at about 12°C to about 15°C, about 280% more bees entering or exiting a beehive housing the population relative to a population of bees comprising the reference bee(s) at about 12°C to about 15°C to about 300% more bees entering or exiting a beehive housing the population relative to a population of bees comprising the reference bee(s) at about 12°C to about 13°C, about 14°C, about 15°C, about 16°C, about 17°C, about 18°C, or about 19°C. In some embodiments,280% more bees entering or exiting a beehive housing the population relative to a population of bees comprising the reference bee(s) at about 12°C to about 15°C to about 320% more bees entering or exiting a beehive housing the population relative to a population of bees comprising the reference bee(s) at about 12°C to about 15°C, or about 300% more bees entering or exiting a beehive housing the population relative to a population of bees comprising the reference bee(s) at most about 12°C to about 15°C, about 15°C, about 16°C, about 17°C, about 18°C, about 19°C, or about 20°C. to about 320% more bees entering or exiting a beehive housing the population relative to a population of bees comprising the reference bee(s) at about 12°C to about 15°C. In some embodiments, a population of bees comprising the bee(s) comprises about 100% more bees entering or exiting a beehive housing the population relative to a population of bees comprising the reference bee(s) at about 12°C to about 15°C, about 120% more bees entering or exiting a beehive housing the population relative to a population of bees comprising the reference bee(s) at about 12°C to about 15°C, about 140% more bees entering or exiting a beehive housing the population relative to a population of bees comprising the reference bee(s) at about 12°C to about 15°C, about 160% more bees entering or exiting a beehive housing the population relative to a population of bees comprising the reference bee(s) at about 12°C to about 15°C, about 180% more bees entering or exiting a beehive housing the population relative to a population of bees comprising the reference bee(s) at about 12°C to about 15°C, about 200% more bees entering or exiting a beehive housing the population relative to a population of bees comprising the reference bee(s) at about 12°C to about 15°C, about 220% more bees entering or exiting a beehive housing the population relative to a population of bees comprising the reference bee(s) at about 12°C to about 15°C, about 240% more bees entering or exiting a beehive housing the population relative to a population of bees comprising the reference bee(s) at about 12°C to about 15°C, about 260% more bees entering or exiting a beehive housing the population relative to a population of bees comprising the reference bee(s) at about 12°C to about 15°C, about 280% more bees entering or exiting a beehive housing the population relative to a population of bees comprising the reference bee(s) at about 12°C to about 15°C, about 300% more bees entering or exiting a beehive housing the population relative to a population of bees comprising the reference bee(s) at about 12°C to about 15°C, or about 320% more bees entering or exiting a beehive housing the population relative to a population of bees comprising the reference bee(s) at about 12°C to about 15°C. In some embodiments, a population of bees comprising the bee(s) comprises at least about 100% more bees entering or exiting a beehive housing the population relative to a population of bees comprising the reference bee(s) at about 12°C to about 15°C, about 120% more bees entering or exiting a beehive housing the population relative to a population of bees comprising the reference bee(s) at about 12°C to about 15°C, about 140% more bees entering or exiting a beehive housing the population relative to a population of bees comprising the reference bee(s) at about 12°C to about 15°C, about 160% more bees entering or exiting a beehive housing the population relative to a population of bees comprising the reference bee(s) at about 12°C to about 15°C, about 180% more bees entering or exiting a beehive housing the population relative to a population of bees comprising the reference bee(s) at about 12°C to about 15°C, about 200% more bees entering or exiting a beehive housing the population relative to a population of bees comprising the reference bee(s) at about 12°C to about 15°C, about 220% more bees entering or exiting a beehive housing the population relative to a population of bees comprising the reference bee(s) at about 12°C to about 15°C, about 240% more bees entering or exiting a beehive housing the population relative to a population of bees comprising the reference bee(s) at about 12°C to about 15°C, about 260% more bees entering or exiting a beehive housing the population relative to a population of bees comprising the reference bee(s) at about 12°C to about 15°C, about 280% more bees entering or exiting a beehive housing the population relative to a population of bees comprising the reference bee(s) at about 12°C to about 15°C, or about 300% more bees entering or exiting a beehive housing the population relative to a population of bees comprising the reference bee(s) at about 12°C to about 15°C. In some embodiments, a population of bees comprising the bee(s) comprises at most about 120% more bees entering or exiting a beehive housing the population relative to a population of bees comprising the reference bee(s) at about 12°C to about 15°C, about 140% more bees entering or exiting a beehive housing the population relative to a population of bees comprising the reference bee(s) at about 12°C to about 15°C, about 160% more bees entering or exiting a beehive housing the population relative to a population of bees comprising the reference bee(s) at about 12°C to about 15°C, about 180% more bees entering or exiting a beehive housing the population relative to a population of bees comprising the reference bee(s) at about 12°C to about 15°C, about 200% more bees entering or exiting a beehive housing the population relative to a population of bees comprising the reference bee(s) at about 12°C to about 15°C, about 220% more bees entering or exiting a beehive housing the population relative to a population of bees comprising the reference bee(s) at about 12°C to about 15°C, about 240% more bees entering or exiting a beehive housing the population relative to a population of bees comprising the reference bee(s) at about 12°C to about 15°C, about 260% more bees entering or exiting a beehive housing the population relative to a population of bees comprising the reference bee(s) at about 12°C to about 15°C, about 280% more bees entering or exiting a beehive housing the population relative to a population of bees comprising the reference bee(s) at about 12°C to about 15°C, about 300% more bees entering or exiting a beehive housing the population relative to a population of bees comprising the reference bee(s) at about 12°C to about 15°C, or about 320% more bees entering or exiting a beehive housing the population relative to a population of bees comprising the reference bee(s) at about 12°C to about 15°C.

In some embodiments, the compositions disclosed herein supplement the immune system of bees that consume the compositions. Improvement in immune system can be measured in a variety of ways. In some embodiments, the bee(s) that consume the compositions disclosed herein develop increased resistance to crop pesticides. In some embodiments, the bee(s) that consume the compositions disclosed herein develop increased hemocytes relative to reference bees.

### V. IMPROVED BEE PERFORMANCE AND FRUIT YIELD

The compositions described herein can be utilized in methods to increase fruit yield of plants that are pollinated by bees. Fruit-yield can be measured in a variety of ways. In some embodiments, fruit-yield is measured in fruit-set. Fruit-set is defined as the percentage of flowers that produce a fruit. This can be measured, by way of non-limiting example, selecting a branch in a tree and counting the number of flowers during bloom. Then, after the bloom, the number of fruits that were developed from those flowers counted. The fruit set can then be calculated by dividing the number of fruits by the number of flowers. The fruit-set can be calculated for a single plant, or across an entire farm or plot. As used herein, unless otherwise specified, fruit-set refers to the measurements taken from random branches across an entire farm or plot. In some embodiments, fruit-yield is measured in fruit-set and fruit-weight. In the examples described herein, in each plot, 5 plants were marked (i.e., 5 plants per plot * 9 plots, 45 plants in total), and within each plant 3 branches of the flowering plant were randomly selected and tagged with paper tape (a total of 225 experimental branches). The number of open flowers between the tag and the end of the branch were counted in order to determine the number of flowers that produced a fruit; thi is the fruit set. The examples described herein provide for the estimation of variation in flowers within and among plants and plantations, subjected to the different pollination practices.

In some embodiments, fruit-yield is measured in fruit-weight. Fruit-weight is defined as the average weight of a single piece of fruit, averaged among the fruit's total harvest. In some embodiments, the number of fruits (corresponding to each tagged branch) developed from each flowering plant was counted in relation to the total number of flowers originally present on the branch to estimate fruit-set. Then, the quality of all tagged fruits harvested was evaluated by quantifying individual weight and firmness.

Alfalfa, Blueberry, Cranberry, Scarlet Runner Bean, Sunflower, Carrot, Guar Bean, Goa Bean, Karite, Quince, Lemon, Lime, Okra, Tamarind, Loquat, Orange, Grapefruit, Tangelo, Strawberry Tree, Tangerine, Rose Hips, Dogroses, Alsike Clover, Arrowleaf Clover, Black Currant, Red Currant, Boysenberry, Broad Bean, Clover, Cotton, Cowpea, Black-Eyed Pea, Blackeye Bean, Crimson Clover, Crownvetch, Flax, Kiwifruit, Lupine, Persimmon, Red Clover, Vetch, Watermelon, White Clover, Almond, Apricot, Peach, Nectarine, Plum, Greengage, Mirabelle, Sloe, Sour Cherry, Sweet Cherry, Blackberry, Pear, Raspberry, Lychee, Apple, Acerola, Azarole, Broccoli, Brussels Sprouts, Buckwheat, Cabbage, Cardamom, Cauliflower, Chestnut, Chinese Cabbage, Coriander, Feijoa, Hyacinth Bean, Lima Bean, Kidney Bean, Haricot Bean, Adzuki Bean, Mungo Bean, String Bean, Green Bean, Rapeseed, Safflower, Sainfoin, Turnip, Canola, Allspice, Pigeon Pea, CajanPea, Congo Bean, Mustard, Pomegranate, Onion, Rowanberry, Caraway, Celery, Fennel, Grape, Jujube, Elderberry, Sesame, Cucumber, Squash, Pumpkin, Gourd, Marrow, Zucchini, Cantaloupe, Melon, Carambola, Starfruit, Coconut, Longan, Hog Plum, ChilliPepper, Red Pepper, Bell Pepper, Green Pepper, Macadamia, Cashew, Strawberry, Guava, Rambutan, Mango, CoffeaSpp. CoffeaArabica, CoffeaCanephora, Papaya, Beet, and avocado plants are pollinated by bees.

In some embodiments, the bees pollinate the flowering plant throughout the flowering period. In some embodiments, the flowering period of the plant is about 7 days to about 50 days. In some embodiments, the flowering period of the plant is about 7 days to about 10 days, about 7 days to about 15 days, about 7 days to about 20 days, about 7 days to about 25 days, about 7 days to about 30 days, about 7 days to about 35 days, about 7 days to about 40 days, about 7 days to about 45 days, about 7 days to about 50 days, about 10 days to about 15 days, about 10 days to about 20 days, about 10 days to about 25 days, about 10 days to about 30 days, about 10 days to about 35 days, about 10 days to about 40 days, about 10 days to about 45 days, about 10 days to about 50 days, about 15 days to about 20 days, about 15 days to about 25 days, about 15 days to about 30 days, about 15 days to about 35 days, about 15 days to about 40 days, about 15 days to about 45 days, about 15 days to about 50 days, about 20 days to about 25 days, about 20 days to about 30 days, about 20 days to about 35 days, about 20 days to about 40 days, about 20 days to about 45 days, about 20 days to about 50 days, about 25 days to about 30 days, about 25 days to about 35 days, about 25 days to about 40 days, about 25 days to about 45 days, about 25 days to about 50 days, about 30 days to about 35 days, about 30 days to about 40 days, about 30 days to about 45 days, about 30 days to about 50 days, about 35 days to about 40 days, about 35 days to about 45 days, about 35 days to about 50 days, about 40 days to about 45 days, about 40 days to about 50 days, or about 45 days to about 50 days. In some embodiments, the flowering period of the plant is about 7 days, about 10 days, about 15 days, about 20 days, about 25 days, about 30 days, about 35 days, about 40 days, about 45 days, or about 50 days. In some embodiments, the flowering period of the plant is at least about 7 days, about 10 days, about 15 days, about 20 days, about 25 days, about 30 days, about 35 days, about 40 days, or about 45 days. In some embodiments, the flowering period of the plant is at most about 10 days, about 15 days, about 20 days, about 25 days, about 30 days, about 35 days, about 40 days, about 45 days, or about 50 days.

In some embodiments, the method comprises improvement of fruit-set of one or more blueberry plant(s) by about 1% to about 30% in blueberry plant(s) pollinated by the bee(s) relative to one or more blueberry plant(s) pollinated by the reference bee(s). In some embodiments, the improvement of fruit-set of one or more blueberry plant(s) is about 1% to about 2%, about 1% to about 3%, about 1% to about 4%, about 1% to about 5%, about 1% to about 10%, about 1% to about 15%, about 1% to about 20%, about 1% to about 25%, about 1% to about 30%, about 2% to about 3%, about 2% to about 4%, about 2% to about 5%, about 2% to about 10%, about 2% to about 15%, about 2% to about 20%, about 2% to about 25%, about 2% to about 30%, about 3% to about 4%, about 3% to about 5%, about 3% to about 10%, about 3% to about 15%, about 3% to about 20%, about 3% to about 25%, about 3% to about 30%, about 4% to about 5%, about 4% to about 10%, about 4% to about 15%, about 4% to about 20%, about 4% to about 25%, about 4% to about 30%, about 5% to about 10%, about 5% to about 15%, about 5% to about 20%, about 5% to about 25%, about 5% to about 30%, about 10% to about 15%, about 10% to about 20%, about 10% to about 25%, about 10% to about 30%, about 15% to about 20%, about 15% to about 25%, about 15% to about 30%, about 20% to about 25%, about 20% to about 30%, or about 25% to about 30%. In some embodiments, the improvement of fruit-set of one or more blueberry plant(s) is about 1%, about 2%, about 3%, about 4%, about 5%, about 10%, about 15%, about 20%, about 25%, or about 30%. In some embodiments, the improvement of fruit-set of one or more blueberry plant(s) is at least about 1%, about 2%, about 3%, about 4%, about 5%, about 10%, about 15%, about 20%, or about 25%. In some embodiments, the improvement of fruit-set of one or more blueberry plant(s) is at most about 2%, about 3%, about 4%, about 5%, about 10%, about 15%, about 20%, about 25%, or about 30%.

In some embodiments, the method comprises improvement of fruit-set of one or more almond plant(s) by about 1% to about 40% in almond plant(s) pollinated by the bee(s) relative to one or more almond plant(s) pollinated by the reference bee(s In some embodiments, the improvement of fruit-set of one or more almond plant(s) is about 1% to about 5%, about 1% to about 10%, about 1% to about 15%, about 1% to about 20%, about 1% to about 25%, about 1% to about 30%, about 1% to about 35%, about 1% to about 40%, about 5% to about 10%, about 5% to about 15%, about 5% to about 20%, about 5% to about 25%, about 5% to about 30%, about 5% to about 35%, about 5% to about 40%, about 10% to about 15%, about 10% to about 20%, about 10% to about 25%, about 10% to about 30%, about 10% to about 35%, about 10% to about 40%, about 15% to about 20%, about 15% to about 25%, about 15% to about 30%, about 15% to about 35%, about 15% to about 40%, about 20% to about 25%, about 20% to about 30%, about 20% to about 35%, about 20% to about 40%, about 25% to about 30%, about 25% to about 35%, about 25% to about 40%, about 30% to about 35%, about 30% to about 40%, or about 35% to about 40%. In some embodiments, the improvement of fruit-set of one or more almond plant(s) is about 1%, about 5%, about 10%, about 15%, about 20%, about 25%, about 30%, about 35%, or about 40%. In some embodiments, the improvement of fruit-set of one or more almond plant(s) is at least about 1%, about 5%, about 10%, about 15%, about 20%, about 25%, about 30%, or about 35%. In some embodiments, the improvement of fruit-set of one or more almond plant(s) is at most about 5%, about 10%, about 15%, about 20%, about 25%, about 30%, about 35%, or about 40%.

In some embodiments, the method comprises improvement of fruit-set of one or more kiwifruit plant(s) by about 20% to about 80% in kiwifruit plant(s) pollinated by the bee(s) relative to one or more kiwifruit plant(s) pollinated by the reference bee(s). In some embodiments, the improvement of fruit-set of one or more kiwifruit plant(s) is about 20% to about 25%, about 20% to about 30%, about 20% to about 35%, about 20% to about 40%, about 20% to about 45%, about 20% to about 50%, about 20% to about 60%, about 20% to about 70%, about 20% to about 80%, about 25% to about 30%, about 25% to about 35%, about 25% to about 40%, about 25% to about 45%, about 25% to about 50%, about 25% to about 60%, about 25% to about 70%, about 25% to about 80%, about 30% to about 35%, about 30% to about 40%, about 30% to about 45%, about 30% to about 50%, about 30% to about 60%, about 30% to about 70%, about 30% to about 80%, about 35% to about 40%, about 35% to about 45%, about 35% to about 50%, about 35% to about 60%, about 35% to about 70%, about 35% to about 80%, about 40% to about 45%, about 40% to about 50%, about 40% to about 60%, about 40% to about 70%, about 40% to about 80%, about 45% to about 50%, about 45% to about 60%, about 45% to about 70%, about 45% to about 80%, about 50% to about 60%, about 50% to about 70%, about 50% to about 80%, about 60% to about 70%, about 60% to about 80%, or about 70% to about 80%. In some embodiments, the improvement of fruit-set of one or more kiwifruit plant(s) is about 20%, about 25%, about 30%, about 35%, about 40%, about 45%, about 50%, about 60%, about 70%, or about 80%. In some embodiments, the improvement of fruit-set of one or more kiwifruit plant(s) is at least about 20%, about 25%, about 30%, about 35%, about 40%, about 45%, about 50%, about 60%, or about 70%. In some embodiments, the improvement of fruit-set of one or more kiwifruit plant(s) is at most about 25%, about 30%, about 35%, about 40%, about 45%, about 50%, about 60%, about 70%, or about 80%.

In some embodiments, the method comprises improvement of fruit-set of one or more apple plant(s) by about 1% to about 40% in apple plant(s) pollinated by the bee(s) relative to one or more apple plants pollinated by the reference bee(s). In some embodiments, the improvement of fruit-set of one or more apple plant(s) is about 1% to about 5%, about 1% to about 10%, about 1% to about 15%, about 1% to about 20%, about 1% to about 25%, about 1% to about 30%, about 1% to about 35%, about 1% to about 40%, about 5% to about 10%, about 5% to about 15%, about 5% to about 20%, about 5% to about 25%, about 5% to about 30%, about 5% to about 35%, about 5% to about 40%, about 10% to about 15%, about 10% to about 20%, about 10% to about 25%, about 10% to about 30%, about 10% to about 35%, about 10% to about 40%, about 15% to about 20%, about 15% to about 25%, about 15% to about 30%, about 15% to about 35%, about 15% to about 40%, about 20% to about 25%, about 20% to about 30%, about 20% to about 35%, about 20% to about 40%, about 25% to about 30%, about 25% to about 35%, about 25% to about 40%, about 30% to about 35%, about 30% to about 40%, or about 35% to about 40%. In some embodiments, the improvement of fruit-set of one or more apple plant(s) is about 1%, about 5%, about 10%, about 15%, about 20%, about 25%, about 30%, about 35%, or about 40%. In some embodiments, the improvement of fruit-set of one or more apple plant(s) is at least about 1%, about 5%, about 10%, about 15%, about 20%, about 25%, about 30%, or about 35%. In some embodiments, the improvement of fruit-set of one or more apple plant(s) is at most about 5%, about 10%, about 15%, about 20%, about 25%, about 30%, about 35%, or about 40%.

In some embodiments, the improvement of fruit weight of one or more blueberry plant(s) is about 1% to about 40% in blueberry plant(s) pollinated by the bee(s) relative to one or more blueberry plant(s) pollinated by the reference bee(s). In some embodiments, the improvement of fruit weight of one or more blueberry plant(s) about 1% to about 5%, about 1% to about 10%, about 1% to about 15%, about 1% to about 20%, about 1% to about 25%, about 1% to about 30%, about 1% to about 35%, about 1% to about 40%, about 5% to about 10%, about 5% to about 15%, about 5% to about 20%, about 5% to about 25%, about 5% to about 30%, about 5% to about 35%, about 5% to about 40%, about 10% to about 15%, about 10% to about 20%, about 10% to about 25%, about 10% to about 30%, about 10% to about 35%, about 10% to about 40%, about 15% to about 20%, about 15% to about 25%, about 15% to about 30%, about 15% to about 35%, about 15% to about 40%, about 20% to about 25%, about 20% to about 30%, about 20% to about 35%, about 20% to about 40%, about 25% to about 30%, about 25% to about 35%, about 25% to about 40%, about 30% to about 35%, about 30% to about 40%, or about 35% to about 40%. In some embodiments, the improvement of fruit weight of one or more blueberry plant(s) about 1%, about 5%, about 10%, about 15%, about 20%, about 25%, about 30%, about 35%, or about 40%. In some embodiments, the improvement of fruit weight of one or more blueberry plant(s) at least about 1%, about 5%, about 10%, about 15%, about 20%, about 25%, about 30%, or about 35%. In some embodiments, the improvement of fruit weight of one or more blueberry plant(s) at most about 5%, about 10%, about 15%, about 20%, about 25%, about 30%, about 35%, or about 40%.

In some embodiments, the improvement of fruit weight of one or more blueberry plant(s) is about 10% to about 11%, about 10% to about 12%, about 10% to about 13%, about 10% to about 14%, about 10% to about 15%, about 10% to about 16%, about 10% to about 17%, about 10% to about 18%, about 10% to about 19%, about 10% to about 20%, about 11% to about 12%, about 11% to about 13%, about 11% to about 14%, about 11% to about 15%, about 11% to about 16%, about 11% to about 17%, about 11% to about 18%, about 11% to about 19%, about 11% to about 20%, about 12% to about 13%, about 12% to about 14%, about 12% to about 15%, about 12% to about 16%, about 12% to about 17%, about 12% to about 18%, about 12% to about 19%, about 12% to about 20%, about 13% to about 14%, about 13% to about 15%, about 13% to about 16%, about 13% to about 17%, about 13% to about 18%, about 13% to about 19%, about 13% to about 20%, about 14% to about 15%, about 14% to about 16%, about 14% to about 17%, about 14% to about 18%, about 14% to about 19%, about 14% to about 20%, about 15% to about 16%, about 15% to about 17%, about 15% to about 18%, about 15% to about 19%, about 15% to about 20%, about 16% to about 17%, about 16% to about 18%, about 16% to about 19%, about 16% to about 20%, about 17% to about 18%, about 17% to about 19%, about 17% to about 20%, about 18% to about 19%, about 18% to about 20%, or about 19% to about 20%. In some embodiments, the improvement of fruit weight of one or more blueberry plant(s) is about 10%, about 11%, about 12%, about 13%, about 14%, about 15%, about 16%, about 17%, about 18%, about 19%, or about 20%. In some embodiments, the improvement of fruit weight of one or more blueberry plant(s) is at least about 10%, about 11%, about 12%, about 13%, about 14%, about 15%, about 16%, about 17%, about 18%, or about 19%. In some embodiments, the improvement of fruit weight of one or more blueberry plant(s) is at most about 11%, about 12%, about 13%, about 14%, about 15%, about 16%, about 17%, about 18%, about 19%, or about 20%.

In some embodiments, the method comprises improvement of fruit weight of one or more kiwifruit plant(s) by about 1% to about40% in kiwifruit plant(s) pollinated by the bee(s) relative to one or more kiwifruit plant(s) pollinated by the reference bee(s). In some embodiments, the improvement of fruit weight of one or more kiwifruit plant(s) about 1% to about 5%, about 1% to about 10%, about 1% to about 15%, about 1% to about 20%, about 1% to about 25%, about 1% to about 30%, about 1% to about 35%, about 1% to about 40%, about 5% to about 10%, about 5% to about 15%, about 5% to about 20%, about 5% to about 25%, about 5% to about 30%, about 5% to about 35%, about 5% to about 40%, about 10% to about 15%, about 10% to about 20%, about 10% to about 25%, about 10% to about 30%, about 10% to about 35%, about 10% to about 40%, about 15% to about 20%, about 15% to about 25%, about 15% to about 30%, about 15% to about 35%, about 15% to about 40%, about 20% to about 25%, about 20% to about 30%, about 20% to about 35%, about 20% to about 40%, about 25% to about 30%, about 25% to about 35%, about 25% to about 40%, about 30% to about 35%, about 30% to about 40%, or about 35% to about 40%. In some embodiments, the improvement of fruit weight of one or more kiwifruit plant(s) about 1%, about 5%, about 10%, about 15%, about 20%, about 25%, about 30%, about 35%, or about 40%. In some embodiments, the improvement of fruit weight of one or more kiwifruit plant(s) at least about 1%, about 5%, about 10%, about 15%, about 20%, about 25%, about 30%, or about 35%. In some embodiments, the improvement of fruit weight of one or more kiwifruit plant(s) at most about 5%, about 10%, about 15%, about 20%, about 25%, about 30%, about 35%, or about 40%.

In some embodiments, the amount of seeds a kiwifruit plant pollinated by bees fed the compositions described herein increases relative to a kiwifruit plant pollinated by reference bees by about 20% to about 80%. In some embodiments, the amount of seeds a kiwifruit plant pollinated by bees fed the compositions described herein increases relative to a kiwifruit plant pollinated by reference bees by about 20% to about 25%, about 20% to about 30%, about 20% to about 35%, about 20% to about 40%, about 20% to about 45%, about 20% to about 50%, about 20% to about 55%, about 20% to about 60%, about 20% to about 65%, about 20% to about 70%, about 20% to about 80%, about 25% to about 30%, about 25% to about 35%, about 25% to about 40%, about 25% to about 45%, about 25% to about 50%, about 25% to about 55%, about 25% to about 60%, about 25% to about 65%, about 25% to about 70%, about 25% to about 80%, about 30% to about 35%, about 30% to about 40%, about 30% to about 45%, about 30% to about 50%, about 30% to about 55%, about 30% to about 60%, about 30% to about 65%, about 30% to about 70%, about 30% to about 80%, about 35% to about 40%, about 35% to about 45%, about 35% to about 50%, about 35% to about 55%, about 35% to about 60%, about 35% to about 65%, about 35% to about 70%, about 35% to about 80%, about 40% to about 45%, about 40% to about 50%, about 40% to about 55%, about 40% to about 60%, about 40% to about 65%, about 40% to about 70%, about 40% to about 80%, about 45% to about 50%, about 45% to about 55%, about 45% to about 60%, about 45% to about 65%, about 45% to about 70%, about 45% to about 80%, about 50% to about 55%, about 50% to about 60%, about 50% to about 65%, about 50% to about 70%, about 50% to about 80%, about 55% to about 60%, about 55% to about 65%, about 55% to about 70%, about 55% to about 80%, about 60% to about 65%, about 60% to about 70%, about 60% to about 80%, about 65% to about 70%, about 65% to about 80%, or about 70% to about 80%. In some embodiments, the amount of seeds a kiwifruit plant pollinated by bees fed the compositions described herein increases relative to a kiwifruit plant pollinated by reference bees by about 20%, about 25%, about 30%, about 35%, about 40%, about 45%, about 50%, about 55%, about 60%, about 65%, about 70%, or about 80%. In some embodiments, the amount of seeds a kiwifruit plant pollinated by bees fed the compositions described herein increases relative to a kiwifruit plant pollinated by reference bees by at least about 20%, about 25%, about 30%, about 35%, about 40%, about 45%, about 50%, about 55%, about 60%, about 65%, or about 70%. In some embodiments, the amount of seeds a kiwifruit plant pollinated by bees fed the compositions described herein increases relative to a kiwifruit plant pollinated by reference bees by at most about 25%, about 30%, about 35%, about 40%, about 45%, about 50%, about 55%, about 60%, about 65%, about 70%, or about 80%.

In some embodiments, the method comprises improvement of fruit-set of one or more plant(s) by about 1% to about 40% in plant(s) pollinated by the bee(s) relative to one or more apple plants pollinated by the reference bee(s). In some embodiments, the improvement of fruit-set of one or more plant(s) is about 1% to about 5%, about 1% to about 10%, about 1% to about 15%, about 1% to about 20%, about 1% to about 25%, about 1% to about 30%, about 1% to about 35%, about 1% to about 40%, about 5% to about 10%, about 5% to about 15%, about 5% to about 20%, about 5% to about 25%, about 5% to about 30%, about 5% to about 35%, about 5% to about 40%, about 10% to about 15%, about 10% to about 20%, about 10% to about 25%, about 10% to about 30%, about 10% to about 35%, about 10% to about 40%, about 15% to about 20%, about 15% to about 25%, about 15% to about 30%, about 15% to about 35%, about 15% to about 40%, about 20% to about 25%, about 20% to about 30%, about 20% to about 35%, about 20% to about 40%, about 25% to about 30%, about 25% to about 35%, about 25% to about 40%, about 30% to about 35%, about 30% to about 40%, or about 35% to about 40%. In some embodiments, the improvement of fruit-set of one or more plant(s) is about 1%, about 5%, about 10%, about 15%, about 20%, about 25%, about 30%, about 35%, or about 40%. In some embodiments, the improvement of fruit-set of one or more plant(s) is at least about 1%, about 5%, about 10%, about 15%, about 20%, about 25%, about 30%, or about 35%. In some embodiments, the improvement of fruit-set of one or more plant(s) is at most about 5%, about 10%, about 15%, about 20%, about 25%, about 30%, about 35%, or about 40%.

In some embodiments, the improvement of fruit weight of one or more plant(s) is about 1% to about 40% in plant(s) pollinated by the bee(s) relative to one or more plant(s) pollinated by the reference bee(s). In some embodiments, the improvement of fruit weight of one or more plant(s) about 1% to about 5%, about 1% to about 10%, about 1% to about 15%, about 1% to about 20%, about 1% to about 25%, about 1% to about 30%, about 1% to about 35%, about 1% to about 40%, about 5% to about 10%, about 5% to about 15%, about 5% to about 20%, about 5% to about 25%, about 5% to about 30%, about 5% to about 35%, about 5% to about 40%, about 10% to about 15%, about 10% to about 20%, about 10% to about 25%, about 10% to about 30%, about 10% to about 35%, about 10% to about 40%, about 15% to about 20%, about 15% to about 25%, about 15% to about 30%, about 15% to about 35%, about 15% to about 40%, about 20% to about 25%, about 20% to about 30%, about 20% to about 35%, about 20% to about 40%, about 25% to about 30%, about 25% to about 35%, about 25% to about 40%, about 30% to about 35%, about 30% to about 40%, or about 35% to about 40%. In some embodiments, the improvement of fruit weight of one or more plant(s) about 1%, about 5%, about 10%, about 15%, about 20%, about 25%, about 30%, about 35%, or about 40%. In some embodiments, the improvement of fruit weight of one or more plant(s) at least about 1%, about 5%, about 10%, about 15%, about 20%, about 25%, about 30%, or about 35%. In some embodiments, the improvement of fruit weight of one or more plant(s) at most about 5%, about 10%, about 15%, about 20%, about 25%, about 30%, about 35%, or about 40%.

### VI. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

Throughout this application, various embodiments may be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

As used in the specification and claims, the singular forms "a", "an" and "the" include plural references unless the context clearly dictates otherwise. For example, the term "a sample" includes a plurality of samples, including mixtures thereof.

The terms "determining," "measuring," "evaluating," "assessing," "assaying," and "analyzing" are often used interchangeably herein to refer to forms of measurement. The terms include determining if an element is present or not (for example, detection). These terms can include quantitative, qualitative or quantitative and qualitative determinations. Assessing can be relative or absolute. "Detecting the presence of" can include determining the amount of something present in addition to determining whether it is present or absent depending on the context.

The terms "control" or "reference bees" or "a reference bee" or "reference bees" or "a reference bee" all refer to bees that do not consume the compositions described herein. These bees can be found in nature or used for experimentation. In some cases, as noted when applicable, reference bees will also not be subject to the distribution methods described herein.

As used herein, "feeding" and "supplementing" mean to administer the compositions described herein to the bees. This administration can include administering the composition directly to the bees in the form of bee feed or it can include adding the compositions described herein to the bees' existing feed.

As used herein, "bee" refers to a flying insect of the clade Anthophila. Bees described herein may be members of the Melittidae, Apidae, Megachilidae, Andrenidae, Halictidae, Colletidae, or Stenotritidae families. Bees described herein may be members of the Apinae, Nomadinae, or Xylocopinae sub-families. Non-limiting examples of bee species include *Apis mellifera, Apis andreniformis, Apis florea, Apis dorsata, Apis cerana, Apis koschevnikovi, Apis nigrocincta, Apis dorsata,* and *Bombus angustus, Bombus terrestris, Bombus atratus, Bombus pauloensis, Bombus impatients.*

As used herein, "improving pollination performance" can be equated to "increase forager bee activity " or "increasing visitation frequency to flowering plant."

The term *"in vivo"* is used to describe an event that takes place in a subject's body.

The term *"ex vivo"* is used to describe an event that takes place outside of a subject's body. An *ex vivo* assay is not performed on a subject. Rather, it is performed upon a sample separate from a subject. An example of an *ex vivo* assay performed on a sample is an *"in vitro"* assay.

As used herein, "nutritional supplement" means a feed supplement that can help a bee colony when it is under stress and/or improve the health and/or performance of a bee colony. Such stress can be an environmental stress, such as temperature or too little/too much rain. Nutritional supplements may comprise protein sources, amino acid sources, sugar sources, one or more pollen substitutes, probiotics, prebiotics, antibiotics, stimulants, oils, essential oils and/or vitamins.

As used herein, the term "about" a number refers to that number plus or minus 10% of that number. The term "about" a range refers to that range minus 10% of its lowest value and plus 10% of its greatest value.

As used herein, the terms "treatment" or "treating" are used in reference to a pharmaceutical or other intervention regimen for obtaining beneficial or desired results in the recipient. Beneficial or desired results include but are not limited to a therapeutic benefit and/or a prophylactic benefit. A therapeutic benefit may refer to eradication or amelioration of symptoms or of an underlying disorder being treated. Also, a therapeutic benefit can be achieved with the eradication or amelioration of one or more of the physiological symptoms associated with the underlying disorder such that an improvement is observed in the subject, notwithstanding that the subject may still be afflicted with the underlying disorder. A prophylactic effect includes delaying, preventing, or eliminating the appearance of a disease or condition, delaying or eliminating the onset of symptoms of a disease or condition, slowing, halting, or reversing the progression of a disease or condition, or any combination thereof. For prophylactic benefit, a subject at risk of developing a particular disease, or to a subject reporting one or more of the physiological symptoms of a disease may undergo treatment, even though a diagnosis of this disease may not have been made.

The section headings used herein are for organizational purposes only and are not to be construed as limiting the subject matter described.

### VII. EXAMPLES

### Example 1 - Composition containing ABA and L-Arginine ("L-Arg")

The following experiment was carried out with the generous support of Consejo Nacional de Investigaciones Científicas y Técnicas (CONICET) - https://www.conicet.gov.ar/.

ABA was added to sucrose syrup (2: 1, sucrose:water) to achieve a concentration of 10 µM ABA. Two groups of small colonies of honey bees were used to test if the compound was reaching the bee larvae. One group of colonies was exposed to the solution by way of adding the solution to feed the bees in the beehive, while the other was not. In order to corroborate that the ABA supplemented in the syrup actually reached the larvae, the ABA content was measured in fifth instar larvae (L5) two months after starting treatment. The larvae were found to have 28x higher concentration of ABA compared to the control colony.

ABA and L-Arg were added to sucrose syrup (2:1, sucrose:water) to achieve a concentration of 50 µM ABA and 5 mM L-Arg. This formulation was utilized in the following Examples 2 to 9.

### Example 2 - Pollination Performance of Honey Bees that consume the Composition in Almond Fields.

3 hectares of Marinada variety almond trees and 1.5 hectares of a combination of one to one Tarraco and Marinada varieties were pollinated with bees fed with the ABA and L-Arg supplemented bee feed described in **Example 1.** The bees were fed 1L/week of this supplemented bee feed for one month before the start of the blooming period and throughout the pollination period. The 3 hectares of Marinada variety almond trees were pollinated with 9 hives (3 hives/hectare), and the 1.5 hectares of mixed Tarraco/Marinada variety almond trees were pollinated by 8 hives (~5 hives/hectare). Supplemented honey bee hives in the 3 hectare Marinada variety and in the 1.5 hectare Tarraco-Marinada varieties were placed within the field, homogeneously and individually distributed between almond trees. Prior to pollination, bees were fed a nutritional solution with another supplement to increase the attractiveness of bees to almond blossoms. After 15 days, bees were given the supplement to increase the attractiveness again. Bee colonies were checked, fed, and incentivized to maximize foraging 3 times during the pollination period of 24 days.

As a control, bee hives with bees not fed the supplement from **Example 1** were placed in groups of ~10 hives on the edge of a field containing Marinada variety almond trees at a rate of 3 hives/hectare and in groups of ~10 hives on the edge of a field containing mixed Tarraco/Marinada variety almond trees at a rate of 5 hives/hectare. These hives also did not receive the supplement to increase the attractiveness of bees to almond flowers.

Activity of the bees from treated and untreated hives was monitored periodically throughout the pollination process. Bees fed the ABA and L-Arg supplement showed substantially increased activity when compared with the reference bees. Several different measurements were taken: brood development (larvae), amount of forager bees in a given population of bees, and flower visitation frequency.

### Brood Development and Increase in Forager Bees

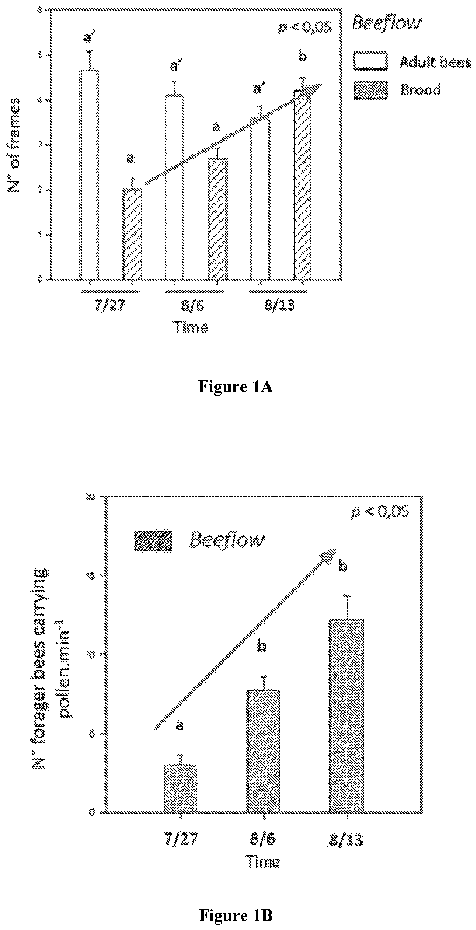

During a time period previous to the pollination period (17 days) of an almond field, brood size and the number of adult bees were measured at day 1 (7/27), day 11 (8/6) and day 18 (8/13). Results can be seen in **Fig. 1A****,** which shows an increase in brood size in a population of bees that were fed the composition measured by number of beehive frames covered with adult bees or brood. On these same days, the number of forager bees carrying pollen entering the hive measured over a period of time (per minute) increased. The results can be seen in **Fig. 1B****.**

### Flower Visitation Frequency

Frequency of visits by bees to flowers was measured periodically throughout the flowering period. Observations were made by censuses, which involved the observation of ~40 flowers for a period of 5 minutes in order to count how many bees visited the flowers in a unit time (number of visits/flower/hour). 224 censuses in the Marinada fields were carried out. Observations were made at a variety of times, including morning, noon, and evening. The result of the aggregate of these counts is shown in **Fig. 2A****.** Marinada flowers pollinated with treated bees that fed both the supplements from **Example** 1 and that to increase the attractiveness of bees to almond, and were placed within the field, received a much higher visitation rate than the reference Marina flowers (a 66% increase).

### Example 3 - Pollination Performance of Honey Bees that Consume the Composition at Low Temperatures

During the experiments detailed in Example 2, the activities of the bees from treated and untreated hives were monitored during lower temperatures. Particularly, the amount of forager bees (responsible for transporting pollen to flowers), and the number of forager bees entering or exiting the given bee population's beehive per minute (unit measuring the pollination activity of honey bees). During periods of low temperature (9-15°C), there was a 300% increase in the number of bees entering or exiting their respective hives (**Fig. 3A**) amongst the bees fed the composition in **Example 1** and were distributed individually and homogenously throughout the fields, relative to the reference bees that were further distributed in big groups, between 5 to 50 beehives, and located either next to or up to hundreds of meters from the field.

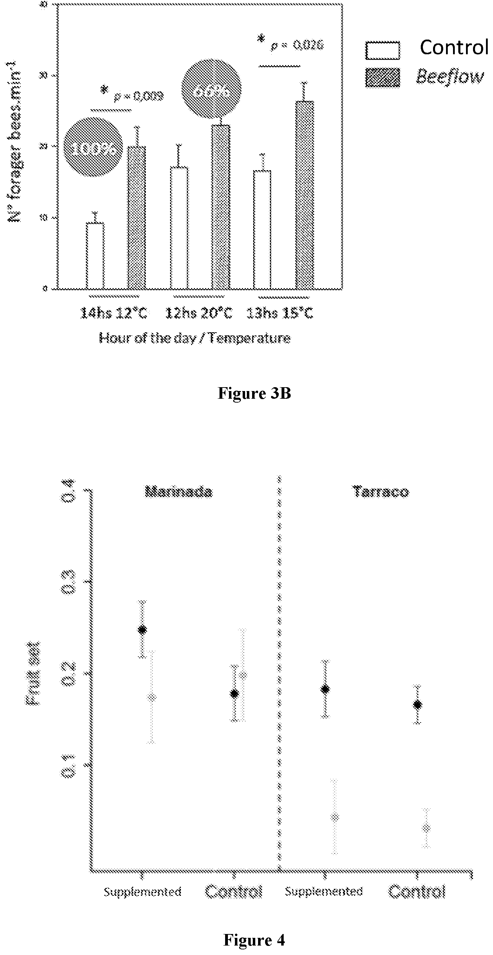

Prior to the blooming described in **Examples 2-3,** and prior to the treated bees' beehives being individually and homogeneously distributed, bee populations fed the composition (supplemented bees) showed up to a 100% increase in the number of forager bees relative to reference bees (control, bees not supplemented with the compositions from **Example 1** and contained in the same apiary as the treated bees) populations at 12°C **(****Fig. 3B****).** Bee populations fed the composition showed up to a 66% increase in the number of forager bees relative to reference bee control, bees not supplemented with the compositions from **Example 1** (and contained in the same apiary as the treated bees) populations at 15°C **(****Fig. 3B****).**

Measurements were taken at various times of the day and at varying temperatures. Bees fed the composition in Example 1 showed 100% more forager bee activity than reference bees not fed the composition from **Example** 1 at 12°C and 66% more activity than reference bees at 15°C **(****Fig. 3B****).** These measurements were continuously taken at various time points.

### Example 4 - Effects of Bees Fed the Composition from Example 1 on Almond Production

The Tarraco and Marinada varieties of almond plants from Example 2 were observed to determine the effects of: (1) the improved pollination performance compound, (2) the increase the attractiveness of bees to almond compound and (3) hives distribution within the field, on fruit-yield. To determine fruit-yield, the number of almond flowers that produced almonds was measured.

For both the experimental bees fed the composition from Example 1, feed a solution with another supplement to increase the attractiveness of bees to almond blossoms, and hives distributed within the field, and reference bee hives, 15 Marinada and 15 Tarraco variety almond trees (60 trees total) were marked prior to flowering to estimate what proportion of flowers develop fruit (hereafter fruit set). On each plant, four branches were labeled, and one was used to evaluate the dependence of pollinators, and three to estimate the efficiency of pollination. The branch dedicated to evaluation the dependency of pollinators was covered with a mesh to prevent access to the flowers by the pollinators, and the number of flowers on each branch were counted, and then counted again later to determine how many of these flowers developed fruit. In total, 30 branches were isolated for each variety. Branches marked to assess efficiency of pollination were marked and the number of flowers on each branch was counted. At the end of the experiment, the number of flowers that had developed fruit was counted to determine fruit-set.

**Fig. 4** shows the results of this experiment. Black bars show the fruit set for the flowers which were accessible to the pollinators (efficiency of pollination). Gray bars show the flowers covered in the mesh (pollination dependency). Supplemented bees with both compound and distributed within the field showed improved fruit set for the Marinada varietals, whereas the reference bees showed minimal effect on fruit set.

Overall, almond fruit-yield increased by 23% in almond plants pollinated by honey bees that: (1) fed the composition from **Example 1,** (2) received a supplement component to increase the attractiveness of bees to almond flowers, and (3) were homogeneously distributed within the almond field.

### Example 5 - Pollination Performance of Honey Bees that consume the Composition in Blueberry Plants.

3 blueberry fields of the Emerald variety were pollinated with bees fed with the ABA and L-Arg supplemented bee feed described in **Example 1.** The bees were fed 1L/week of this supplemented bee feed for one month before the start of the blooming period and throughout the pollination period. The 3 fields of blueberry were pollinated with hives at 10 hives/hectare. Treated honey bee hives in the 3 fields of the blueberries were placed within the field, homogeneously and individually distributed between plants. As a control, 6 blueberry fields also of the Emerald variety were pollinated with bee hives with bees not fed the supplement from **Example 1. Control** bee hives were placed in groups of ~10 hives on the edge of the fields containing Emerald variety blueberry plants at a rate of 10 hives/hectare.

Treated bees that were fed the supplement from **Example 1** and were placed within the field of blueberries had a 70% increase in flower visitation frequency as compared to reference bees **(****Fig. 2B****).**

Blueberry plants from the Emerald variety from **Example 5** were observed to determine the effects of: the improved pollination performance compound of honey bees and hives distribution within the field on fruit-yield and quality. To determine fruit-yield and quality, the number of blueberry flowers that produced fruits was measured and fruits were collected for further analysis (see explanation below).

For both the experimental bees fed the composition from **Example 5** and reference bee hives, 5 Emerald variety blueberry plants per field (45 plants total) were marked prior to flowering to estimate what proportion of flowers develop fruit (fruit set). On each plant, three branches were labeled to estimate the efficiency of pollination. The number of flowers on each branch marked were counted, and then counted again later to determine how many of these flowers developed fruit to determine fruit-set.

Along with fruit-set, the average fruit-weight, per blueberry was also evaluated to determine overall increase in fruit-yield and quality due to the increased bee pollination performance and hives' distribution.

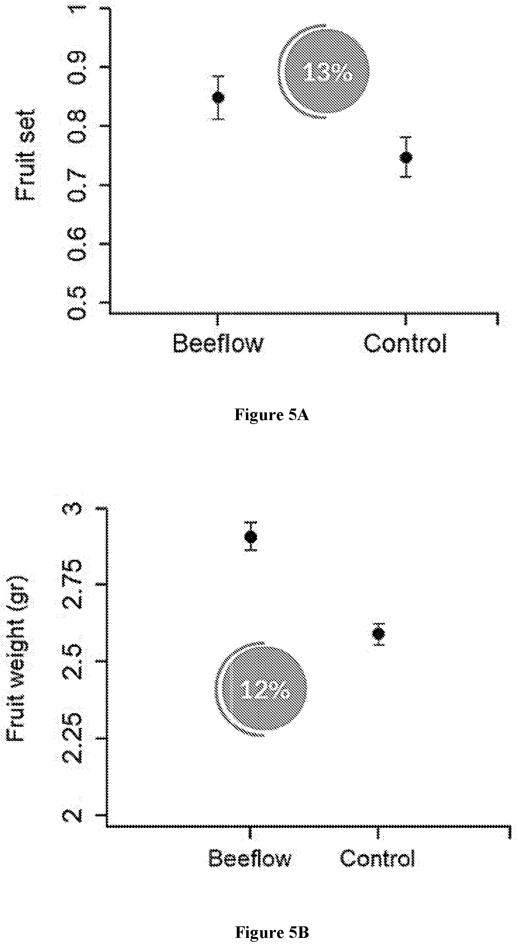

**Fig. 5** shows the results the blueberry flower fruit-set. Blueberry plants pollinated by honey bees that: (1) fed the composition from **Example 1** and (2) were homogeneously distributed within the almond field showed a 13% increase in fruit-set compared to reference plants. The weight of the harvested blueberries was also measured. Blueberry plants pollinated by honey bees that: (1) fed the composition from **Example 1** and (2) were homogeneously distributed within the almond field showed a 12% increase in fruit-weight compared to reference bees.

Overall, blueberry fruit-yield increased by 25% in blueberry plants pollinated by honey bees that: (1) fed the composition from **Example 1** and (2) were homogeneously distributed within the almond field.

### Example 6 -Effects of bees fed the Composition from Example 1 on Kiwifruit Production

One kiwifruit field -Hayward variety- was pollinated with bees fed with the ABA and L-Arg supplemented bee feed described in **Example 1.** The bees were fed 1L/week of this supplemented bee feed for one month before the start of the blooming period and throughout the pollination period. The field of kiwi -Hayward variety- was pollinated with hives at a rate of 12 hives/hectare. Treated honey bee hives in the field Hayward variety were placed within the field, homogeneously and individually distributed between plants. Bee colonies were checked, fed, and incentivized to maximize foraging 3 times during the pollination period of 24 days.

As a control, one kiwifruit field -Hayward variety- was artificially pollinated with spray by man. Pollen was sprayed once during the flowering period, when most flower were receptive.

Kiwi plants from the Hayward variety from **Example 6** were observed to determine the effects of the improved pollination performance of honey bees and hives distribution on fruit-yield and quality. To determine fruit-yield and quality, the number of kiwi flowers that produced fruits was measured and fruits were collected for further analysis (see explanation below).

For both the experimental bees fed the composition from **Example 1** and reference kiwi field pollinated artificially, 20 Hayward variety kiwi plants per field (40 plants total) were marked prior to flowering to estimate what proportion of flowers develop fruit (fruit set). On each plant, 20 flowers were labeled to estimate the efficiency of pollination. Before harvest, the number of fruits were counted to determine how many of marked the flowers developed fruit to determine fruit-set.

Along with fruit-set, the average fruit-weight, per kiwifruit was also evaluated to determine overall increase in fruit-yield due to the increased bee pollination performance.

Kiwifruit plants pollinated by honey bees fed the composition from **Example 1** showed a 43% increase in fruit-set compared to reference plants pollinated artificially (figures not shown). The weight of the harvested kiwifruits was also measured. Kiwifruit plants pollinated by honey bees fed the composition from **Example 1** showed a 34% increase in fruit-weight compared to reference plants pollinated artificially (figures not shown).

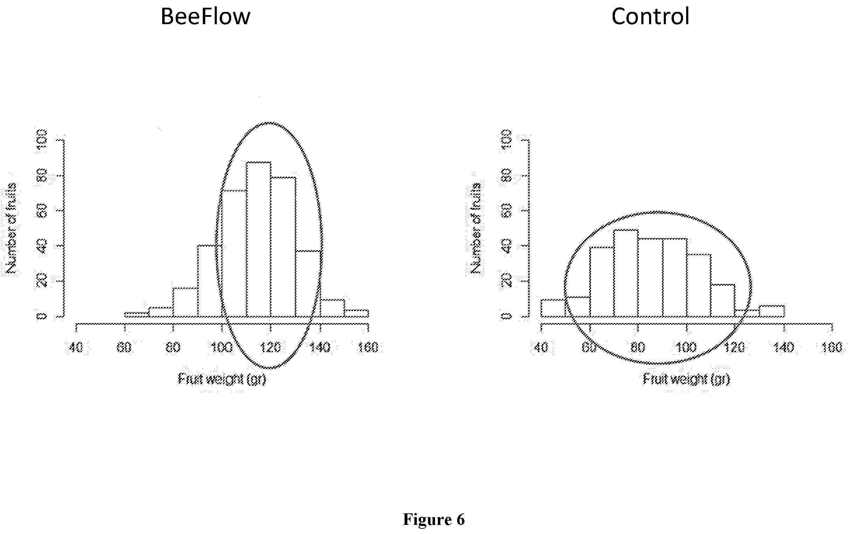

Overall, kiwifruit fruit-yield increased by 91% in kiwifruit plants pollinated by honey bees fed the composition from **Example 1** and were distributed within the field. Interestingly, kiwifruits plants pollinated by honey bees fed the composition from **Example 1** also showed increased homogeneity in fruit weight distribution in comparison with those pollinated artificially **(****Fig. 6****).**

The data on the effects of pollination by honey bees fed the compositions described herein on kiwifruit yield is summarized in **Table 1** below.

| **Table 1. Kiwifruit Results** | | | |
|---|---|---|---|
| | Control | BeeFlow | % Increase in BeeFlow Pollinated Kiwifruit |
| Estimated fruit-set | 65% +/- 0.05% | 93% +/- 0.03% | 43 |
| Estimated seed quantity | 17.92 +/- 1.27 | 24.08 +/- 0.91 | 34 |
| Estimated fruit weight (g) | 85.35 +/- 2.71 | 114.42 +/- 1.94 | 34 |

### Example 7 -Effects of bees fed the Composition from Example 1 on Apple Production

One field of Pink Lady variety apple trees and one field of Gala variety apple trees were pollinated with bees fed with the ABA and L-Arg supplemented bee feed described in **Example 1.** The bees were fed 1L/week of this supplemented bee feed for one month before the start of the blooming period and throughout the pollination period. Both fields of Pink Lady and Gala varieties apple trees were pollinated with a density of 8 hives/hectare. Supplemented honey bee hives in both Pink Lady and Gala varieties were placed within the field, homogeneously and individually distributed between apple trees. Prior to pollination, bees were fed a nutritional solution with another supplement to increase the attractiveness of bees to apple blossoms. After 15 days, bees were given this supplement again. Bee colonies were checked, fed, and incentivized to maximize foraging 3 times during the pollination period of 24 days.

As a control, one field of Pink Lady variety apple trees and one field of Gala variety apple were pollinated with bee hives with bees not fed the supplement from **Example 1.** Both Pink Lady and Gala varieties were pollinated at unknown density of hives/hectare. These hives also did not receive the supplement to increase the attractiveness of bees to almond flowers.

Activity of the bees from treated and untreated hives was monitored periodically throughout the pollination process. Honey bees fed the composition had a 79% increase in flower visitation frequency for Pink Lady Apple plants and a 73% increase in Gala Apple plants, as compared to reference bees **(****Fig. 2C****).**

Along with fruit-set, the average fruit-weight, per apple was also evaluated to determine overall increase in fruit-yield due to the increased bee pollination performance.

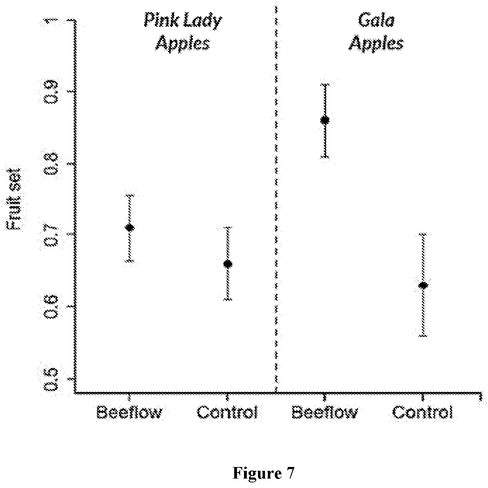

Pink Lady Apple plants and Gala Apple plants pollinated by honey bees fed the composition from **Example 1,** received the supplement to increase attractiveness of bees to flowers and were distributed within the field showed an increase in fruit-set compared to reference plants, pollinated at unknow stocking rate **(****Fig. 7****).**

### Example 8-Increased Flower Visitation and Fruit Set of Blueberry Flowers

Field work was conducted during the flowering period (from July 17 to August 23) on 3 blueberry plots (*Vaccinium corymbosum* variety "Emerald"). One plot was pollinated with the bees fed with the composition herein while other 2 plots were pollinated with bees fed with sugar syrup but without being supplemented with the composition (control). Each plot comprised a 1-ha area, planted with Emerald variety. The plants were all from the same age and subjected to the same agricultural management in respect to fertilization, water, etc. The bees were fed 1L/week of the composition described herein or only sugar syrup, for one month, before the pollination period. All the bee hives came from the same stock of bees from the same beekeeper. All the plots were pollinated by honey bees (*Apis mellifera* L.), at a density of 10 hives per cultivated ha.

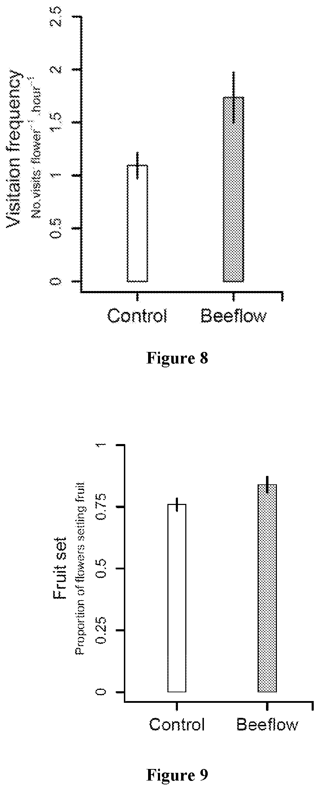

Visitation frequency to blueberry flowers: Activity of the bees fed and not-fed with the composition was monitored periodically throughout the blueberry bloom associated with the pollination process. Bees fed with the composition increased visitation frequency to blueberry flowers in 70% when compared with the reference (control) bees (see **Fig. 8****).**

Fruit set: The field pollinated by the bees fed with the composition produced 11% more fruit set than the fields pollinated by the control bees (see **Fig. 9****).**

### Example 9 -Effects of bees fed the Composition from Example 1 on Raspberry Production

3 raspberry fields -Maravilla variety- were pollinated with bees fed with the ABA and L-Arg supplemented bee feed described in **Example 1.** The bees were fed 1L/week of this supplemented bee feed for one month before the start of the blooming period and throughout the pollination period. The 3 fields of raspberry -Maravilla variety- were pollinated with hives at a rate of 10 hives/hectare. Treated honey bee hives in the 3 fields of Maravilla variety were placed within the field, homogeneously and individually distributed between plants. Bee colonies were checked, fed, and incentivized to maximize foraging 3 times during the pollination period of 24 days.

As a control, 3 raspberry fields -Maravilla variety- were pollinated with bee hives with bees not fed the supplement from **Example 1.** Bee hives were placed in groups of ~5 hives on the edge of the fields containing Maravilla variety raspberry plants at a rate of 10 hives/hectare.

Raspberry plants from the Maravilla variety were observed to determine the effects of the improved pollination performance of honey bees on fruit-yield and quality. To determine fruit-yield and quality, the number and quality of raspberry fruits was measured and fruits were collected for further analysis (see explanation below).

For both the experimental bees fed the composition from **Example 1** and reference bee hives, 15 Maravilla variety raspberry plants per field (90 plants total) were sampled to estimate fruit quality.

The average fruit-weight and quality, per raspberry was also evaluated to determine overall increase in fruit-yield and quality due to the increased bee pollination performance and hives distribution.

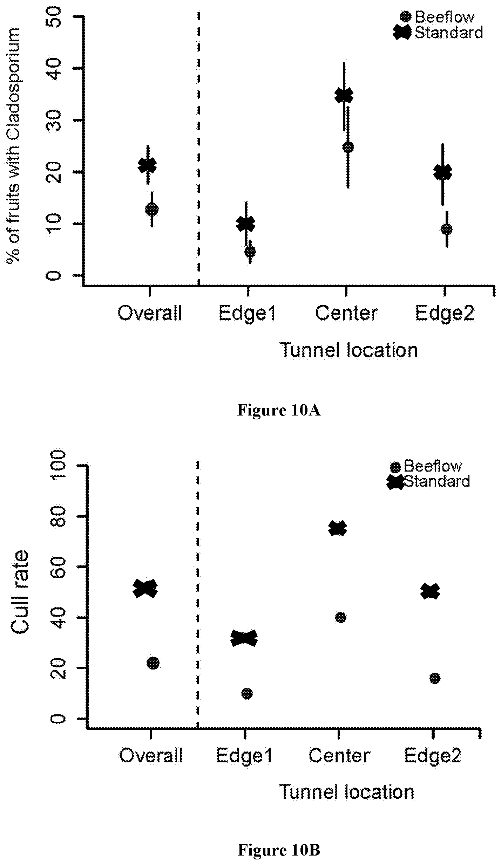

Raspberry flowers emit a nectar during bloom. The presence of this nectar can lead to increased microbe presence on the fruit. Increased microbe presence leads to culled (discarded) fruit that provides no value. **Fig. 10A** shows the reduction in raspberries developed with cladosprium. **Fig. 10B** shows in decrease in cull rate of raspberries produced by treated bees. Raspberry plants pollinated by honey bees that: (1) fed the composition from **Example 1,** and (2) were homogeneously distributed within the raspberry field showed about a 60% reduction cladosporium and an about 80% reduction in cull rate.

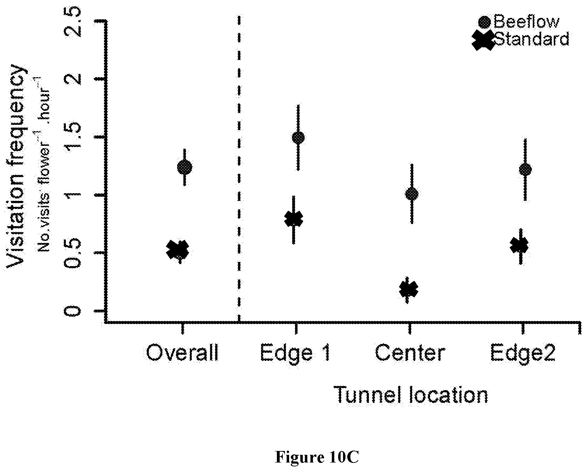

Further, **Fig. 10C** shows a 200% increase in flower visitation frequency in honey bees that: (1) were fed the composition from **Example 1,** and (2) were homogeneously distributed within the raspberry field.

## Claims

1. A method of enhancing fruit-yield of a flowering plant, the method comprising exposing said flowering plant to a plurality of bees, wherein said plurality of bees comprise one or more bees supplemented with a solution comprising about 1 µM to 1500 µM abscisic acid (ABA) or a salt thereof and about 0.1 mM to 10 mM L-arginine or a salt thereof, and further comprising distributing one or more beehives containing said plurality of bees adjacent to a plurality of flowering plants at a rate of at most 10 beehives/hectare of land comprising said plurality of flowering plants, wherein said one or more beehives are homogeneously distributed, wherein the supplement is fed to the one or more bees at least about 1 month prior to the blooming period of the flowering plant, and wherein the supplement is fed to the one or more bees in an amount of 0.4 L/week to 2.2 L/week.

2. The method of claim 1, further comprising exposing said flowering plant to said plurality of bees, wherein said plurality of bees comprise one or more bees supplemented with a formulation that increases said one or more bees' attractiveness to said flowering plant.

3. The method of claim 1, wherein said solution further comprises a sugar to water ratio, wherein said sugar to water ratio is in the range of about 1:1 sugar:water and about 2:1 sugar:water, wherein said sugar comprises sucrose, fructose, glucose, or a combination thereof.

4. The method of claim 3, wherein said sugar comprises sucrose.

5. The method of claim 1, wherein said solution comprises about 50 µM ABA and about 5 mM of L-arginine.

6. The method of claim 1, wherein said flowering plant comprises blueberry, almond, apple, kiwifruit, or raspberry.

7. The method of claim 1, wherein said plurality of bees comprises one or more bees of the species *Apis mellifera, Apis andreniformis, Apis florea, Apis dorsata, Apis cerana, Apis koschevnikovi, Apis nigrocincta, Apis dorsata,* or *Bombus angustus, Bombus terrestris, Bombus atratus, Bombus pauloensis, Bombus impatients.*

8. The method of claim 7, wherein said plurality of bees comprises one or more bees of the species *Apis mellifera.*

9. The method of claim 1, wherein fruit-yield comprises a flower fruit-set, a fruit weight, a reduction in cull rate, or a combination thereof, wherein said enhanced fruit-yield is relative to a fruit field of a flowering plant contacted with one or more reference bees not supplemented with the solution comprising about 1 µM to 1500 µM abscisic acid (ABA) and about 0.1 mM to 10 mM L- arginine.

10. A method of enhancing a pollination performance of one or more bees, the method comprising contacting said one or more bees with a supplement, wherein said supplement comprises about 1 µM to 1500 µM abscisic acid (ABA) or a salt thereof and about 0.1 mM to 10 mM L-arginine or a salt thereof, and further comprising distributing one or more beehives containing said one or more bees adjacent to a plurality of flowering plants at a rate of at most 10 beehives/hectare of land comprising said plurality of flowering plants, wherein said one or more beehives are homogeneously distributed, wherein the supplement is fed to the one or more bees at least about 1 month prior to the blooming period of the flowering plant, and wherein the supplement is fed to the one or more bees in an amount of 0.4 L/week to 2.2 L/week.

11. The method of claim 10, further comprising contacting one or more bees with a formulation that increases said one or more bees' attractiveness to a flowering plant.

12. The method of claim 10, wherein said pollination performance comprises forager bee activity, visitation frequency to a flowering plant, or a combination thereof.

13. The method of claim 10, wherein said supplement further comprises a sugar to water ratio, wherein said sugar to water ratio is in the range of about 1:1 sugar:water and about 2:1 sugar:water, wherein said sugar comprises sucrose, fructose, glucose, or a combination thereof.

14. The method of claim 13, wherein said sugar comprises sucrose.

15. The method of claim 10, wherein said supplement is water soluble.

16. The method of claim 10, wherein said supplement comprises about 50 µM ABA and about 5 mM of L-arginine.

17. The method of claim 11, wherein said flowering plant comprises blueberry, almond, apple, or kiwifruit.

18. The method of claim 10, wherein said one or more bees comprises one or more bees of the species *Apis mellifera, Apis andreniformis, Apis florea, Apis dorsata, Apis cerana, Apis koschevnikovi, Apis nigrocincta, Apis dorsata,* or *Bombus angustus, Bombus terrestris, Bombus atratus, Bombus pauloensis, Bombus impatients.*

19. The method of claim 18, wherein said one or more bees comprises one or more bees of the species *Apis mellifera.*

## Patentansprüche

1. Verfahren zur Steigerung des Fruchtertrags einer blühenden Pflanze, wobei das Verfahren umfasst, die blühende Pflanze einer Vielzahl von Bienen auszusetzen, wobei die Vielzahl von Bienen eine oder mehrere Bienen umfasst, die mit einer Lösung versetzt sind, die eine Lösung, die etwa 1 µM bis 1500 µM Abscisinsäure (ABA) oder ein Salz davon und etwa 0,1 mM bis 10 mM L-Arginin oder ein Salz davon umfasst, und ferner umfassend das Verteilen eines oder mehrerer Bienenstöcke, die die Vielzahl von Bienen enthalten, neben einer Vielzahl von blühenden Pflanzen mit einer Dichte von höchstens 10 Bienenstöcken pro Hektar Land, das die Vielzahl von blühenden Pflanzen umfasst, wobei die eine oder mehreren Bienenstöcke homogen verteilt sind, wobei das Ergänzungsmittel den einen oder mehreren Bienen mindestens etwa einen Monat vor der Blütezeit der blühenden Pflanze verabreicht wird und wobei das Ergänzungsmittel den einen oder mehreren Bienen in einer Menge von 0,4 I/Woche bis 2,2 I/Woche verabreicht wird.

2. Verfahren nach Anspruch 1, das ferner das Aussetzen der blühenden Pflanze gegenüber der Vielzahl von Bienen umfasst, wobei die Vielzahl von Bienen eine oder mehrere Bienen umfasst, denen eine Formulierung zugesetzt wurde, die die Attraktivität der einen oder mehreren Bienen für die blühende Pflanze erhöht.

3. Verfahren nach Anspruch 1, wobei die Lösung ferner ein Zucker-Wasser-Verhältnis umfasst, wobei das Zucker-Wasser-Verhältnis im Bereich von etwa 1:1 Zucker:Wasser und etwa 2:1 Zucker:Wasser liegt, wobei der Zucker Saccharose, Fructose, Glucose oder eine Kombination davon umfasst.

4. Verfahren nach Anspruch 3, wobei der Zucker Saccharose umfasst.

5. Verfahren nach Anspruch 1, wobei die Lösung etwa 50 µM ABA und etwa 5 mM L-Arginin umfasst.

6. Verfahren nach Anspruch 1, wobei die blühende Pflanze Heidelbeere, Mandel, Apfel, Kiwi oder Himbeere umfasst.

7. Verfahren nach Anspruch 1, wobei die Vielzahl von Bienen eine oder mehrere Bienen der Arten *Apis mellifera, Apis andreniformis, Apis florea, Apis dorsata, Apis cerana, Apis koschevnikovi, Apis nigrocincta, Apis dorsata oder Bombus angustus, Bombus terrestris, Bombus atratus, Bombus pauloensis, Bombus impatients* umfasst.

8. Verfahren nach Anspruch 7, wobei die Vielzahl von Bienen eine oder mehrere Bienen der Art *Apis mellifera* umfasst.

9. Verfahren nach Anspruch 1, wobei der Fruchtertrag einen Blütenfruchtansatz, ein Fruchtgewicht, eine Verringerung der Ausschussrate oder eine Kombination davon umfasst, wobei der gesteigerte Fruchtertrag relativ zu einem Obstfeld einer blühenden Pflanze ist, die mit einer oder mehreren Referenzbienen in Kontakt gekommen ist, denen die Lösung, die etwa 1 µM bis 1500 µM Abscisinsäure (ABA) und etwa 0,1 mM bis 10 mM L-Arginin enthält, nicht verabreicht wurde.

10. Verfahren zur Verbesserung der Bestäubungsleistung einer oder mehrerer Bienen, wobei das Verfahren das in Kontakt bringen der einen oder mehreren Bienen mit einem Ergänzungsmittel umfasst, wobei das Ergänzungsmittel etwa 1 µM bis 1500 µM Abscisinsäure (ABA) oder ein Salz davon und etwa 0,1 mM bis 10 mM L-Arginin oder ein Salz davon umfasst, und ferner umfassend das Verteilen eines oder mehrerer Bienenstöcke, die die eine oder mehreren Bienen enthalten, in der Nähe einer Vielzahl von blühenden Pflanzen mit einer Dichte von höchstens 10 Bienenstöcken pro Hektar Land, das die Vielzahl von blühenden Pflanzen umfasst, wobei die eine oder mehreren Bienenstöcke homogen verteilt sind, wobei das Ergänzungsmittel den einen oder mehreren Bienen mindestens etwa einen Monat vor der Blütezeit der Blütenpflanze verabreicht wird und wobei das Ergänzungsmittel den einen oder mehreren Bienen in einer Menge von 0,4 I/Woche bis 2,2 I/Woche verabreicht wird.

11. Verfahren nach Anspruch 10, das ferner das in Kontakt bringen einer oder mehrerer Bienen mit einer Formulierung umfasst, die die Attraktivität der einen oder mehreren Bienen für eine blühende Pflanze erhöht.

12. Verfahren nach Anspruch 10, wobei die Bestäubungsleistung die Aktivität der Sammelbienen, die Besuchsfrequenz einer blühenden Pflanze oder eine Kombination davon umfasst.

13. Verfahren nach Anspruch 10, wobei das Ergänzungsmittel ferner ein Zucker-Wasser-Verhältnis umfasst, wobei das Zucker-Wasser-Verhältnis im Bereich von etwa 1:1 Zucker:Wasser und etwa 2:1 Zucker:Wasser liegt, wobei der Zucker Saccharose, Fructose, Glucose oder eine Kombination davon umfasst.

14. Verfahren nach Anspruch 13, wobei der Zucker Saccharose umfasst.

15. Verfahren nach Anspruch 10, wobei der Zusatz wasserlöslich ist.

16. Verfahren nach Anspruch 10, wobei der Zusatz etwa 50 µM ABA und etwa 5 mM L-Arginin umfasst.

17. Verfahren nach Anspruch 11, wobei die Blütenpflanze Heidelbeere, Mandel, Apfel oder Kiwi umfasst.

18. Verfahren nach Anspruch 10, wobei die eine oder mehreren Bienen eine oder mehrere Bienen der Arten *Apis mellifera, Apis andreniformis, Apis florea, Apis dorsata, Apis cerana, Apis koschevnikovi, Apis nigrocincta, Apis dorsata oder Bombus angustus, Bombus terrestris, Bombus atratus, Bombus pauloensis, Bombus impatients* umfassen.

19. Verfahren nach Anspruch 18, wobei die eine oder mehreren Bienen eine oder mehrere Bienen der Art *Apis mellifera* umfassen.

## Revendications

1. Procédé pour améliorer le rendement en fruits d'une plante à fleurs, le procédé comprenant l'exposition de ladite plante à fleurs à une pluralité d'abeilles, dans lequel ladite pluralité d'abeilles comprend une ou plusieurs abeilles supplémentées avec une solution comprenant environ 1 µM à 1500 µM d'acide abscissique (ABA) ou un sel de celui-ci et environ 0,1 mM à 10 mM de L-arginine ou un sel de celle-ci, et comprenant en outre la distribution d'une ou plusieurs ruches contenant ladite pluralité d'abeilles à proximité d'une pluralité de plantes à fleurs à raison d'au plus 10 ruches/ hectare de terrain comprenant ladite pluralité de plantes à fleurs, dans lequel ladite ou lesdites ruches sont réparties de manière homogène, dans lequel le supplément est administré à la ou aux abeilles au moins environ 1 mois avant la période de floraison de la plante à fleurs, et dans lequel le supplément est administré à la ou aux abeilles à raison de 0,4 L/semaine à 2,2 L/semaine.

2. Procédé de la revendication 1, comprenant en outre l'exposition de ladite plante à fleurs à ladite pluralité d'abeilles, dans lequel ladite pluralité d'abeilles comprend une ou plusieurs abeilles supplémentées avec une formulation qui augmente l'attractivité de ladite ou desdites abeilles pour ladite plante à fleurs.

3. Procédé de la revendication 1, dans lequel ladite solution comprend en outre un rapport sucre/eau, ledit rapport sucre/eau étant compris entre environ 1:1 sucre:eau et environ 2:1 sucre:eau, ledit sucre comprenant du saccharose, du fructose, du glucose ou une combinaison de ceux-ci.

4. Procédé de la revendication 3, dans lequel ledit sucre comprend du saccharose.

5. Procédé de la revendication 1, dans lequel ladite solution comprend environ 50 µM d'ABA et environ 5 mM de L-arginine.

6. Procédé de la revendication 1, dans lequel ladite plante à fleurs comprend la myrtille, l'amande, la pomme, le kiwi ou la framboise.

7. Procédé de la revendication 1, dans lequel ladite pluralité d'abeilles comprend une ou plusieurs abeilles de l'espèce *Apis mellifera, Apis andreniformis, Apis florea, Apis dorsata, Apis cerana, Apis koschevnikovi, Apis nigrocincta, Apis dorsata,* ou *Bombus angustus, Bombus terrestris, Bombus atratus, Bombus pauloensis, Bombus impatients.*

8. Procédé de la revendication 7, dans lequel ladite pluralité d'abeilles comprend une ou plusieurs abeilles de l'espèce *Apis mellifera.*

9. Procédé de la revendication 1, dans lequel le rendement en fruits comprend la nouaison, un poids des fruits, une réduction du taux de rejet, ou une combinaison de ceux-ci, dans laquelle ledit rendement fruitière amélioré est relatif à un champ fruitière d'une plante à fleurs en contact avec une ou plusieurs abeilles de référence non supplémentées avec la solution comprenant environ 1 µM à 1500 µM d'acide abscissique (ABA) et environ 0,1 mM à 10 mM de L-arginine.

10. Procédé pour améliorer les performances de pollinisation d'une ou plusieurs abeilles, le procédé comprenant la mise en contact de ladite ou desdites abeilles avec un supplément, dans lequel ledit supplément comprend environ 1 µM à 1500 µM d'acide abscissique (ABA) ou un sel de celui-ci et environ 0,1 mM à 10 mM de L-arginine ou un sel de celle-ci, et comprenant en outre la distribution d'une ou plusieurs ruches contenant ladite ou lesdites abeilles à proximité d'une pluralité de plantes à fleurs à un taux maximal de 10 ruches/ hectare de terrain comprenant ladite pluralité de plantes à fleurs, dans lequel ladite ou lesdites ruches sont réparties de manière homogène, dans lequel le supplément est administré à la ou aux abeilles au moins environ 1 mois avant la période de floraison de la plante à fleurs, et dans lequel le supplément est administré à la ou aux abeilles à raison de 0,4 L/semaine à 2,2 L/semaine.

11. Procédé de la revendication 10, comprenant en outre la mise en contact d'une ou plusieurs abeilles avec une formulation qui augmente l'attractivité de ladite ou desdites abeilles pour une plante à fleurs.

12. Procédé de la revendication 10, dans lequel ladite performance de pollinisation comprend l'activité des abeilles butineuses, la fréquence de visite d'une plante à fleurs, ou une combinaison de celles-ci.

13. Procédé de la revendication 10, dans lequel ledit supplément comprend en outre un rapport sucre/eau, ledit rapport sucre/eau étant compris entre environ 1:1 sucre:eau et environ 2:1 sucre:eau, ledit sucre comprenant du saccharose, du fructose, du glucose ou une combinaison de ceux-ci.

14. Procédé de la revendication 13, dans lequel ledit sucre comprend du saccharose.

15. Procédé de la revendication 10, dans lequel ledit supplément est soluble dans l'eau.

16. Procédé de la revendication 10, dans lequel ledit supplément comprend environ 50 µM d'ABA et environ 5 mM de L-arginine.

17. Procédé de la revendication 11, dans lequel ladite plante à fleurs comprend la myrtille, l'amande, la pomme ou le kiwi.

18. Procédé de la revendication 10, dans lequel ladite ou lesdites abeilles comprennent une ou plusieurs abeilles de l'espèce *Apis mellifera, Apis andreniformis, Apis florea, Apis dorsata, Apis cerana, Apis koschevnikovi, Apis nigrocincta, Apis dorsata,* ou *Bombus angustus, Bombus terrestris, Bombus atratus, Bombus pauloensis, Bombus impatients.*

19. Procédé de la revendication 18, dans lequel ladite ou lesdites abeilles comprennent une ou plusieurs abeilles de l'espèce *Apis mellifera.*
